(19) Europäisches Patentamt
European Patent Office
Office européen des brevets

(11) EP 3 597 640 A1

(12) **EUROPEAN PATENT APPLICATION**
published in accordance with Art. 153(4) EPC

(43) Date of publication:
**22.01.2020 Bulletin 2020/04**

(21) Application number: **18768087.1**

(22) Date of filing: **14.03.2018**

(51) Int Cl.:
*C07D 235/26* (2006.01) *A61K 31/4439* (2006.01)
*A61K 45/00* (2006.01) *A61P 13/00* (2006.01)
*A61P 25/00* (2006.01) *A61P 25/04* (2006.01)
*A61P 29/00* (2006.01) *A61P 43/00* (2006.01)
*C07D 401/12* (2006.01) *C07D 405/12* (2006.01)
*C07D 405/14* (2006.01)

(86) International application number:
**PCT/JP2018/009881**

(87) International publication number:
**WO 2018/168898 (20.09.2018 Gazette 2018/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.03.2017 JP 2017050341**

(71) Applicant: **Sumitomo Dainippon Pharma Co., Ltd.**
**Osaka-shi**
**Osaka 541-8524 (JP)**

(72) Inventors:
- **MIZUSHIMA, Shingo**
  **Osaka-shi**
  **Osaka 554-0022 (JP)**
- **WATANABE, Masaki**
  **Osaka-shi**
  **Osaka 554-0022 (JP)**
- **IWAMOTO, Kohei**
  **Osaka-shi**
  **Osaka 554-0022 (JP)**
- **URASHIMA, Kuniko**
  **Suita-shi**
  **Osaka 564-0053 (JP)**

(74) Representative: **Mewburn Ellis LLP**
**City Tower**
**40 Basinghall Street**
**London EC2V 5DE (GB)**

# (54) NOVEL BENZIMIDAZOLONE COMPOUND AND PHARMACEUTICAL USE THEREOF

(57) The present invention relates to a medicament for treating or preventing a disease involving Nav 1.7, specifically such neuropathic pain, nociceptive pain, inflammatory pain, small-fiber neuropathy, erythromelalgia, paroxysmal extreme pain disorder, dysuria, and multiple sclerosis, comprising a compound of formula (I) wherein $R^{1a}$, $R^{1b}$, $R^{1c}$, and $R^{1d}$ are hydrogen, halogen, cyano, $C_{1-4}$ alkyl, $C_{1-4}$ alkoxy, etc., provided that at least one of $R^{1a}$, $R^{1b}$, $R^{1c}$ and $R^{1d}$ is the above $C_{6-10}$ aryl, $C_{6-10}$ aryloxy, etc., $R^2$ and $R^3$ are hydrogen, $C_{1-6}$ alkyl, $C_{3-10}$ cycloalkyl, etc., $R^4$ is hydrogen, $C_{1-6}$ alkyl, $C_{3-4}$cycloalkyl, etc., m is 0, 1, 2, or 3, L is $CR^7R^8$, $R^7$ and $R^8$ are hydrogen, hydroxy group, $C_{1-4}$ alkyl, $C_{1-4}$ alkoxy, etc., or a pharmaceutically acceptable salt thereof.

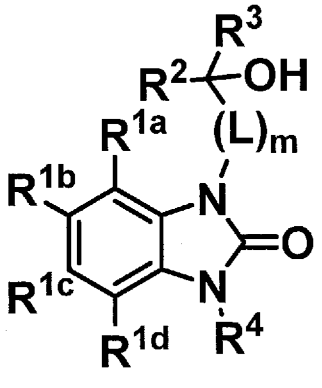

(I)



Printed by Jouve, 75001 PARIS (FR)

## Description

TECHNICAL FIELD

The present invention relates to a medicament for treating or preventing a disease involving Na channel, particularly SCN9A (Nav 1.7), which comprises a novel compound having a benzimidazolone skeleton or a pharmaceutically acceptable salt thereof as an active ingredient. In more detail, it relates to a medicament for treating or preventing a disease such as neuropathic pain, nociceptive pain, inflammatory pain, small-fiber neuropathy, erythromelalgia, paroxysmal extreme pain disorder, dysuria, and multiple sclerosis.

BACKGROUND ART

Voltage-dependent Na channel $\alpha$ subunit that forms pore is known to include 9 kinds at present. Recently, it has been evidenced that the subunit, particularly Nav 1.7 is broadly concerned in the signal transduction of acute and chronic pain.

SCN9A (Nav 1.7) is tetrodotoxin (TTX)-sensitive Na channel localized in the peripheral sensory nerve or sympathetic nerve, which is also referred to as NENA or PN1. Physiologically, Nav 1.7 channel functions to amplify a pain signal (i.e., generate a generator potential) at the sensory nerve ending. In the field of genetic investigation, it has been getting evident that a human whose SCN9A gene mutates to result in loss-of-function shows congenital insensitivity to pain. Reversely, in patients suffering from a severe orphan disease such as erythromelalgia and paroxysmal extreme pain disorder, it is observed that SCN9A gene mutates to result in gain-of-function. Furthermore, it has been reported that approximately 30% of patients suffering from small fiber neuropathy have genetic polymorphism to enhance Nav 1.7 function (Non-Patent Literature 1). And, it is suggested that Nav 1.7 channel function is directly concerned in the hyperexcitability of DRG neuron in patients suffering from pain since the expression level and activity increase in DRG neuron of model animals suffering from chronic pain, and neuropathic pain and inflammatory pain decrease in a knockout experiment (Non-Patent Literature 2).

Patent Literature 1 discloses a benzimidazolone derivative represented by the following formula (A), but the invention described in Patent Literature 1 is directed to an activated factor X inhibitor, thus Patent Literature 1 does not disclose the present invention at all.

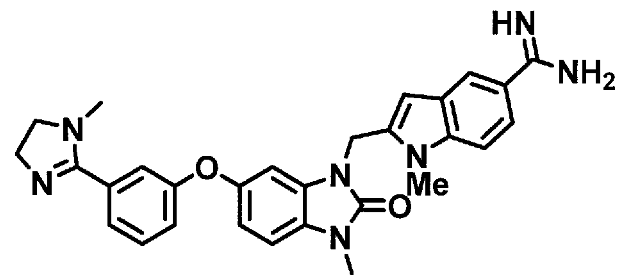

**(A)**

PRIOR ART

[Patent Literature]

[Patent Literature 1] WO 2001/057019

[Non-patent R Literature]

[Non-Patent Literature 1] Nat Rev Neurosci. 14: 49, 2013
[Non-Patent Literature 2] Nat Commun. 3: 791, 2012

Summary of Invention

(Technical Problem)

The purpose of the present invention may be to provide a medicament for treating or preventing a disease involving Nav 1.7, specifically such as neuropathic pain, nociceptive pain, inflammatory pain, small-fiber neuropathy, erythromelalgia, paroxysmal extreme pain disorder, dysuria, and multiple sclerosis.

(Solution to Problem)

The present inventors have conducted intensive studies in an attempt to solve the aforementioned problem and found that a compound having a benzimidazolone ring mentioned below or a pharmaceutically acceptable salt thereof can inhibit the membrane potential change or the Na ion current itself via Na channel in Nav 1.7 gene expressing cell, i.e., the compound or a pharmaceutically acceptable salt thereof is a blocker having a inhibitory activity for Nav 1.7. In addition, the present inventors have found that the derivative is useful as a medicament for treating or preventing a disease such as neuropathic pain, nociceptive pain, inflammatory pain, small-fiber neuropathy, erythromelalgia, and paroxysmal extreme pain disorder, which resulted in the completion of the present invention. Accordingly, the present invention can provide a benzimidazolone compound represented by the following formula (I) (hereinafter, also referred to as "compound represented by formula (I)" or "compound of formula (I)") or a pharmaceutically acceptable salt thereof (hereinafter, also referred to as "compound of the present invention"). The present invention can show as follows.

(Item 1)

A compound of formula (I):

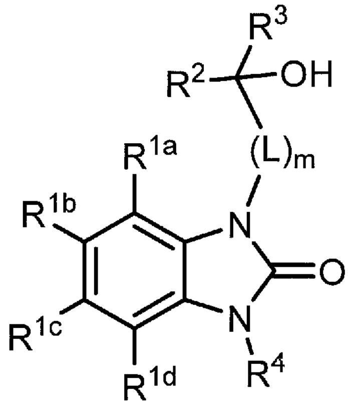

(I)

or a pharmaceutically acceptable salt thereof, wherein

$R^{1a}$, $R^{1b}$, $R^{1c}$, and $R^{1d}$ are independently hydrogen, halogen, $C_{1-4}$ alkyl, $C_{1-4}$ alkoxy (wherein the alkyl and the alkyl moiety in the alkoxy may be independently substituted with 1 to 5 substituents selected independently from the group consisting of halogen, hydroxy group, $C_{1-4}$ alkoxy optionally-substituted with 1 to 3 substituents selected independently from Substituent-group A, $C_{3-7}$ cycloalkyl optionally-substituted with 1 to 3 substituents selected independently from Substituent-group B, $C_{3-7}$ cycloalkoxy optionally-substituted with 1 to 3 substituents selected independently from Substituent-group B, and 3- to 7-membered non-aromatic heterocyclyl optionally-substituted with 1 to 3 substituents selected independently from Substituent-group B), $C_{3-7}$ cycloalkyl, $C_{3-7}$ cycloalkoxy (wherein the cycloalkyl and the cycloalkyl moiety in the cycloalkoxy may be independently substituted with 1 to 5 substituents selected independently from the group consisting of halogen, hydroxy group, $C_{1-4}$ alkyl optionally-substituted with 1 to 3 substituents selected independently from Substituent-group A, $C_{1-4}$ alkoxy optionally-substituted with 1 to 3 substituents selected independently from Substituent-group A, $C_{3-7}$ cycloalkyl optionally-substituted with 1 to 3 substituents selected independently from Substituent-group B, and $C_{3-7}$ cycloalkoxy optionally-substituted with 1 to 3 substituents selected independently from Substituent-group B), $C_{6-10}$ aryl, $C_{6-10}$ aryloxy, 5- to 12-membered heteroaryl, or 5- to 12-membered heteroaryloxy (wherein the aryl and the aryl moiety in the aryloxy, and the heteroaryl and the heteroaryl moiety in the heteroaryloxy may be independently substituted with 1 to 5 substituents selected independently from the group consisting of halogen, cyano, $C_{1-4}$ alkyl optionally-substituted with 1 to 3 substituents selected independently from Substituent-group A, $C_{1-4}$ alkoxy optionally-substituted with 1 to 3 substituents selected

independently from Substituent-group A, $C_{3-7}$ cycloalkyl optionally-substituted with 1 to 3 substituents selected independently from Substituent-group B, $C_{3-7}$ cycloalkoxy optionally-substituted with 1 to 3 substituents selected independently from Substituent-group B, and 3- to 7-membered non-aromatic heterocyclyl optionally-substituted with 1 to 3 substituents selected independently from Substituent-group B), provided that at least one of $R^{1a}$, $R^{1b}$, $R^{1c}$ and $R^{1d}$ is the above $C_{6-10}$ aryl, $C_{6-10}$ aryloxy, 5- to 12-membered heteroaryl or 5- to 12-membered heteroaryloxy,

$R^2$ and $R^3$ are independently hydrogen, $C_{1-6}$ alkyl (which may be independently substituted with 1 to 5 substituents selected independently from the group consisting of cyano, halogen, hydroxy group, $C_{1-4}$ alkoxy optionally-substituted with 1 to 3 substituents selected independently from Substituent-group A, $C_{3-7}$ cycloalkyl optionally-substituted with 1 to 3 substituents selected independently from Substituent-group B, and $C_{3-7}$ cycloalkoxy optionally-substituted with 1 to 3 substituents selected independently from Substituent-group B), or $C_{3-10}$ cycloalkyl,

$R^4$ is hydrogen, $C_{1-4}$ alkyl or $C_{3-4}$ cycloalkyl, wherein the $C_{1-4}$ alkyl and the $C_{3-4}$ cycloalkyl may be substituted with 1 - 5 the same or different halogen atoms,

m is 1, 2, or 3,

L is $CR^7R^8$ provided that when m is 2 or 3, each $CR^7R^8$ is independently the same or different,

$R^7$ and $R^8$ are independently hydrogen, hydroxy group, $C_{1-4}$ alkyl, $C_{1-4}$ alkoxy (wherein the alkyl and the alkyl moiety in the alkoxy may be independently substituted with 1 to 3 substituents selected independently from the group consisting of halogen, hydroxy group, $C_{1-4}$ alkoxy optionally-substituted with 1 to 3 substituents selected independently from Substituent-group A, $C_{3-7}$ cycloalkyl optionally-substituted with 1 to 3 substituents selected independently from Substituent-group B, $C_{3-7}$ cycloalkoxy optionally-substituted with 1 to 3 substituents selected independently from Substituent-group B, and 3- to 7-membered non-aromatic heterocyclyl optionally-substituted with 1 to 3 substituents selected independently from Substituent-group B), $C_{3-7}$ cycloalkyl, or $C_{3-7}$ cycloalkoxy (wherein the cycloalkyl and the cycloalkyl moiety in the cycloalkoxy may be independently substituted with 1 to 3 substituents selected independently from the group consisting of halogen, hydroxy group, $C_{1-4}$ alkyl optionally-substituted with 1 to 3 substituents selected independently from Substituent-group A, $C_{1-4}$ alkoxy optionally-substituted with 1 to 3 substituents selected independently from Substituent-group A, $C_{3-7}$ cycloalkyl optionally-substituted with 1 to 3 substituents selected independently from Substituent-group B, and $C_{3-7}$ cycloalkoxy optionally-substituted with 1 to 3 substituents selected independently from Substituent-group B), or

in $R^2$, $R^3$, and the hydroxy group bound to the carbon atom which is connected to $R^2$ and $R^3$,

$R^2$ and $R^3$ may be combined together with the carbon atom to which they are attached to form the following group of formula (II) with the hydroxy group

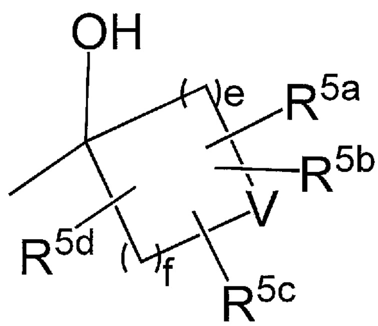

(II)

in formula (II),

e and f are independently 1, 2 or 3,

V is single bond or oxygen atom,

$R^{5a}$, $R^{5b}$, $R^{5c}$, and $R^{5d}$ are independently hydrogen, halogen, hydroxy group, $C_{1-4}$ alkyl, or $C_{1-4}$ alkoxy, wherein the .alkyl and the alkyl moiety in the alkoxy may be independently substituted with 1 to 3 substituents selected independently from the group consisting of halogen, hydroxy group, $C_{1-4}$ alkoxy optionally-substituted with 1 to 3 substituents selected independently from Substituent-group A, $C_{3-7}$ cycloalkyl optionally-substituted with 1 to 3 substituents selected independently from Substituent-group B, $C_{3-7}$ cycloalkoxy optionally-substituted with 1 to 3 substituents selected independently from Substituent-group B, and 3- to 7-membered non-aromatic heterocyclyl optionally-substituted with 1 to 3 substituents selected independently from Substituent-group B, or

in $R^2$, $R^3$, the hydroxy group bound to the carbon atom which is connected to $R^2$ and $R^3$, and $CR^7R^8$ in L,

$R^2$ and $R^7$ may be combined together with the carbon atom to which they are attached to form the following group of formula (III) with $R^3$, the hydroxy group and $R^8$

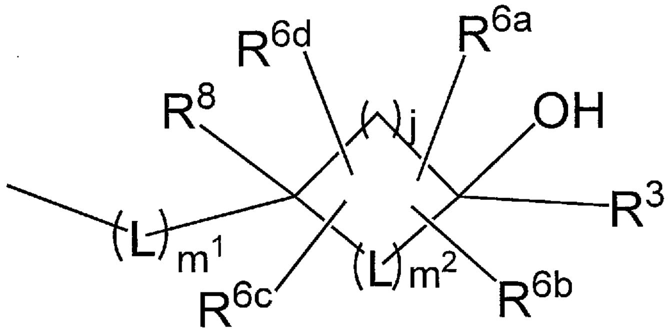

(III)

in formula (III),

$m^1$ is 0 or 1,
$m^2$ is 0 or 1 and j is 1, 2, 3 or 4 when $m^1$ is 1, or
$m^2$ is 0, 1 or 2 and j is 1, 2, 3 or 4 when $m^1$ is 0,
$R^8$ and L are as defined above,
$R^{6a}$, $R^{6b}$, $R^{6c}$, and $R^{6d}$ are independently hydrogen, halogen, hydroxy group, $C_{1-4}$ alkyl, or $C_{1-4}$ alkoxy, wherein the alkyl and the alkyl moiety in the alkoxy may be independently substituted with 1 to 3 substituents selected independently from the group consisting of halogen, hydroxy group, $C_{1-4}$ alkoxy optionally-substituted with 1 to 3 substituents selected independently from Substituent-group A, $C_{3-7}$ cycloalkyl optionally-substituted with 1 to 3 substituents selected independently from Substituent-group B, $C_{3-7}$ cycloalkoxy optionally-substituted with 1 to 3 substituents selected independently from Substituent-group B, and 3- to 7-membered non-aromatic heterocyclyl optionally-substituted with 1 to 3 substituents selected independently from Substituent-group B,
Substituent-group A is independently halogen, hydroxy group, $C_{1-4}$ alkoxy, $C_{3-7}$ cycloalkyl, or $C_{3-7}$ cycloalkoxy,
Substituent-group B is independently halogen, hydroxy group, $C_{1-4}$ alkyl, $C_{1-4}$ alkoxy, $C_{3-7}$ cycloalkyl, or $C_{3-7}$ cycloalkoxy,
provided that the following compounds are excluded:

5-(2-butyl-1-oxo-1,2,3,4-tetrahydropyrrolo[1,2-a]pyrazin-6-yl)-1-(2-hydroxyethyl)-1,3-dihydro-2H-benzimidazol-2-one,
3-[3-methyl-2-oxo-1-(3,3,3-trifluoro-2-hydroxypropyl)-2,3-dihydro-1H-benzimidazol-5-yl]pyridine-4-carbonitrile, and
3-[3-methyl-2-oxo-1-(3,3,3-trifluoro-2-hydroxy-2-methylpropyl)-2,3-dihydro-1H-benzimidazol-5-yl]pyridine-4-carbonitrile.

(Item 2)

The compound of Item 1 or a pharmaceutically acceptable salt thereof, wherein
$R^{1a}$, $R^{1b}$, $R^{1c}$, and $R^{1d}$ are independently, hydrogen, halogen, $C_{1-4}$ alkyl, $C_{1-4}$ alkoxy (wherein the alkyl and the alkyl moiety in the alkoxy may be independently substituted with 1 to 3, the same or different halogen atoms), $C_{6-10}$ aryl, $C_{6-10}$ aryloxy, 5- to 12-membered heteroaryl, or 5- to 12-membered heteroaryloxy (wherein the aryl and the aryl moiety in the aryloxy, and the heteroaryl and the heteroaryl moiety in the heteroaryloxy may be independently substituted with 1 to 3 substituents selected independently from the group consisting of halogen, cyano, $C_{1-4}$ alkyl optionally-substituted with 1 to 3 substituents selected independently from Substituent-group A, and $C_{1-4}$ alkoxy optionally-substituted with 1 to 3 substituents selected independently from Substituent-group A).

(Item 3)

The compound of Item 1 or 2 or a pharmaceutically acceptable salt thereof, wherein
$R^{1a}$, $R^{1b}$, $R^{1c}$, and $R^{1d}$ are independently, hydrogen, $C_{6-10}$ aryl, $C_{6-10}$ aryloxy, 5- to 12-membered heteroaryl, or 5- to 12-membered heteroaryloxy, wherein the aryl and the aryl moiety in the aryloxy, and the heteroaryl and the heteroaryl moiety in the heteroaryloxy may be independently substituted with 1 to 3 substituents selected independently from the group consisting of halogen, cyano, $C_{1-4}$ alkyl optionally-substituted with 1 to 3 substituents selected independently from Substituent-group A, and $C_{1-4}$ alkoxy optionally-substituted with 1 to 3 substituents selected independently from Substituent-group A.

(Item 4)

The compound of any one of Items 1 to 3 or a pharmaceutically acceptable salt thereof, wherein $R^{1a}$ and $R^{1d}$ are hydrogen.

(Item 5)

The compound of any one of Items 1 to 4 or a pharmaceutically acceptable salt thereof, wherein
$R^{1b}$ or $R^{1c}$ is $C_{6-10}$ aryl, $C_{6-10}$ aryloxy, 5- to 12-membered heteroaryl, or 5- to 12-membered heteroaryloxy, wherein the aryl and the aryl moiety in the aryloxy, and the heteroaryl and the heteroaryl moiety in the heteroaryloxy may be independently substituted with 1 to 3 substituents selected independently from the group consisting of halogen, $C_{1-4}$ alkyl optionally-substituted with 1 to 3 substituents selected independently from Substituent-group A, and $C_{1-4}$ alkoxy optionally-substituted with 1 to 3 substituents selected independently from Substituent-group A.

(Item 6)

The compound of any one of Items 1 to 5 or a pharmaceutically acceptable salt thereof, wherein
$R^2$ and $R^3$ are independently hydrogen or $C_{1-6}$ alkyl which may be independently substituted with 1 to 5 substituents selected independently from the group consisting of halogen, hydroxy group, and $C_{1-4}$ alkoxy optionally-substituted with 1 to 3 substituents selected independently from Substituent-group A, or
in $R^2$, $R^3$, and the hydroxy group bound to the carbon atom which is connected to $R^2$ and $R^3$,
$R^2$ and $R^3$ may be combined together with the carbon atom to which they are attached to form the following group of formula (IIa) with the hydroxy group

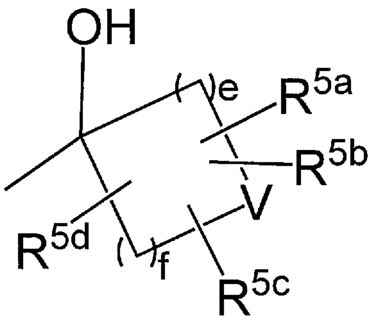

(IIa)

in formula (IIa),

e and f are independently 1 or 2,
V is as defined in Item 1, and
$R^{5a}$, $R^{5b}$, $R^{5c}$, and $R^{5d}$ are independently hydrogen or halogen, or
in $R^2$, $R^3$, the hydroxy group bound to the carbon atom which is connected to $R^2$ and $R^3$, and $CR^7R^8$ in L,
$R^2$ and $R^7$ may be combined together with the carbon atom to which they are attached to form the following group of formula (IIIa) with $R^3$, the hydroxy group and $R^8$

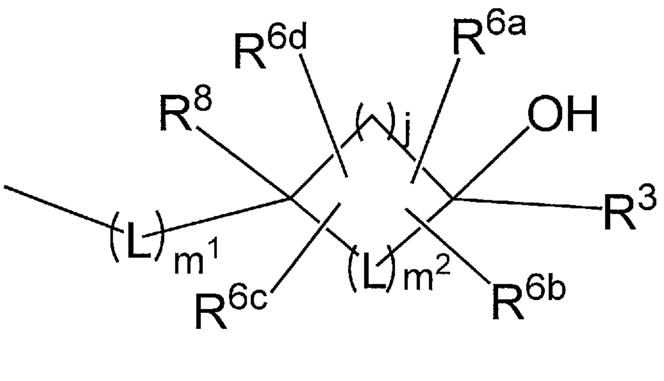

(IIIa)

in formula (IIIa),

$m^1$ is 0,
$m^2$ is 1 or 2, j is 1 or 2,
$R^8$ is hydrogen,
L is as defined in Item 1,
$R^{6a}$, $R^{6b}$, $R^{6c}$, and $R^{6d}$ are independently hydrogen or halogen.

(Item 7)

The compound of any one of Items 1 to 6 or a pharmaceutically acceptable salt thereof, wherein
$R^7$ and $R^8$ are independently hydrogen or $C_{1-4}$ alkyl which may be substituted with 1 to 3 substituents selected independently from the group consisting of halogen, hydroxy group, $C_{1-4}$ alkoxy optionally-substituted with 1 to 3 substituents selected independently from Substituent-group A, $C_{3-7}$ cycloalkyl optionally-substituted with 1 to 3 substituents selected independently from Substituent-group B, $C_{3-7}$ cycloalkoxy optionally-substituted with 1 to 3 substituents selected independently from Substituent-group B, and 3- to 7-membered non-aromatic heterocyclyl optionally-substituted with 1 to 3 substituents selected independently from Substituent-group B, and
m is 1 or 2.

(Item 8)

The compound of any one of Items 1 to 7 or a pharmaceutically acceptable salt thereof, wherein $R^7$ and $R^8$ are hydrogen, and m is 1.

(Item 9)

The compound of any one of Items 1 to 8 or a pharmaceutically acceptable salt thereof, wherein $R^4$ is hydrogen or $C_{1-4}$ alkyl optionally-substituted with 1 to 5 the same or different halogen atoms.

(Item 10)

The compound of any one of Items 1 to 9 or a pharmaceutically acceptable salt thereof, wherein
$R^{1b}$ is $C_{6-10}$ aryl, $C_{6-10}$ aryloxy, 5- to 12-membered heteroaryl, or 5- to 12-membered heteroaryloxy, wherein the aryl and the aryl moiety in the aryloxy, and the heteroaryl and the heteroaryl moiety in the heteroaryloxy may be independently substituted with 1 to 3 substituents selected independently from the group consisting of halogen, cyano, $C_{1-4}$ alkyl optionally-substituted with 1 to 3 substituents selected independently from Substituent-group A, and $C_{1-4}$ alkoxy optionally-substituted with 1 to 3 substituents selected independently from Substituent-group A.

(Item 11)

The compound of any one of Items 1 to 9 or a pharmaceutically acceptable salt thereof, wherein
$R^{1c}$ is $C_{6-10}$ aryl, $C_{6-10}$ aryloxy, 5- to 12-membered heteroaryl, or 5- to 12-membered heteroaryloxy, wherein the aryl and the aryl moiety in the aryloxy, and the heteroaryl and the heteroaryl moiety in the heteroaryloxy may be independently substituted with 1 to 3 substituents selected independently from the group consisting of halogen, cyano, $C_{1-4}$ alkyl optionally-substituted with 1 to 3 substituents selected independently from Substituent-group A, and $C_{1-4}$ alkoxy optionally-substituted with 1 to 3 substituents selected independently from Substituent-group A.

(Item 12)

The compound of Item 1 or a pharmaceutically acceptable salt thereof, which is selected from the following compounds:

Example 1: 1-(2-hydroxy-2-methylpropyl)-6-{[5-(trifluoromethyl)pyridin-2-yl]oxy}-1,3-dihydro-2H-benzimidazol-2-one,
Example 2: 1-[(3-hydroxyoxetan-3-yl)methyl]-6-{[5-(trifluoromethyl)pyridin-2-yl]oxy}-1,3-dihydro-2H-benzimidazol-2-one,
Example 3: 1-[(3-hydroxyoxetan-3-yl)methyl]-6-{[6-(trifluoromethyl)pyridin-3-yl]oxy}-1,3-dihydro-2H-benzimidazol-2-one,

Example 4: 1-(cis-4-hydroxycyclohexyl)-6-{[5-(trifluoromethyl)pyridin-2-yl]oxy}-1,3-dihydro-2H-benzimidazol-2-one,
Example 5: 1-[(3-hydroxyoxetan-3-yl)methyl]-6-[4-(trifluoromethoxy)phenoxy]-1,3-dihydro-2H-benzimidazol-2-one,
Example 6: 6-(4-fluorophenoxy)-1-(2-hydroxy-2-methylpropyl)-1,3-dihydro-2H-benzimidazol-2-one,
Example 7: 6-(4-fluorophenoxy)-1-[(3-hydroxyoxetan-3-yl)methyl]-1,3-dihydro-2H-benzimidazol-2-one,
Example 8: 6-(4-chlorophenoxy)-1-[(3-hydroxyoxetan-3-yl)methyl]-1,3-dihydro-2H-benzimidazol-2-one,
Example 9: 1-[(2S)-3-hydroxy-3-methylbutan-2-yl]-6-{[5-(trifluoromethyl)pyridin-2-yl]oxy}-1,3-dihydro-2H-benzimidazol-2-one,
Example 10: 6-(4-fluorophenoxy)-1-[(2S)-3-hydroxy-3-methylbutan-2-yl]-1,3-dihydro-2H-benzimidazol-2-one,
Example 11: 6-(4-fluorophenoxy)-1-(cis-4-hydroxycyclohexyl)-1,3-dihydro-2H-benzimidazol-2-one,
Example 12: 1-ethyl-3-(2-hydroxy-2-methylpropyl)-5-{[5-(trifluoromethyl)pyridin-2-yl]oxy}-1,3-dihydro-2H-benzimidazol-2-one,
Example 13: 3-(2-hydroxy-2-methylpropyl)-1-methyl-5-{[5-(trifluoromethyl)pyridin-2-yl]oxy}-1,3-dihydro-2H-benzimidazol-2-one,
Example 14: 3-[(3-hydroxyoxetan-3-yl)methyl]-1-methyl-5-{[5-(trifluoromethyl)pyridin-2-yl]oxy}-1,3-dihydro-2H-benzimidazol-2-one,
Example 15: 3-[(3-hydroxyoxetan-3-yl)methyl]-1-methyl-5-{[6-(trifluoromethyl)pyridin-3-yl]oxy}-1,3-dihydro-2H-benzimidazol-2-one,
Example 16: 5-(4-fluorophenoxy)-3-[(3-hydroxyoxetan-3-yl)methyl]-1-methyl-1,3-dihydro-2H-benzimidazol-2-one,
Example 17: 3-[(3-hydroxyoxetan-3-yl)methyl]-1-methyl-5-[4-(trifluoromethoxy)phenoxy]-1,3-dihydro-2H-benzimidazol-2-one,
Example 18: 5-(4-chlorophenoxy)-3-[(3-hydroxyoxetan-3-yl)methyl]-1-methyl-1,3-dihydro-2H-benzimidazol-2-one,
Example 19: 1-(2-hydroxy-2-methylpropyl)-6-[6-(trifluoromethyl)pyridin-3-yl]-1,3-dihydro-2H-benzimidazol-2-one,
Example 20: 1-(2-hydroxy-2-methylpropyl)-6-[5-(trifluoromethyl)pyridin-2-yl]-1,3-dihydro-2H-benzimidazol-2-one,
Example 21: 1-(2-hydroxy-2-methylpropyl)-6-[4-(trifluoromethyl)phenyl]-1,3-dihydro-2H-benzimidazol-2-one,
Example 22: 6-(4-fluorophenyl)-1-(2-hydroxy-2-methylpropyl)-1,3-dihydro-2H-benzimidazol-2-one,
Example 23: 1-(2-hydroxy-2-methylpropyl)-5-[6-(trifluoromethyl)pyridin-3-yl]-1,3-dihydro-2H-benzimidazol-2-one,
Example 24: 5-(4-fluorophenyl)-1-(2-hydroxy-2-methylpropyl)-1,3-dihydro-2H-benzimidazol-2-one,
Example 25: 1-(2-hydroxy-2-methylpropyl)-5-[4-(trifluoromethoxy)phenoxy]-1,3-dihydro-2H-benzimidazol-2-one,
Example 26: 1-(2-hydroxy-2-methylpropyl)-5-{[5-(trifluoromethyl)pyridin-2-yl]oxy}-1,3-dihydro-2H-benzimidazol-2-one,
Example 27: 1-[(3-hydroxyoxetan-3-yl)methyl]-5-[4-(trifluoromethoxy)phenoxy]-1,3-dihydro-2H-benzimidazol-2-one, and
Example 28: 1-[(3-hydroxyoxetan-3-yl)methyl]-3-methyl-5-[4-(trifluoromethoxy)phenoxy]-1,3-dihydro-2H-benzimidazol-2-one.

(Item 13)

The compound of Item 1 or a pharmaceutically acceptable salt thereof, which is selected from the following compounds:

Example 1: 1-(2-hydroxy-2-methylpropyl)-6-{[5-(trifluoromethyl)pyridin-2-yl]oxy}-1,3-dihydro-2H-benzimidazol-2-one,
Example 5: 1-[(3-hydroxyoxetan-3-yl)methyl]-6-[4-(trifluoromethoxy)phenoxy]-1,3-dihydro-2H-benzimidazol-2-one,
Example 8: 6-(4-chlorophenoxy)-1-[(3-hydroxyoxetan-3-yl)methyl]-1,3-dihydro-2H-benzimidazol-2-one,
Example 10: 6-(4-fluorophenoxy)-1-[(2S)-3-hydroxy-3-methylbutan-2-yl]-1,3-dihydro-2H-benzimidazol-2-one,
Example 11: 6-(4-fluorophenoxy)-1-(cis-4-hydroxycyclohexyl)-1,3-dihydro-2H-benzimidazol-2-one,
Example 17: 3-[(3-hydroxyoxetan-3-yl)methyl]-1-methyl-5-[4-(trifluoromethoxy)phenoxy]-1,3-dihydro-2H-benzimidazol-2-one,
Example 20: 1-(2-hydroxy-2-methylpropyl)-6-[5-(trifluoromethyl)pyridin-2-yl]-1,3-dihydro-2H-benzimidazol-2-one,
Example 21: 1-(2-hydroxy-2-methylpropyl)-6-[4-(trifluoromethyl)phenyl]-1,3-dihydro-2H-benzimidazol-2-one,
Example 23: 1-(2-hydroxy-2-methylpropyl)-5-[6-(trifluoromethyl)pyridin-3-yl]-1,3-dihydro-2H-benzimidazol-2-one,
Example 24: 5-(4-fluorophenyl)-1-(2-hydroxy-2-methylpropyl)-1,3-dihydro-2H-benzimidazol-2-one,
Example 26: 1-(2-hydroxy-2-methylpropyl)-5-{[5-(trifluoromethyl)pyridin-2-yl]oxy}-1,3-dihydro-2H-benzimidazol-2-one, and
Example 28: 1-[(3-hydroxyoxetan-3-yl)methyl]-3-methyl-5-[4-(trifluoromethoxy)phenoxy]-1,3-dihydro-2H-benzimidazol-2-one.

(Item 14)

A pharmaceutical combination comprising the compound of any one of Items 1 to 13 or a pharmaceutically acceptable salt thereof.

(Item 15)

A medicament for treating a disease involving Nav 1.7 (SCN9A), comprising the compound of any one of Items 1 to 13 or a pharmaceutically acceptable salt thereof as an active ingredient.

(Item 16)

A medicament for treating neuropathic pain, nociceptive pain, inflammatory pain, small-fiber neuropathy, erythromelalgia, paroxysmal extreme pain disorder, dysuria, or multiple sclerosis, which comprises the compound of any one of Items 1 to 13 or a pharmaceutically acceptable salt thereof as an active ingredient.

(Item 17)

A pharmaceutical combination comprising the compound of any one of Items 1 to 13 or a pharmaceutically acceptable salt thereof, and at least one drug selected from the group consisting of an antiepileptic agent, an antidepressive agent, a narcotic analgesic, an anti-inflammatory agent, a reductase inhibitor, and a prostaglandin derivative drug.

(Item 18)

Use of the compound of any one of Items 1 to 13 or a pharmaceutically acceptable salt thereof in the manufacture of a medicament for treating neuropathic pain, nociceptive pain, inflammatory pain, small-fiber neuropathy, erythromelalgia, paroxysmal extreme pain disorder, dysuria, or multiple sclerosis.

(Item 19)

A method for treating neuropathic pain, nociceptive pain, inflammatory pain, small-fiber neuropathy, erythromelalgia, paroxysmal extreme pain disorder, dysuria, or multiple sclerosis, which comprises administering a therapeutically effective amount of the compound of any one of Items 1 to 13 or a pharmaceutically acceptable salt thereof to a mammal in need thereof.

(Effect of Invention)

The present invention provides a Nav 1.7 blocker comprising a novel benzimidazolone compound or a pharmaceutically acceptable salt thereof. The compounds of the present invention are useful as a medicament for treating or preventing a disease involving Nav 1.7 (SCN9A), namely, the compounds are applicable to a patient suffering from neuropathic pain, nociceptive pain, inflammatory pain, small-fiber neuropathy, erythromelalgia, paroxysmal extreme pain disorder, and the like.

Description of Embodiments

Hereinafter, the present invention is explained in detail. In the description, the number of carbon atoms in the definition of "substituents" can indicates, for example, "$C_{1-6}$". The specific definition "$C_{1-6}$ alkyl" means an alkyl group having 1 to 6 carbon atoms. In the present description, a substituent group which is not accompanied with "optionally-substituted" or "substituted" means an "unsubstituted" substituent group. For example, "$C_{1-6}$ alkyl" means "unsubstituted $C_{1-6}$ alkyl".

The substituent groups in the present description may be sometimes expressed without the term "group". In case that "optionally-substituted" is used in the definition of substituent groups, the number of the substituting groups is not limited as long as the substitutions are available, i.e., it is one or more. It means that the possible number of substituting groups is the substitution-available number on carbon atoms or carbon-nitrogen atoms in a substituent group which are acceptable for substitution. Unless otherwise specified, the definition of each substituent group also extends over the case of partially-including the substituent group or the case of the substituent group substituting another substituent group.

Unless otherwise specified, the binding site of substituent groups is not limited as long as the site is available

to be bound.

The "halogen" includes, for example, fluorine, chlorine, bromine, and iodine, preferably fluorine and chlorine.

The "$C_{1-2}$ alkyl" means a saturated hydrocarbon group having 1 to 2 carbon atoms, the "$C_{1-3}$ alkyl" means a saturated straight or branched chain hydrocarbon group having 1 to 3 carbon atoms, the "$C_{1-4}$ alkyl" means a saturated straight or branched chain hydrocarbon group having 1 to 4 carbon atoms, and the "$C_{1-6}$ alkyl" means a saturated straight or branched chain hydrocarbon group having 1 to 6 carbon atoms. The "$C_{1-2}$ alkyl" includes, for example, methyl and ethyl; the "$C_{1-3}$ alkyl" includes, for example, propyl and isopropyl, besides the above alkyl; the "$C_{1-4}$ alkyl" includes, for example, butyl, isobutyl, sec-butyl, and tert-butyl, besides the above alkyl; and the "$C_{1-6}$ alkyl" includes, for example, pentyl, isopentyl, neopentyl, 1-ethylpropyl, hexyl, and a structural isomer thereof, besides the above alkyl. Preferred examples of the "$C_{1-6}$ alkyl" or "$C_{1-4}$ alkyl" include "$C_{1-3}$ alkyl", and more preferably methyl and ethyl.

The "$C_{3-7}$ cycloalkyl" means a non-aromatic cyclic hydrocarbon group (i.e., saturated hydrocarbon group and partially-unsaturated hydrocarbon group) having 3 to 7 carbon atoms, and the "$C_{3-10}$ cycloalkyl" means a non-aromatic cyclic hydrocarbon group (i.e., saturated hydrocarbon group and partially-unsaturated hydrocarbon group) having 3 to 10 carbon atoms. The "$C_{3-7}$ cycloalkyl" and the "$C_{3-10}$ cycloalkyl" also include a bridged one. The "$C_{3-7}$ cycloalkyl" includes, for example, cyclopropyl, cyclobutyl, cyclopentyl, cyclohexyl, cyclopentenyl, cyclohexenyl, and cycloheptyl. The "$C_{3-10}$ cycloalkyl" includes, for example, cyclooctyl and adamantyl, besides the above, preferably, "$C_{3-7}$ cycloalkyl".

The "$C_{3-7}$ cycloalkyl" and the "$C_{3-10}$ cycloalkyl" also include a bi-cyclic condensed ring in which the "$C_{3-7}$ cycloalkyl" and "$C_{3-10}$ cycloalkyl" are fused with benzene or a 5- or 6-membered ring having one heteroatom selected from nitrogen, sulfur, or oxygen atom, or two or more (for example, 2 to 4) the same or different heteroatoms thereof (for example, "5- or 6-membered mono-cyclic heteroaryl" mentioned below, and 5- or 6-membered ring in "3- to 7-membered non-aromatic heterocyclyl" mentioned below), respectively. Examples of the bi-cyclic condensed ring include groups of the following formulae.

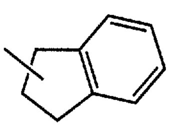 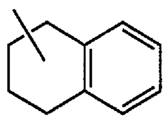 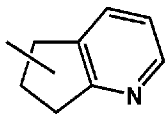 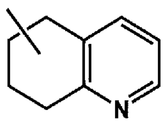 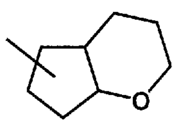 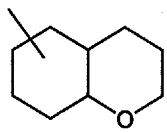

The "$C_{6-10}$ aryl" used herein means an aromatic hydrocarbon group having 6 - 10 carbon atoms, preferably phenyl. The "$C_{6-10}$ aryl" includes, for example, phenyl, 1-naphthyl, and 2-naphthyl.

The "$C_{6-10}$ aryl" also includes a condensed ring in which "phenyl" is fused with a 5- or 6-membered ring having one heteroatom selected from nitrogen, sulfur, or oxygen atom, or two or more (for example, 2 to 4) the same or different heteroatoms thereof (for example, "5- or 6-membered mono-cyclic heteroaryl" mentioned below, and 5- or 6-membered ring in "3- to 7-membered non-aromatic heterocyclyl" mentioned below), or a 5- to 7-membered cycloalkyl ring (for example, cyclopentane, cyclohexane and cycloheptane). Examples of the condensed ring include groups of the following formulae.

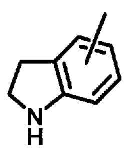 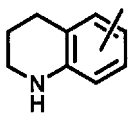 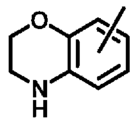 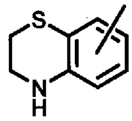 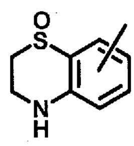 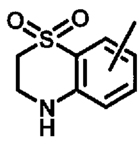

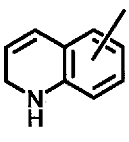 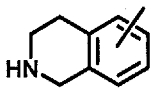 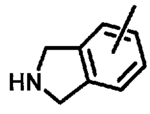 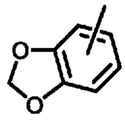 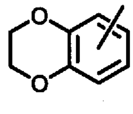 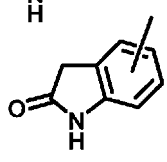

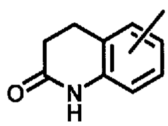 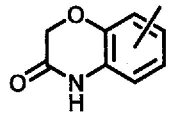 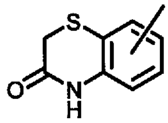 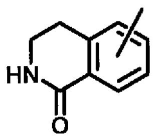 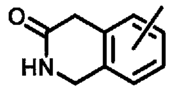 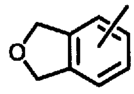

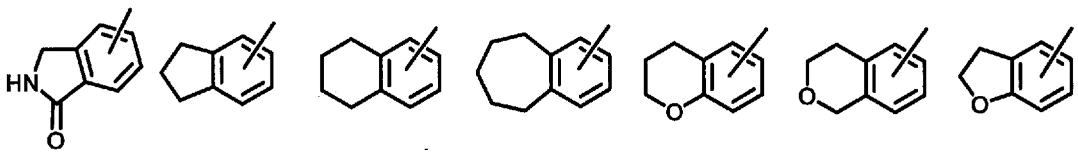

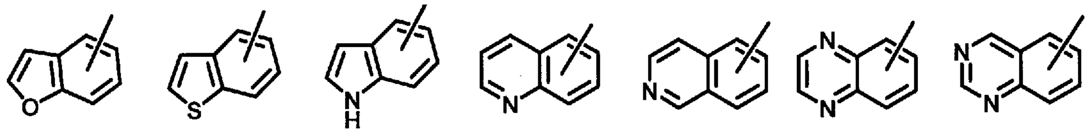

The "5- to 12-membered heteroaryl" means a 5- to 12-membered mono- or multiple-cyclic aromatic group having one heteroatom selected from nitrogen, sulfur, or oxygen atom, or two or more (for example, 2 to 4) the same or different heteroatoms thereof, besides carbon atoms as the ring atoms, preferably, "5- or 6-membered mono-cyclic heteroaryl". The "5- or 6-membered mono-cyclic heteroaryl" means a 5- or 6-membered mono-cyclic aromatic group within the "5- to 12-membered heteroaryl".

The multiple-cyclic heteroaryl in the "5- to 12-membered heteroaryl" includes, for example, a condensed ring in which two the same or different mono-cyclic heteroaryls are fused, or a mono-cyclic heteroaryl and an aromatic ring (for example, benzene) or a non-aromatic ring (for example, cyclohexane) are fused.

The "5- to 12-membered heteroaryl" includes, for example, groups of the formulae shown below. Preferably, the "5- to 12-membered heteroaryl" includes pyrazolyl, imidazolyl, pyridyl, pyrimidinyl, pyrazinyl, and pyridazinyl. Another embodiment includes, preferably, benzofuranyl in which the binding site is on the heteroaryl (furan) ring, pyridyl, pyrimidinyl, pyrazinyl, and pyridazinyl. Examples of the "5- or 6-membered mono-cyclic heteroaryl" include mono-cyclic groups out of the groups of the following formulae.

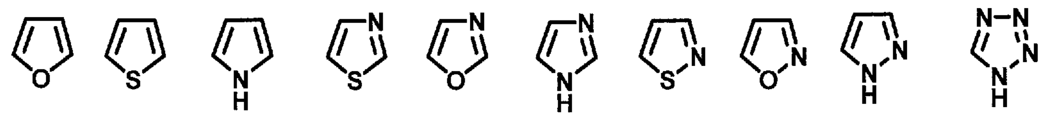

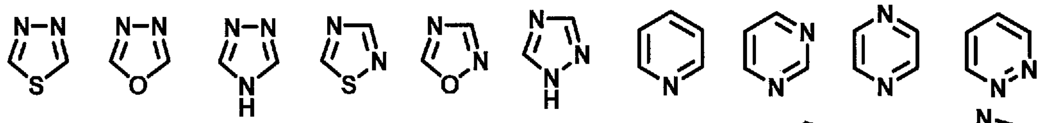

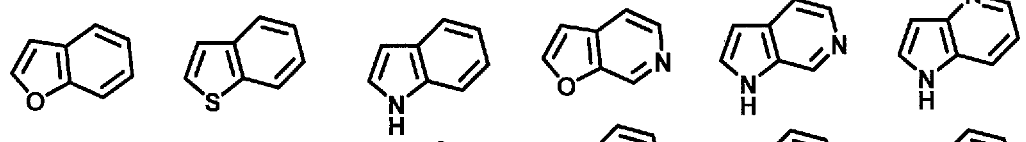

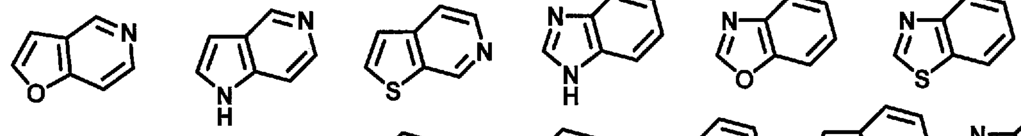

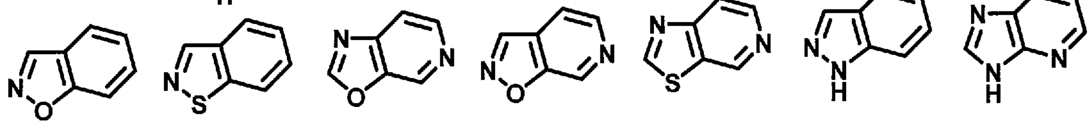

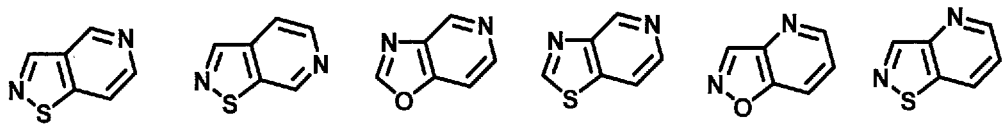

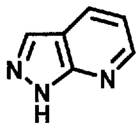 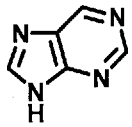 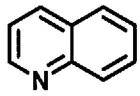 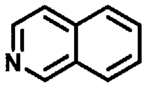 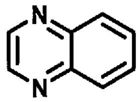 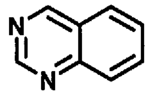

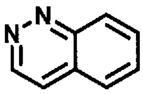 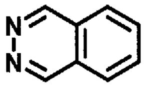 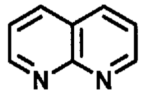 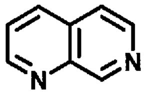 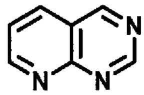 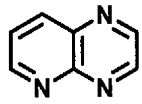

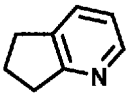 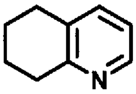 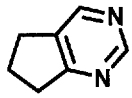 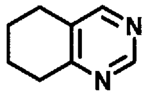 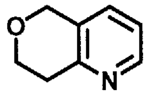 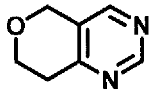

The "3- to 7-membered non-aromatic heterocyclyl" means 3- to 7-membered cyclic group having one heteroatom selected from nitrogen, oxygen, or sulfur atom, or two or more (for example, 2 to 4, preferably 2 to 3) the same or different heteroatoms thereof, besides carbon atoms as the ring atoms. The heterocyclyl is non-aromatic, which may be a saturated one or a partially-unsaturated one. Preferred one thereof is a saturated heterocyclyl, more preferably 5- or 6-membered saturated heterocyclyl. The "3- to 7-membered non-aromatic heterocyclyl" includes, for example, oxetanyl, azetidinyl, pyranyl, tetrahydrofuryl, pyrrolidinyl, pyrazolidinyl, imidazolidinyl, piperidinyl, morpholinyl, thiomorpholinyl, dioxothiomorpholinyl, hexamethyleneiminyl, oxazolidinyl, thiazolidinyl, imidazolidinyl, oxoimidazolidinyl, dioxoimidazolidinyl, oxo-oxazolidinyl, dioxo-oxazolidinyl, dioxothiazolidinyl, tetrahydropyranyl, and tetrahydropyridinyl, and preferably pyranyl, tetrahydrofuryl, pyrrolidinyl, piperidinyl, and morpholinyl.

The "3- to 7-membered non-aromatic heterocyclyl" also includes a condensed ring in which the 3- to 7-membered non-aromatic heterocyclyl is fused with benzene or a 6-membered heteroaryl (for example, pyridine, pyrimidine or pyridazine). The examples thereof include dihydroindolyl, dihydroisoindolyl, dihydropurinyl, dihydrothiazolopyrimidinyl, dihydrobenzodioxanyl, isoindolinyl, indazolyl, pyrrolopyridinyl, tetrahydroquinolinyl, decahydroquinolinyl, tetrahydroisoquinolinyl, decahydroisoquinolinyl, tetrahydronaphthyridinyl, and tetrahydropyridoazepinyl.

The "$C_{1-2}$ alkoxy" means oxy group substituted with the above "$C_{1-2}$ alkyl", and the "$C_{1-4}$ alkoxy" means oxy group substituted with the above "$C_{1-4}$ alkyl". The "$C_{1-2}$ alkoxy" includes, for example, methoxy and ethoxy, and the "$C_{1-4}$ alkoxy" includes, for example, propoxy, isopropoxy, butoxy, isobutoxy, sec-butoxy, and tert-butoxy, besides the above examples. Preferably, the "$C_{1-4}$ alkoxy" includes methoxy, ethoxy, and isopropoxy.

The "$C_{3-7}$ cycloalkoxy" means oxy group substituted with the above "$C_{3-7}$ cycloalkyl". The "$C_{3-7}$ cycloalkoxy" includes, for example, cyclopropyloxy, cyclobutyloxy, cyclopentyloxy, and cyclohexyloxy, and preferably cyclohexyloxy. The "$C_{5-6}$ cycloalkoxy" means a cycloalkoxy having 5 or 6 carbon atoms within the "$C_{3-7}$ cycloalkoxy".

The "$C_{6-10}$ aryloxy" means oxy group substituted with the above "$C_{6-10}$ aryl". The "$C_{6-10}$ aryloxy" includes, for example, phenyloxy and naphthyloxy, and preferably phenyloxy.

The "5- to 12-membered heteroaryloxy" means oxy group substituted with the above "5- to 12-membered heteroaryl". The "5- to 12-membered heteroaryloxy" includes, for example, pyridyloxy, imidazolyloxy and furyloxy, and preferably pyridyloxy.

In order to disclose the present compound of the above formula (I) in more detail, each symbol used in the formula (I) is further explained below showing preferred examples.

Preferably, $R^{1a}$, $R^{1b}$, $R^{1c}$, and $R^{1d}$ are independently hydrogen, halogen, $C_{1-4}$ alkyl, $C_{1-4}$ alkoxy (wherein the alkyl and the alkyl moiety in the alkoxy may be independently substituted with 1 to 3 substituents selected independently from the group consisting of halogen, hydroxy group, and $C_{1-4}$ alkoxy optionally-substituted with 1 to 3 substituents selected independently from Substituent-group A), $C_{3-7}$ cycloalkyl, $C_{3-7}$ cycloalkoxy (wherein the cycloalkyl and the cycloalkyl moiety in the cycloalkoxy may be independently substituted with 1 to 3 substituents selected independently from the group consisting of halogen, hydroxy group, $C_{1-4}$ alkyl optionally-substituted with 1 to 3 substituents selected independently from Substituent-group A, and $C_{1-4}$ alkoxy optionally-substituted with 1 to 3 substituents selected independently from Substituent-group A), $C_{6-10}$ aryl, $C_{6-10}$ aryloxy, 5- to 12-membered heteroaryl, or 5- to 12-membered heteroaryloxy (wherein the aryl and the aryl moiety in the aryloxy, and the heteroaryl and the heteroaryl moiety in the heteroaryloxy may be independently substituted with 1 to 3 substituents selected independently from the group consisting of halogen, cyano, $C_{1-4}$ alkyl optionally-substituted with 1 to 3 substituents selected independently from Substituent-group A, and $C_{1-4}$ alkoxy optionally-substituted with 1 to 3 substituents selected independently from Substituent-group A). Provided that at least one of $R^{1a}$, $R^{1b}$, $R^{1c}$ and $R^{1d}$ is the above-mentioned $C_{6-10}$ aryl, $C_{6-10}$ aryloxy, 5- to 12-membered heteroaryl, or 5- to 12-membered heteroaryloxy.

More preferably, $R^{1a}$, $R^{1b}$, $R^{1c}$, and $R^{1d}$ are independently hydrogen, halogen, $C_{1-4}$ alkyl, $C_{1-4}$ alkoxy (wherein the alkyl and the alkyl moiety in the alkoxy may be independently substituted with 1 to 3 substituents selected independently from the group consisting of halogen, hydroxy group, and $C_{1-4}$ alkoxy optionally-substituted with 1 to 3 substituents selected independently from Substituent-group A), $C_{6-10}$ aryl, $C_{6-10}$ aryloxy, 5- to 12-membered heteroaryl, or 5- to 12-membered heteroaryloxy (wherein the aryl and the aryl moiety in the aryloxy, and the heteroaryl and the heteroaryl moiety in the heteroaryloxy may be independently substituted with 1 to 3 substituents selected independently from the group consisting of halogen, cyano, $C_{1-4}$ alkyl optionally-substituted with 1 to 3 substituents selected independently from Substituent-group A, and $C_{1-4}$ alkoxy optionally-substituted with 1 to 3 substituents selected independently from Substituent-group A). Provided that at least one of $R^{1a}$, $R^{1b}$, $R^{1c}$ and $R^{1d}$ is the above-mentioned $C_{6-10}$ aryl, $C_{6-10}$ aryloxy, 5- to 12-membered heteroaryl, or 5- to 12-membered heteroaryloxy.

Even more preferably, $R^{1a}$, $R^{1b}$, $R^{1c}$, and $R^{1d}$ are independently hydrogen, halogen, $C_{1-4}$ alkyl, $C_{1-4}$ alkoxy (wherein the alkyl and the alkyl moiety in the alkoxy may be independently substituted with 1 to 3 the same or different halogen atoms), $C_{6-10}$ aryl, $C_{6-10}$ aryloxy, 5- to 12-membered heteroaryl, or 5-to 12-membered heteroaryloxy (wherein the aryl and the aryl moiety in the aryloxy, and the heteroaryl and the heteroaryl moiety in the heteroaryloxy may be independently substituted with 1 to 3 substituents selected independently from the group consisting of halogen, cyano, $C_{1-4}$ alkyl optionally-substituted with 1 to 3 substituents selected independently from Substituent-group A, and $C_{1-4}$ alkoxy optionally-substituted with 1 to 3 substituents selected independently from Substituent-group A). Provided that at least one of $R^{1a}$, $R^{1b}$, $R^{1c}$ and $R^{1d}$ is the above-mentioned $C_{6-10}$ aryl, $C_{6-10}$ aryloxy, 5- to 12-membered heteroaryl, or 5- to 12-membered heteroaryloxy.

In another embodiment of $R^{1a}$, $R^{1b}$, $R^{1c}$, and $R^{1d}$, $R^{1a}$ and $R^{1d}$ are hydrogen; and $R^{1b}$ and $R^{1c}$ are independently hydrogen, $C_{6-10}$ aryl, $C_{6-10}$ aryloxy, 5- to 12-membered heteroaryl, or 5- to 12-membered heteroaryloxy, wherein the aryl and the aryl moiety in the aryloxy, and the heteroaryl and the heteroaryl moiety in the heteroaryloxy may be independently substituted with 1 to 3 substituents selected independently from the group consisting of halogen and optionally-substituted $C_{1-4}$ alkyl. Provided that both of $R^{1b}$ and $R^{1c}$ are not hydrogen.

Preferred examples of $R^{1a}$, $R^{1b}$, $R^{1c}$, and $R^{1d}$ include hydrogen, fluorine, chlorine, methyl, ethyl, isopropyl, isobutyl, cyclopropyl, cyclopentyl, cyclohexyl, methoxy, ethoxy, phenyl, 2-fluorophenyl, 3-fluorophenyl, 4-fluorophenyl, 4-(trifluoromethyl)phenyl, 5-(trifluoromethyl)pyridin-2-yl, 6-(trifluoromethyl)pyridin-3-yl, 3-chlorophenyl, 4-chlorophenyl, phenoxy, 3-fluorophenoxy, 4-fluorophenoxy, 3,4-difluorophenoxy, 3,5-difluorophenoxy, 4-chlorophenoxy, 4-methylphenoxy, 4-(trifluoromethyl)phenoxy, 4-methoxyphenoxy, 4-(trifluoromethoxy)phenoxy, 4-cyanophenoxy, (5-methylpyridin-2-yl)oxy, (5-(trifluoromethyl)pyridin-2-yl)oxy, (6-(trifluoromethyl)pyridin-3-yl)oxy, (5-fluoropyridin-2-yl)oxy, 2-methoxy-4-(trifluoromethyl)phenyl, 2-fluoro-4-(trifluoromethyl)phenyl, 2-chloro-4-(trifluoromethyl)phenyl, 4-(trifluoromethoxy)phenyl, (5-chloropyridin-2-yl)oxy, 2,4-dichlorophenyl, 2-chloro-4-fluorophenoxy, 4-chloro-2-fluorophenoxy, and 2,4-dichlorophenoxy.

More preferred examples of $R^{1a}$, $R^{1b}$, $R^{1c}$, and $R^{1d}$ include hydrogen, fluorine, 4-(trifluoromethyl)phenyl, 5-(trifluoromethyl)pyridin-2-yl, 6-(trifluoromethyl)pyridin-3-yl, 3-fluorophenyl, 4-fluorophenyl, 4-chlorophenyl, 3-fluorophenoxy, 4-fluorophenoxy, 4-chlorophenoxy, 4-methylphenoxy, 4-(trifluoromethyl)phenoxy, 4-(trifluoromethoxy)phenoxy, (5-methylpyridin-2-yl)oxy, (5-(trifluoromethyl)pyridin-2-yl)oxy, (6-(trifluoromethyl)pyridin-3-yl)oxy, 2-methoxy-4-(trifluoromethyl)phenyl, 4-(trifluoromethoxy)phenyl, and (5-chloropyridin-2-yl)oxy.

Preferably, $R^2$ and $R^3$ are independently hydrogen or $C_{1-6}$ alkyl which may be independently substituted with 1 to 5 substituents selected independently from the group consisting of cyano, halogen, hydroxy group, and $C_{1-4}$ alkoxy optionally-substituted with 1 to 3 substituents selected independently from Substituent-group A; more preferably hydrogen or $C_{1-6}$ alkyl optionally-substituted with 1 to 5 halogen atoms.

Preferably, $R^2$ and $R^3$ are, for example, hydrogen, methyl, ethyl, isopropyl, isobutyl, trifluoromethyl, cyclopropyl, cyclopentyl, and cyclohexyl, more preferably hydrogen, methyl, and ethyl.

Additionally, in another preferred embodiment, $R^2$ and $R^3$ may be combined together with the carbon atom to which they are attached to form a ring to give the following group of formula (II) with the hydroxy group attached to the common carbon atom.

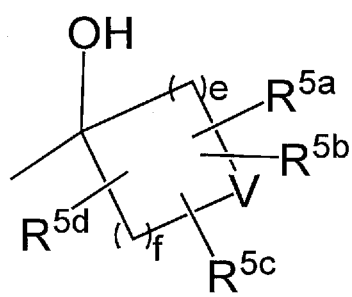

(II)

In the above formula (II),

preferably, e and f are independently 1 or 2,
preferably, $R^{5a}$, $R^{5b}$, $R^{5c}$, and $R^{5d}$ are independently hydrogen or halogen,
preferably, $R^4$ is hydrogen,
preferably, V is oxygen atom.

Additionally, as another preferred embodiment of $R^2$ and $R^3$, in $R^2$, $R^3$, the hydroxy group bound to the carbon atom which is connected to $R^2$ and $R^3$, and $CR^7R^8$ in L,

$R^2$ and $R^7$ may be combined together with the carbon atom to which they are attached to form the following group of formula (III) with $R^3$, the hydroxy group and $R^8$.

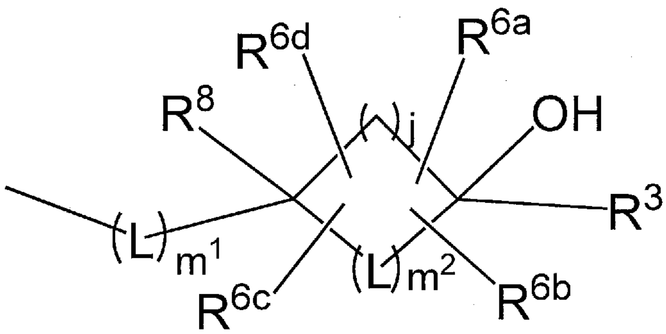
(III)

In the above formula (III),

preferably $m^1$ is 0,
preferably $m^2$ is 1 or 2,
preferably j is 1 or 2,
L is $CR^7R^8$,
preferably $R^8$ is hydrogen,
preferably $R^{6a}$, $R^{6b}$, $R^{6c}$, and $R^{6d}$ are independently hydrogen or halogen.

Preferably, m is 1 or 2,
more preferably, m is 1.

L is $CR^7R^8$ provided that when m is 2 or 3, each $CR^7R^8$ is independently the same or different.

Preferably, $R^4$ is hydrogen or $C_{1-4}$ alkyl which may be substituted with 1 - 5 the same or different halogen atoms; more preferably hydrogen or methyl.

Preferably, $R^7$ and $R^8$ include hydrogen or $C_{1-4}$ alkyl which may be independently substituted with 1 to 3 substituents selected independently from the group consisting of halogen, hydroxy group, $C_{1-4}$ alkoxy optionally-substituted with 1 to 3 substituents selected independently from Substituent-group A, $C_{3-7}$ cycloalkyl optionally-substituted with 1 to 3 substituents selected independently from Substituent-group B, $C_{3-7}$ cycloalkoxy optionally-substituted with 1 to 3 substituents selected independently from Substituent-group B, and 3- to 7-membered non-aromatic heterocyclyl optionally-substituted with 1 to 3 substituents selected independently from Substituent-group B; more preferably hydrogen.

Preferably, $R^7$ and $R^8$ include, for example, hydrogen, methyl, and ethyl; more preferably hydrogen.

Preferably, Substituent-group A includes fluorine, chlorine, hydroxy group, $C_{1-2}$ alkoxy, and $C_{5-6}$ cycloalkoxy; more preferably fluorine, hydroxy group, and $C_{1-2}$ alkoxy.

Preferably, Substituent-group B includes fluorine, chlorine, hydroxy group, $C_{1-2}$ alkyl, $C_{1-2}$ alkoxy, and $C_{5-6}$ cycloalkoxy; more preferably fluorine, hydroxy group, $C_{1-2}$ alkyl, and $C_{1-2}$ alkoxy.

One embodiment of the compound of formula (I) includes the following:

the compound or a pharmaceutically acceptable salt thereof wherein
$R^{1a}$, $R^{1b}$, $R^{1c}$, and $R^{1d}$ are independently hydrogen, halogen, $C_{1-4}$ alkyl, $C_{1-4}$ alkoxy (wherein the alkyl and the alkyl moiety in the alkoxy may be independently substituted with 1 to 3 substituents selected independently from the group consisting of halogen, hydroxy group, and $C_{1-4}$ alkoxy optionally-substituted with 1 to 3 substituents selected independently from Substituent-group A), $C_{3-7}$ cycloalkyl, $C_{3-7}$ cycloalkoxy (wherein the cycloalkyl and the cycloalkyl moiety in the cycloalkoxy may be independently substituted with 1 to 3 substituents selected independently from

the group consisting of halogen, hydroxy group, $C_{1-4}$ alkyl optionally-substituted with 1 to 3 substituents selected independently from Substituent-group A, and $C_{1-4}$ alkoxy optionally-substituted with 1 to 3 substituents selected independently from Substituent-group A), $C_{6-10}$ aryl, $C_{6-10}$ aryloxy, 5- to 12-membered heteroaryl, or 5- to 12-membered heteroaryloxy (wherein the aryl and the aryl moiety in the aryloxy, and the heteroaryl and the heteroaryl moiety in the heteroaryloxy may be independently substituted with 1 to 3 substituents selected independently from the group consisting of halogen, cyano, $C_{1-4}$ alkyl optionally-substituted with 1 to 3 substituents selected independently from Substituent-group A, and $C_{1-4}$ alkoxy optionally-substituted with 1 to 3 substituents selected independently from Substituent-group A),

$R^2$ and $R^3$ are independently hydrogen or $C_{1-6}$ alkyl which may be independently substituted with 1 to 5 substituents selected independently from the group consisting of halogen, hydroxy group, and $C_{1-4}$ alkoxy optionally-substituted with 1 to 3 substituents selected independently from Substituent-group A, or

$R^2$ and $R^3$ may be combined together with the carbon atom to which they are attached to form a ring, i.e., the following group of formula (IIa) with the hydroxy group

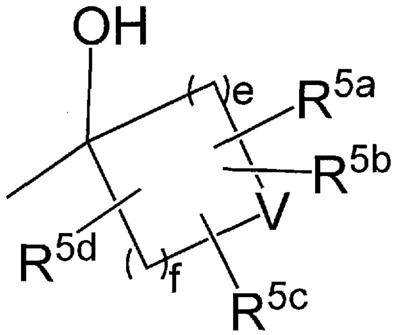

(IIa)

in formula (IIa),

e and f are independently 1 or 2,

V is single bond or oxygen atom,

$R^{5a}$, $R^{5b}$, $R^{5c}$, and $R^{5d}$ are independently hydrogen or halogen, or

in $R^2$, $R^3$, the hydroxy group bound to the carbon atom which is connected to $R^2$ and $R^3$, and $CR^7R^8$ in L,

$R^2$ and $R^7$ may be combined together with the carbon atom to which they are attached to form a ring, i.e., the following group of formula (IIIa) with $R^3$, the hydroxy group and $R^8$

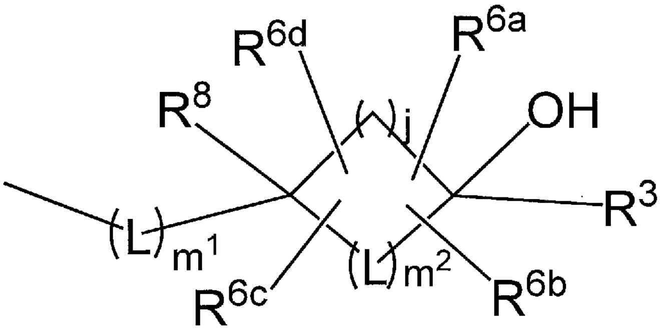

(IIIa)

in formula (IIIa),

$m^1$ is 0,

$m^2$ is 1 or 2,

j is 1 or 2,

$R^3$ is hydrogen or $C_{1-6}$ alkyl which may be independently substituted with 1 to 3 the same or different halogen atoms,

$R^8$ is hydrogen,

L is $CR^7R^8$, provided that when $m^2$ is 2, each $CR^7R^8$ are independently the same or different,

$R^{6a}$, $R^{6b}$, $R^{6c}$, and $R^{6d}$ are independently hydrogen or halogen,

$R^4$ is hydrogen or $C_{1-4}$ alkyl which may be substituted with 1 - 5 the same or different halogen atoms,

m is 1 or 2,

L is $CR^7R^8$, provided that when m is 2, each $CR^7R^8$ is independently the same or different,
$R^7$ and $R^8$ are independently hydrogen or $C_{1-4}$ alkyl which may be substituted with 1 - 3 the same or different halogen atoms.

Another embodiment of the compound of formula (I) includes the following:

the compound or a pharmaceutically acceptable salt thereof wherein
$R^{1a}$, $R^{1b}$, $R^{1c}$, and $R^{1d}$ are independently hydrogen, fluorine, chlorine, methyl, ethyl, isopropyl, isobutyl, cyclopropyl, cyclopentyl, cyclohexyl, methoxy, ethoxy, phenyl, 2-fluorophenyl, 3-fluorophenyl, 4-fluorophenyl, 4-(trifluoromethyl)phenyl, 5-(trifluoromethyl)pyridin-2-yl, 6-(trifluoromethyl)pyridin-3-yl, 3-chlorophenyl, 4-chlorophenyl, phenoxy, 3-fluorophenoxy, 4-fluorophenoxy, 3,4-difluorophenoxy, 3,5-difluorophenoxy, 4-chlorophenoxy, 4-methylphenoxy, 4-(trifluoromethyl)phenoxy, 4-methoxyphenoxy, 4-(trifluoromethoxy)phenoxy, 4-cyanophenoxy, (5-methylpyridin-2-yl)oxy, (5-(trifluoromethyl)pyridin-2-yl)oxy, (6-(trifluoromethyl)pyridin-3-yl)oxy, (5-fluoropyridin-2-yl)oxy, 2-methoxy-4-(trifluoromethyl)phenyl, 2-fluoro-4-(trifluoromethyl)phenyl, 2-chloro-4-(trifluoromethyl)phenyl, 4-(trifluoromethoxy)phenyl, (5-chloropyridin-2-yl)oxy, 2,4-dichlorophenyl, 2-chloro-4-fluorophenoxy, 4-chloro-2-fluorophenoxy, or 2,4-dichlorophenoxy,
$R^2$ and $R^3$ are independently hydrogen or $C_{1-6}$ alkyl which may be independently substituted with 1 to 5 the same or different halogen atoms, or
$R^2$ and $R^3$ may be combined together with the carbon atom to which they are attached to form a ring, i.e., the following group of formula (IIa) with the hydroxy group

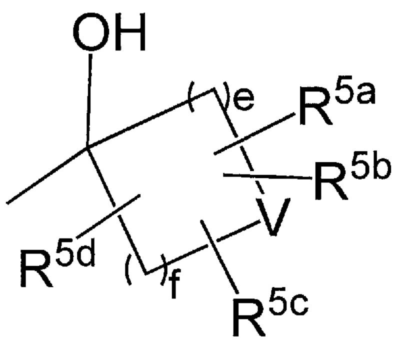
(IIa)

in formula (IIa),

e and f are independently 1 or 2,
V is oxygen atom,
$R^{5a}$, $R^{5b}$, $R^{5c}$, and $R^{5d}$ are hydrogen, or
in $R^2$, $R^3$, the hydroxy group bound to the carbon atom which is connected to $R^2$ and $R^3$, and $CR^7R^8$ in L,
$R^2$ and $R^7$ may be combined together with the carbon atom to which they are attached to form a ring, i.e., the following group of formula (IIIa) with $R^3$, the hydroxy group and $R^8$

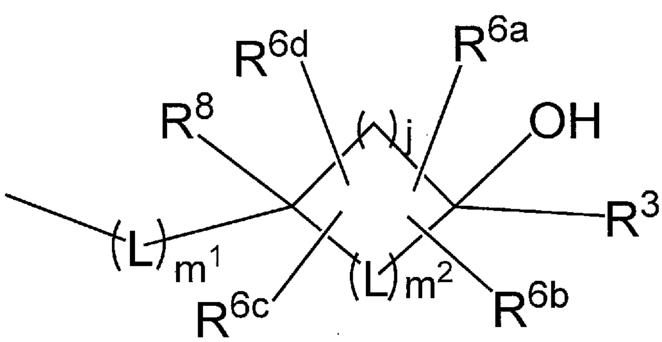
(IIIa)

in formula (IIIa),

$m^1$ is 0,
$m^2$ is 1 or 2,

j is 1 or 2,
$R^3$ is hydrogen or $C_{1-6}$ alkyl,
$R^8$ is hydrogen,
L is $CR^7R^8$, provided that when $m^2$ is 2, each $CR^7R^8$ are independently the same or different,
$R^{6a}$, $R^{6b}$, $R^{6c}$, and $R^{6d}$ are hydrogen,
$R^4$ is hydrogen or $C_{1-4}$ alkyl,
m is 1 or 2,
L is $CR^7R^8$, provided that when m is 2, each $CR^7R^8$ is independently the same or different,
$R^7$ and $R^8$ are independently hydrogen or $C_{1-4}$ alkyl.

Another embodiment of the compound of formula (I) includes the following:

the compound or a pharmaceutically acceptable salt thereof wherein
$R^{1a}$, $R^{1b}$, $R^{1c}$, and $R^{1d}$ are independently hydrogen, fluorine, 4-(trifluoromethyl)phenyl, 5-(trifluoromethyl)pyridin-2-yl, 6-(trifluoromethyl)pyridin-3-yl, 3-fluorophenyl, 4-fluorophenyl, 4-chlorophenyl, 3-fluorophenoxy, 4-fluorophenoxy, 4-chlorophenoxy, 4-methylphenoxy, 4-(trifluoromethyl)phenoxy, 4-(trifluoromethoxy)phenoxy, (5-methylpyridin-2-yl)oxy, (5-(trifluoromethyl)pyridin-2-yl)oxy, (6-(trifluoromethyl)pyridin-3-yl)oxy, 2-methoxy-4-(trifluoromethyl)phenyl, 4-(trifluoromethoxy)phenyl, or (5-chloropyridin-2-yl)oxy,
$R^2$ and $R^3$ are independently hydrogen or methyl, or
$R^2$ and $R^3$ may be combined together with the carbon atom to which they are attached to form a ring, i.e., the following group of formula (IIa) with the hydroxy group

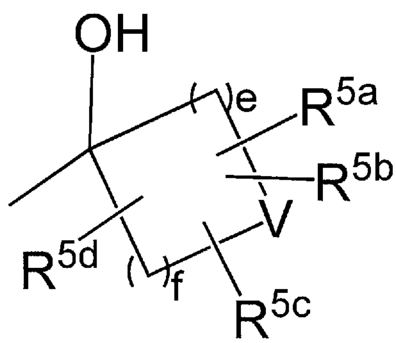
(IIa)

in formula (IIa),

e and f are 1,
V is oxygen atom,
$R^{5a}$, $R^{5b}$, $R^{5c}$, and $R^{5d}$ are hydrogen, or
in $R^2$, $R^3$, the hydroxy group bound to the carbon atom which is connected to $R^2$ and $R^3$, and $CR^7R^8$ in L,
$R^2$ and $R^7$ may be combined together with the carbon atom to which they are attached to form a ring, i.e., the following group of formula (IIIa) with $R^3$, the hydroxy group and $R^8$

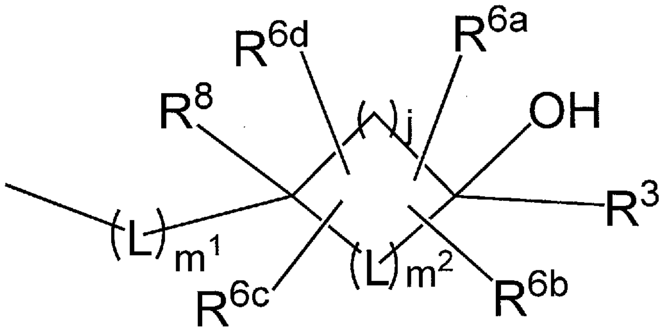
(IIIa)

in formula (IIIa),

$m^1$ is 0,

$m^2$ is 2,
j is 2,
$R^3$ is hydrogen,
$R^8$ is hydrogen,
L is $CH_2$, and
$R^{6a}$, $R^{6b}$, $R^{6c}$, and $R^{6d}$ are hydrogen,
$R^4$ is hydrogen or $C_{1-4}$ alkyl,
m is 1,
L is $CR^7R^8$,
$R^7$ and $R^8$ are independently hydrogen or methyl.

Processes to prepare the compounds of the present invention are mentioned below. The compound (I) of the present invention can be prepared, for example, according to Processes 1 to 5 shown below.

Process 1:

In the compounds of formula (I), a compound of formula (7) or (9) wherein $R^{1b}$ is $OR^a$ or a pharmaceutically acceptable salt thereof can be prepared, for example, according to the following process.

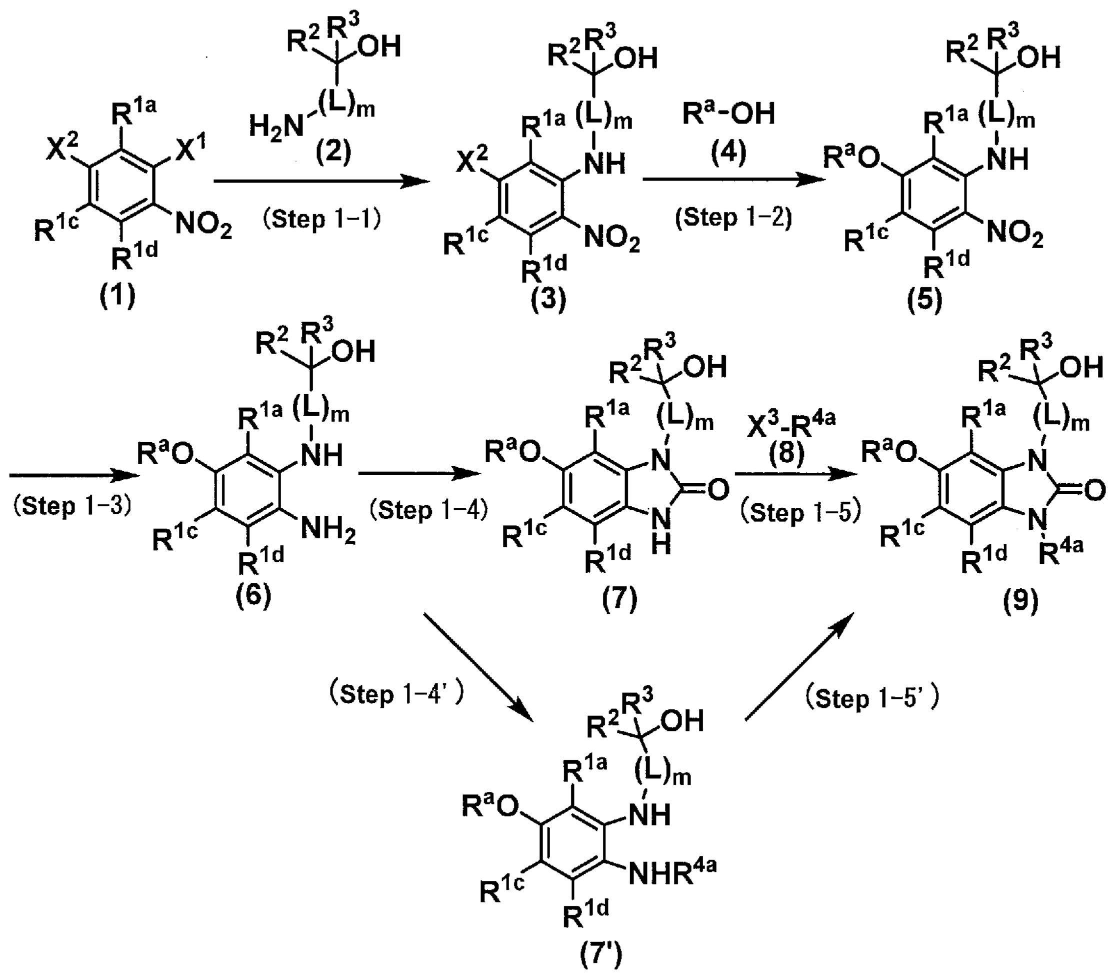


In the above scheme, $R^{1a}$, $R^{1c}$, $R^{1d}$, $R^2$, $R^3$, L, and m are as defined in Item 1; $R^a$ means $R^{1b}$ which is selected from $C_{1-4}$ alkyl optionally-substituted with 1 to 3 substituents selected independently from Substituent-group A, $C_{3-7}$ cycloalkyl optionally-substituted with 1 to 3 substituents selected independently from Substituent-group B, $C_{6-10}$ aryl optionally-substituted with 1 to 3 substituents selected independently from Substituent-group B, or 5- to 12-membered heteroaryl optionally-substituted with 1 to 3 substituents selected independently from Substituent-group B; $X^1$, $X^2$, and $X^3$ are independently a leaving group such as halogen, trifluoromethanesulfonyloxy, and methanesulfonyloxy; and $R^{4a}$ means $R^4$ which is selected from $C_{1-4}$ alkyl or $C_{3-4}$ cycloalkyl (where in the $C_{1-4}$ alkyl and the $C_{3-4}$ cycloalkyl may be

substituted with 1 - 5 the same or different halogen atoms).

Step (1-1):

Nitroaniline compound (3) can be prepared by reacting nitrobenzene compound (1) and amine compound (2) in a suitable inert solvent in the presence of a base. The base used herein includes inorganic bases such as sodium hydroxide, potassium hydroxide, potassium carbonate, and cesium carbonate; and organic bases such as triethylamine, diisopropylethylamine, and DABCO. When the amine compound is used in large excess, it is not necessary to use such base. The solvent used herein includes ethers such as THF, 1,2-dimethoxyethane, and 1,4-dioxane, DMF, NMP, and acetonitrile. The reaction time is about 10 minutes to about 10 hours, and the reaction temperature is 0°C to boiling point of a solvent used herein.

Step 1-2:

Compound (5) can be prepared by reacting Compound (3) and Compound (4) in a suitable inert solvent in the presence of a base. The base used herein includes sodium hydroxide, potassium hydroxide, potassium carbonate, cesium carbonate, and sodium hydride. The solvent used herein includes ethers such as THF, 1,2-dimethoxyethane, and 1,4-dioxane, DMF, NMP, and acetonitrile. The reaction time is about 10 minutes to about 10 hours, and the reaction temperature is 0°C to boiling point of a solvent used herein.

Step 1-3:

Compound (6) can be prepared by reducing Compound (5) in a suitable inert solvent under a generally-used condition for reducing a nitro group. The reaction condition in this step includes catalytic reduction with palladium-carbon, etc. under hydrogenation condition; metal reduction with zinc, iron, etc.; hydride reduction with lithium aluminum hydride, etc. The solvent used in this reduction includes various solvents generally-used in each reduction condition. In case of catalytic reduction, it includes methanol, ethanol, THF, and ethyl acetate; in case of metal reduction, it includes THF, acetic acid, methanol, and ethanol; and in case of hydride reduction, it includes diethyl ether, and THF. The reaction time is about 10 minutes to about 24 hours, and the reaction temperature is 0°C to boiling point of a solvent used herein.

Step 1-4:

Compound (7) can be prepared by reacting Compound (6) in a suitable inert solvent under a generally-used condition for forming an imidazolone-ring. The condition of the reaction includes, for example, a cyclization condition using 1,1'-carbonyldiimidazole, triphosgene, or urea. As appropriate, a base such as triethylamine and diisopropylethylamine may be added to the reaction. The solvent used herein includes ethers such as THF, 1,2-dimethoxyethane, and 1,4-dioxane; aprotic polar solvents such as DMF, NMP, and acetonitrile; and aromatic hydrocarbons such as benzene and toluene. The reaction time is about 10 minutes to about 10 hours, and the reaction temperature is 0°C to boiling point of a solvent used herein.

Step 1-5:

Compound (9) can be prepared by reacting Compound (7) and Compound (8) in a suitable inert solvent in the presence of a base. The base used herein includes sodium hydride, potassium carbonate, cesium carbonate, and calcium carbonate. The solvent used herein includes ethers such as THF, 1,2-dimethoxyethane, and 1,4-dioxane, DMF, NMP, and acetonitrile. The reaction time is about 10 minutes to about 10 hours, and the reaction temperature is 0°C to boiling point of a solvent used herein.

Step 1-4':

Compound (7') can be prepared by reacting Compound (6) and the corresponding aldehyde or ketone compound in a suitable inert solvent in the presence of a reducing agent. The reducing agent used herein includes sodium borohydride and sodium cyanotrihydroborate. The solvent used herein includes halogenated carbons such as chloroform and dichloromethane; ethers such as diethyl ether, THF, and 1,4-dioxane; acetonitrile; and alcohols such as methanol and ethanol. The reaction time is generally about 10 minutes to about 10 hours, and the reaction temperature is 0°C to boiling point of a solvent used herein. Compound (7') can be also prepared from Compound (6) in the same manner as Step (1-5).

Step 1-5':

Compound (9) can be also prepared from Compound (7') in the same manner as Step (1-4).

The above-mentioned Step (1-1) and subsequent Step (1-2) may be sequentially performed, *i.e.,* Compound (5) can be prepared in one step from Compound (1). The reaction time of the sequential reactions is 10 minutes to 12 hours, and the reaction temperature is room temperature to boiling point of a solvent used herein.

Process 2:

In the compounds of formula (I), a compound of formula (7) wherein $R^{1b}$ is $OR^a$ or a pharmaceutically acceptable salt thereof can be also prepared, for example, according to the following process.

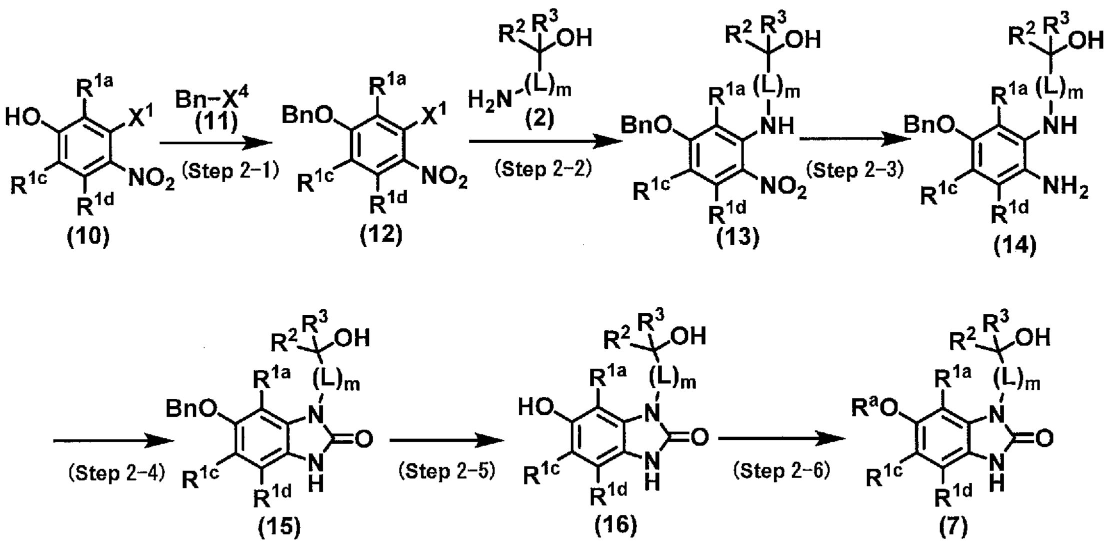

(10) (12) (13) (14) (15) (16) (7)

In the above scheme, $R^{1a}$, $R1^c$, $R^{1d}$, $R^2$, $R^3$, L, and m are as defined in Item 1; $R^a$ and $X^1$ are as defined above; $X^4$ is a leaving group such as halogen, trifluoromethanesulfonyloxy, and methanesulfonyloxy. Bn means benzyl group, which may encompass a protecting group that can be deprotected like benzyl group, for example, substituted benzyl group disclosed in Protective Groups in Organic Synthesis.

Step 2-1:

Compound (12) can be prepared, for example, by reacting Compound (10) and Compound (11) in a suitable inert solvent in the presence of a base. The base used herein includes sodium carbonate, potassium carbonate, cesium carbonate, and sodium hydride. Compound (11) includes benzyl chloride and benzyl bromide. As appropriate, sodium iodide, potassium iodide, tetrabutylammonium iodide, tetrabutylammonium hydrogen sulfate, etc. may be added to the reaction. The solvent used herein includes acetone, acetonitrile, THF, diethyl ether, 1,4-dioxane, 1,2-dimethoxyethane, DMF, and NMP. The reaction time is generally 30 minutes to 24 hours, and the reaction temperature is 0°C to boiling point of a solvent used herein. In addition, compound (12) can be prepared from compound (10) according to the method (condition) described in Protective Groups in Organic Synthesis or the like.

Step 2-2:

Compound (13) can be prepared from Compound (12) in the same manner as Step (1-1).

Step 2-3:

Compound (14) can be prepared by selectively reducing the nitro group in Compound (13). The reaction condition in this step includes catalytic reduction with sulfur-poisoning platinum-carbon, etc. under hydrogenation condition; metal reduction with zinc, iron, tin, etc.; hydride reduction with lithium aluminum hydride, etc. The solvent used in this reduction includes various solvents generally-used in each reduction condition. In case of catalytic reduction, it includes methanol, ethanol, THF, and ethyl acetate; in case of metal reduction, it includes THF, acetic acid, methanol,

and ethanol; and in case of hydride reduction, it includes diethyl ether, and THF. The reaction time is generally about 10 minutes to about 24 hours, and the reaction temperature is 0°C to boiling point of a solvent used herein.

Step 2-4:

Compound (15) can be prepared from Compound (14) in the same manner as Step (1-4).

Step 2-5:

Compound (16) can be prepared, for example, by catalytic reduction of Compound (15) under hydrogenation condition. The catalyst used herein includes heterogenous catalysts such as palladium-carbon. The hydrogenation condition means "under hydrogen atmosphere", or "in the presence of formic acid, ammonium formate, etc." The solvent used herein includes methanol, ethanol, THF, and ethyl acetate. The reaction time is 30 minutes to 24 hours, and the reaction temperature is 0°C to boiling point of a solvent used herein. In addition, compound (16) can be prepared from compound (15) according to the method (condition) described in Protective Groups in Organic Synthesis or the like.

Step 2-6:

This step is a process to prepare Compound (7) from Compound (16), which includes two reaction conditions, but should not be limited thereto.

1) A reaction condition herein using a base includes the following step: Compound (7) is prepared by reacting Compound (16) and $R^a$-$X^5$ (wherein $R^a$ is as defined above, $X^5$ is a leaving group such as halogen, trifluoromethanesulfonyloxy, and methanesulfonyloxy) in the same manner as Step (2-1).
2) A reaction condition herein using a catalyst and a base includes a reaction with a boronate compound or a halogen compound which has $R^a$ group. The catalyst used herein includes copper(II) acetate, copper(I) iodide, and copper(II) oxide. The base used herein includes potassium carbonate, cesium carbonate, potassium hydroxide, and triethylamine. The solvent used herein includes chloroform, 1,4-dioxane, DMF, dimethylsulfoxide, and NMP. The reaction time is generally 30 minutes to 24 hours, and the reaction temperature is room temperature to boiling point of a solvent used herein.

A compound of formula (I) wherein any one or more of $R^{1a}$, $R^{1b}$, $R^{1c}$, and $R^{1d}$ are $OR^a$ or a pharmaceutically acceptable salt thereof can be also prepared in the same manner as above.

Process 3:

In the compounds of formual (I), a compound of formula (7) wherein $R^{1b}$ is $OR^a$ or a pharmaceutically acceptable salt thereof can be also prepared, for example, according to the following process.

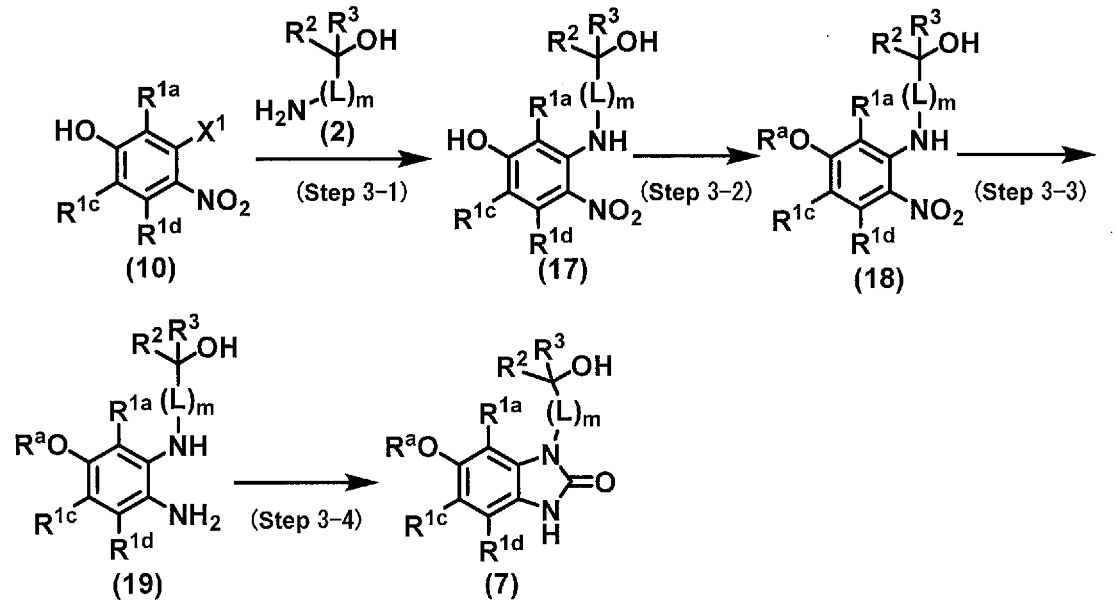


(10) (2) (17) (18) (19) (7)

In the above scheme, $R^{1a}$, $R^{1c}$, $R^{1d}$, $R^2$, $R^3$, L, and m are as defined in Item 1, and $R^a$ and $X^1$ are as defined above.

<u>Step 3-1:</u>

Compound (17) can be prepared from Compound (10) in the same manner as Step (1-1).

<u>Step 3-2:</u>

Compound (18) can be prepared from Compound (17) in the same manner as Step (2-6).

<u>Step 3-3:</u>

Compound (19) can be prepared from Compound (18) in the same manner as Step (1-3).

<u>Step 3-4:</u>

Compound (7) can be prepared from Compound (19) in the same manner as Step (1-4).

The above-mentioned Step (3-1) and subsequent Step (3-2) may be sequentially performed, *i.e.,* Compound (18) can be prepared in one step from Compound (10). The reaction time of the sequential reactions is 10 minutes to 12 hours, and the reaction temperature is room temperature to boiling point of a solvent used herein.

<u>Process 4:</u>

In the compounds of formula (I), compound of formula (22) or (23) wherein $R^{1b}$ is $R^b$ or a pharmaceutically acceptable salt thereof can be prepared, for example, according to the following process.

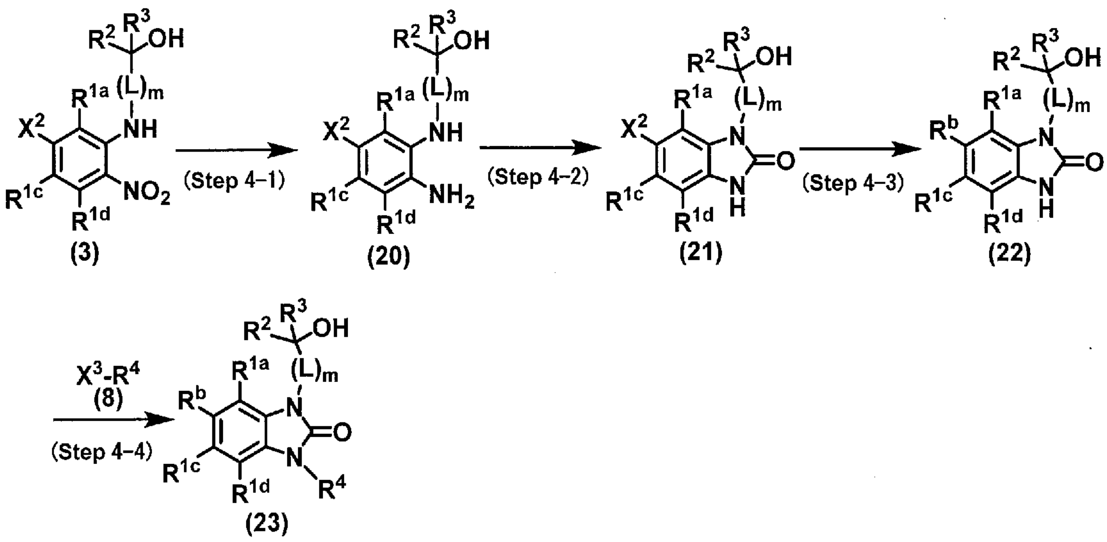


In the above scheme, $R^{1a}$, R1$^c$, $R^{1d}$, $R^2$, $R^3$, $R^4$, L, and m are as defined in Item 1; $X^2$ and $X^3$ are as defined above; and $R^b$ is $C_{6-10}$ aryl or 5- to 12-membered heteroaryl (wherein the aryl and the heteroaryl may be independently substituted with 1 to 5 substituents selected independently from the group consisting of halogen, cyano, $C_{1-4}$ alkyl optionally-substituted with 1 to 3 substituents selected independently from Substituent-group A, $C_{1-4}$ alkoxy optionally-substituted with 1 to 3 substituents selected independently from Substituent-group A, $C_{3-7}$ cycloalkyl optionally-substituted with 1 to 3 substituents selected independently from Substituent-group B, $C_{3-7}$ cycloalkoxy optionally-substituted with 1 to 3 substituents selected independently from Substituent-group B, and 3- to 7-membered non-aromatic heterocyclyl optionally-substituted with 1 to 3 substituents selected independently from Substituent-group B).

Step 4-1:

Compound (20) can be prepared from Compound (3) in the same manner as Step (2-3) (selective reduction of nitro group).

Step 4-2:

Compound (21) can be prepared from Compound (20) in the same manner as Step (1-4).

Step 4-3:

Compound (22) can be prepared by reacting Compound (21) and boronic acid or boronate compound which has $R^b$ group in the presence of a base and a catalyst. For example, this step is Suzuki coupling reaction. The base used herein includes sodium carbonate, potassium carbonate, cesium carbonate, and tripotassium phosphate. The catalyst used herein includes palladium acetate, tetrakis(triphenylphosphine)palladium, and tris(dibenzylideneacetone)dipalladium. The solvent used herein includes 1,4-dioxane, toluene, and 1,2-dimethoxyethane. The reaction time is about 30 minutes to about 24 hours, and the reaction temperature is room temperature to boiling point of a solvent used herein.

Step 4-4:

Compound (23) can be prepared from Compound (22) and Compound (8) in the same manner as Step (1-5).

A compound of formula (I) wherein any one or more of $R^{1a}$, $R^{1b}$, $R^{1c}$, and $R^{1d}$ are $R^b$ or a pharmaceutically acceptable salt thereof can be also prepared in the same manner as above.

Process 5:

In the compounds of formual (I), a compound of formula (22) wherein $R^{1b}$ is $R^b$ or a pharmaceutically acceptable salt thereof can be also prepared, for example, according to the following process.

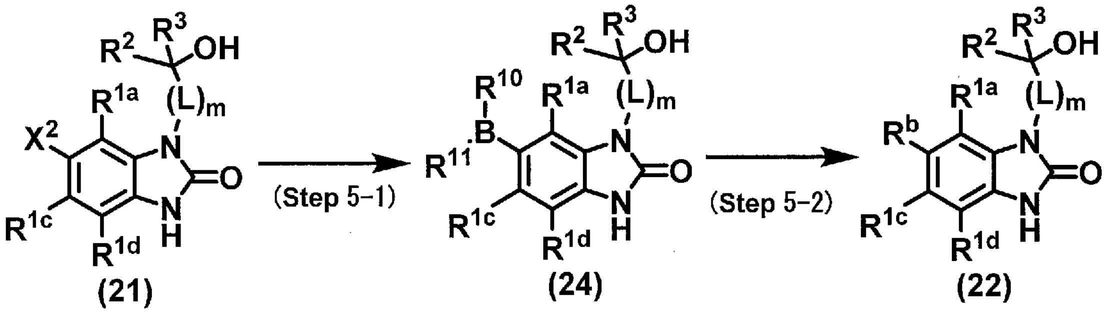


(21) (24) (22)

In the above scheme, $R^{1a}$, $R^{1c}$, $R^{1d}$, $R^2$, $R^3$, L, and m are as defined in Item 1; $R^b$ and $X^2$ are as defined above; $R^{10}$ and $R^{11}$ are independently optionally-substituted $C_{1-4}$ alkyl, optionally-substituted $C_{1-4}$ alkoxy, optionally-substituted $C_{1-4}$ dialkylamino, optionally-substituted $C_{6-10}$ aryl, optionally-substituted $C_{6-10}$ aryloxy, optionally-substituted 5- to 12-membered heteroaryl, optionally-substituted 5- to 12-membered heteroaryloxy, or hydroxy group. Preferably, $R^{10}R^{11}B$- includes the following structures, but not limited thereto.

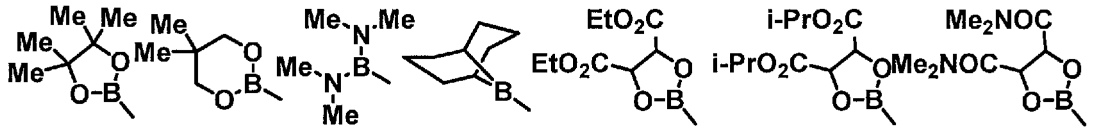

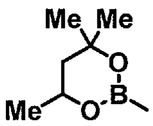 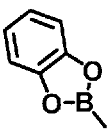 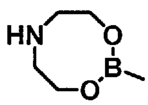 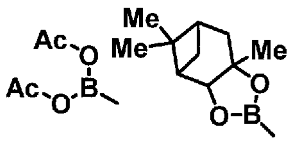 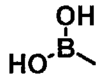 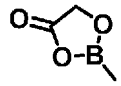 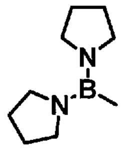

Step 5-1:

Compound (24) can be prepared by reacting Compound (21) and diborons such as bis(pinacolato)diboron in the presence of a catalyst and a base. The catalyst used herein includes [1,1'-bis(diphenylphosphino)ferrocene]dichloropalladium, tetrakis(triphenylphosphine)palladium, tris(dibenzylideneacetone)dipalladium, and dichlorobis(triphenylphosphine)palladium. The base used herein includes potassium acetate, tripotassium phosphate, and potassium carbonate. The solvent used herein includes 1,4-dioxane, toluene, and 1,2-dimethoxyethane. The reaction time is about 1 hour to about 48 hours, and the reaction temperature is room temperature to boiling point of a solvent used herein.

Step 5-2:

Compound (22) can be prepared by reacting Compound (24) and a halide or triflate compound having $R^b$ group ($R^b$-X (X: halogen atom) or $CF_3SO_2O$-$R^b$, etc.) in the presence of a catalyst and a base. For example, this step is Suzuki coupling reaction. The base used herein includes sodium carbonate, potassium carbonate, cesium carbonate, and tripotassium phosphate. The catalyst used herein includes palladium acetate, tetrakis(triphenylphosphine)palladium, and tris(dibenzylideneacetone)dipalladium. The solvent used herein includes 1,4-dioxane, toluene, and 1,2-dimethoxyethane. The reaction time is about 30 minutes to 48 hours, and the reaction temperature is room temperature to boiling point of a solvent used herein.

A compound of formula (I) wherein any one or more of $R^{1a}$, $R^{1b}$, $R^{1c}$, and $R^{1d}$ are $R^b$ or a pharmaceutically acceptable salt thereof can be also prepared in the same manner as above.

The room temperature in the above processes means specifically 10°C to 30°C.

The starting materials and intermediates in the above processes are known compounds or can be prepared from known compounds according to a known method. In case that any functional group other than a target reaction site can be reacted or can be unsuitable in the above processes, the functional group other than the target reaction site can be protected for the reaction, and the protective group can be cleaved to give a desired compound after the reaction is completed. The protective group used herein includes, for example, a conventional protective group disclosed in the aforementioned Protective Groups in Organic Synthesis and such. Specifically, the protective group for amino group includes, for example, ethoxycarbonyl, tert-butoxycarbonyl, acetyl, benzyl, and the like; and the protective group for hydroxy includes, for example, tri-lower alkylsilyl, acetyl, benzyl, and the like.

The introduction and cleavage of protective groups can be done by a conventional method in organic chemistry (for example, *see,* the aforementioned Protective Groups in Organic Synthesis), or a similar method.

By appropriately changing functional group(s) in an intermediate or final product in the above processes, it is also possible to prepare a different compound defined in the present invention. The conversion of functional group(s) can be done according to a conventional method (e.g. Comprehensive Organic Transformations, R. C. Larock (1989)).

The intermediates and desired compounds in the above processes can be isolated/purified by a purification generally-used in synthetic organic chemistry, for example, neutralization, filtration, extraction, washing, drying, concentration, recrystallization, various chromatography, etc. Some intermediates can be used in next step without any purification.

The optical isomers of the present invention can be isolated by using a known division method at an appropriate step, for example, separation with an optically-active column, and fractionated crystallization. And, it is workable to use an optically-active starting material.

The compounds of the present invention may be sometimes an optical isomer, a stereoisomer, a tautomer such as a keto-enol compound, and/or a geometric isomer, hence which include all possible isomers including the above isomers, and a mixture thereof.

The compounds of the present invention may also include the compound of formula (I), a prodrug thereof, and a pharmaceutically acceptable salt thereof, besides the above isomers. And, the compounds of the present invention or a pharmaceutically acceptable salt thereof may be in a form of an adduct with water or each solvent, hence which also include such adducts. In addition, the compounds of the present invention may also include various embodiments of the crystals and the compounds in which a part or all of atoms composing the compounds are replaced with another isotope (for example, replacing hydrogen with deuterium, and replacing $^{12}C$ with $^{14}C$).

The term "prodrug of the compound of formula (I)" used herein means a compound which can be converted to the compound of formula (I) by reacting with an enzyme, gastric acid, etc. under intravitally physiological condition, i.e., a compound which can be enzymatically oxidized, reduced, hydrolyzed, or taken somehow to be converted to the compound of formula (I), and a compound which can be hydrolyzed with gastric acid or the like to be converted to the compound of formula (I).

The "pharmaceutically acceptable salt" used herein includes, for example, a base addition salt or an acid addition salt. The base addition salt includes, for example, an alkali metal salt such as potassium salt and sodium salt; an alkaline earth metal salt such as calcium salt and magnesium salt; a water-soluble amine addition salt such as ammonium salt and N-methylglucamine (meglumine); and a lower alkanol ammonium salt of an organic amine. The acid addition salt includes, for example, hydrochloride, hydrobromide, hydroiodide, nitrate, sulfate, bisulfate, phosphate, acetate, lactate, citrate, tartrate, bitartrate, succinate, maleate, fumarate, gluconate, saccharate, benzoate, methanesulfonate, ethanesulfonate, benzene sulfonate, p-toluenesulfonate, and pamoate[1,1'-methylene-bis-(2-hydroxy-3-naphthoate)].

Salts of the present compound can be prepared, for example, in the following manners. For example, when the present compound is obtained in a salt form, the salt thereof can be prepared by directly purifying it. When the present compound is obtained in a free form, the salt thereof can be prepared by dissolving or suspending it in an appropriate organic solvent, adding a possible acid or base thereto, and then treating the obtained mixture in a general manner.

The compound of formula (I) prepared by the above processes may be isolated/purified in a conventional manner such as extraction, column chromatography, recrystallization, and reprecipitation. The extraction solvent used herein includes, for example, diethyl ether, ethyl acetate, chloroform, dichloromethane, toluene, and the like. The purification by column chromatography can be done with an acid-, basic-, or variously-chemical-treating silica gel, alumina, or the like. The elute solvent used herein includes, for example, hexane/ethyl acetate, hexane/chloroform, ethyl acetate/methanol, chloroform/methanol, acetonitrile/water, methanol/water, and the like.

The novel compounds of the present invention or a pharmaceutically acceptable salt thereof having a benzimidazolone ring have a property inhibiting Nav 1.7 and thereby can be used as a medicament for treating or preventing a pain involving peripheral nerve such as C-fibres and Aδ-fibres, spontaneous pain such as numbness, burning pain, dull pain, pricking pain and shooting pain, neuropathic pain accompanied by hyperalgesia such as mechanical stimulation and cold stimulation or allodynia, nociceptive pain, inflammatory pain, small-fiber neuropathy, erythromelalgia, paroxysmal extreme pain disorder, etc. The neuropathic pain includes, for example, diabetic neuropathy, postherpetic neuralgia, chemotherapy-induced neuropathy, cancer pain, sensory nerve damage caused by viral infection in human immune deficiency syndrome, trigeminal neuralgia, complex regional pain syndrome, reflex sympathetic dystrophy, neuralgia after low back surgery, phantom limb pain, pain after spinal cord injury, persistent postoperative pain, inflammatory demyelinating polyradiculopathy, alcoholic neuropathy, entrapment peripheral neuropathy, iatrogenic neuropathy, sudden sensorineural disorder, malnutrition-induced neuropathy, radiation-induced neuropathy, radiculopathy, toxic peripheral neuropathy, traumatic peripheral neuropathy, brachial plexus avulsion injury, glossopharyngeal neuralgia, autoimmune neuropathy, and chronic cauda equina syndrome. The nociceptive pain or inflammatory pain includes low

back pain, abdominal pain, chronic rheumatoid arthritis, a pain caused by osteoarthritis, myalgia, acute postoperative pain, fracture pain, pain after burn injury, and the like. In addition, the present compounds or a pharmaceutically acceptable salt thereof can be also used as a medicament for treating or preventing dysuria. The dysuria includes frequent urination, bladder pain caused by prostatic hyperplasia, and the like. Furthermore, the present compounds or a pharmaceutically acceptable salt thereof can be also used as a medicament for treating or preventing ataxia developed by suppressing abnormal nervous firing in the cerebellum in multiple sclerosis. In addition, the present compounds or a pharmaceutically acceptable salt thereof can be a drug having no side effect in heart or central nerve which is a problem in existing medication, since they have a selective inhibitory activity to Nav 1.7.

The present compounds may be administered orally, parenterally or rectally, and the daily dose can vary depending on the compound, the mode of administration, patient's condition/age, etc. For oral administration, for example, the present compounds may be administered generally in a dosage of about 0.01 to 1000 mg, preferably about 0.1 to 500 mg a day per kilogram of body weight of human or mammal and once to several times. For parenteral administration such as intravenous injection, for example, the present compounds may be administered generally in a dosage of about 0.01 to 300 mg, preferably about 1 to 100 mg per kilogram of body weight of human or mammal.

The present compounds can be orally or parenterally administered directly or as a suitable formulation comprising it. The formulation thereof may be, for example, tablet, capsule, powder, granule, liquid, suspension, injection, patch, gel patch, and the like, but not limited thereto. The formulation can be prepared with pharmaceutically acceptable additive agents in known means. The additive agents can be chosen for any purpose, including an excipient, a disintegrant, a binder, a fluidizer, a lubricant, a coating agent, a solubilizer, a solubilizing agent, a thickener, dispersant, a stabilizing agent, a sweetening agent, a flavor, and the like. Specifically, they include, for example, lactose, mannitol, microcrystalline cellulose, low-substituted hydroxypropylcellulose, cornstarch, partially-pregelatinized starch, carmellose calcium, croscarmellose sodium, hydroxypropylcellulose, hydroxypropyl methylcellulose, polyvinyl alcohol, magnesium stearate, sodium stearyl fumarate, polyethylene glycol, propylene glycol, titanium oxide, talc, and the like.

The present compounds and a pharmaceutically acceptable salt thereof may be used in combination with, for example, a non-steroidal anti-inflammatory agent such as celecoxib, Voltaren, ibuprofen, loxoprofen, acetaminophen, diclofenac and dexamethasone, and an opioid analgesic such as tramadol, morphine and oxycodone, in order to strengthen the action thereof. In addition, the present compounds and a pharmaceutically acceptable salt thereof may be also used in combination with an antiepileptic agent (such as pregabalin and carbamazepine), an aldose reductase inhibitor (such as epalrestat), a prostaglandin derivative drug (such as limaprost alfadex), an antidepressive agent (such as amitriptyline and duloxetine), an anticonvulsant agent, an anxiolytic agent, a dopamine receptor agonist, an antiparkinsonian agent, a hormone preparation, a migraine medication, an adrenergic $\beta$ receptor antagonist, a drug for treating dementia, a drug for treating mood disorder, or the like. Preferred drugs used in combination with the present compound and a pharmaceutically acceptable salt thereof include an antiepileptic agent such as pregabalin and carbamazepine, an antidepressive agent such as amitriptyline and duloxetine, a narcotic analgesic such as morphine, oxycodone and tramadol, an anti-inflammatory agent such as acetaminophen, diclofenac and dexamethasone, an aldose reductase inhibitor such as epalrestat, and a prostaglandin derivative such as limaprost alfadex. In order to reduce the side effects thereof, the present compounds and a pharmaceutically acceptable salt thereof may be used in combination with an antiemetic drug and a sleep-inducing drug. The administration interval of the present compound and its concomitant drug is not limited, i.e., the concomitant drug may be administered at the same time as the present compound or at a suitable interval. Or, the present compound and its concomitant drug can be formulated into a combination drug. The dose of the combination drug can be suitably determined based on the standard of the clinically-used dose thereof. The combination ratio of the present compound and its concomitant drug can be suitably determined based on its subject patient, administration route, disease, pathology, concomitant drug, etc. For example, when the subject patient is a human being, the concomitant drug may be used in 0.01 to 1000 part by weight per part of the present compound.

## EXAMPLES

The present invention is explained in more detail in the following by referring to Reference examples, Examples, and Pharmacological tests; however, the technical scope of the present invention is not limited to such Examples and the like. The silica gel chromatography used in Examples was silica gel column chromatography or amino silica gel column chromatography made by YAMAZEN CORPORATION. Each compound was identified with a proton nuclear magnetic resonance spectrum ($^1$H-NMR), high-performance liquid chromatograph-mass spectrometer; LCMS, etc. $^1$H-NMR was measured with JNM-ECS400 (JEOL).

The measuring condition of high-performance liquid chromatography-mass spectrometer; LCMS is shown below, and the detected value of mass spectrography [MS (m/z)] is shown as M+H.

MS detector: ACQITY SQD
HPLC: ACQITY UPLC
Column: ACQITY BEH C18 1.7 $\mu$m, 2.1 x 50 mm

Flow rate: 0.75 mL/min
Wave length: 254 nm
Mobile phase: A: 0.05 % aqueous formic acid
B: acetonitrile

Time program:
Step time (min)

| | | |
|---|---|---|
| 1 | 0.0-1.3 | A : B = 90 : 10 => 1 : 99 |
| 2 | 1.3-1.5 | A : B = 1 : 99 |
| 3 | 1.5-2.0 | A : B = 90 : 10 |

Unless otherwise specified, the starting material compounds, reaction reagents and solvents used herein were commercially available products or were prepared according to known methods.

In Reference examples, Examples, and Pharmacological examples, abbreviations shown below may be sometimes used to simplify the description of the present specification. Me: methyl, Et: ethyl, THF: tetrahydrofuran, DMF: N,N-dimethylformamide, NMP: N-methyl-2-pyrrolidinone, DMSO: dimethylsulfoxide, HEPES: 2-[4-(2-hydroxyethyl)-1-piperazinyl]ethanesulfonic acid, EGTA: O,O'-bis(2-aminoethyl)ethylene glycol-N,N,N',N'-tetraacetate, J: coupling constant, s: singlet, d: doublet, t: triplet, q: quartet, dd: double doublet, m: multiplet, br: broad.

Reference example 1: 2-methyl-1-[(2-nitro-5-{[5-(trifluoromethyl)pyridin-2-yl]oxy}phenyl)amino]propan-2-ol

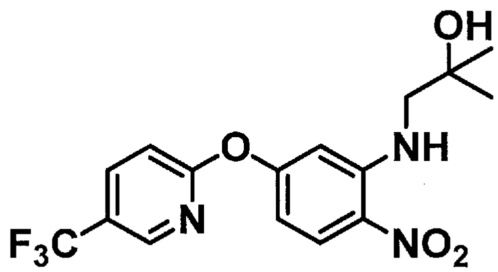

To a solution of 3-fluoro-4-nitrophenol (1.0 g) in NMP (15.9 mL) were added 1-amino-2-methylpropan-2-ol (0.74 g) and diisopropylethylamine (2.78 mL), and the mixture was stirred at 110°C for 3 hours. The reaction solution was cooled to room temperature, and 2-fluoro-5-(trifluoromethyl)pyridine (1.37 g) and cesium carbonate (3.11 g) were added to the reaction solution. The mixture was stirred at 110°C for 2 hours. The reaction solution was cooled to room temperature, and water and ethyl acetate/hexane (3/1) were added to the reaction solution. The desired product was extracted in the organic layer. The organic layer was washed with brine, dried over anhydrous sodium sulfate, and concentrated *in vacuo.* To the obtained residue were added ethyl acetate (10 mL) and then hexane (100 mL). The mixture was treated with an ultrasonication to give a precipitated solid. The solid was collected on a filter, washed with hexane, and dried *in vacuo* to give the title compound (1.53 g).
LC-MS, m/z; 372 $[M+H]^+$

Reference example 2: 1-[(2-amino-5-1[5-(trifluoromethyl)pyridin-2-yl]oxy}phenyl)amino]-2-methylpropan-2-ol

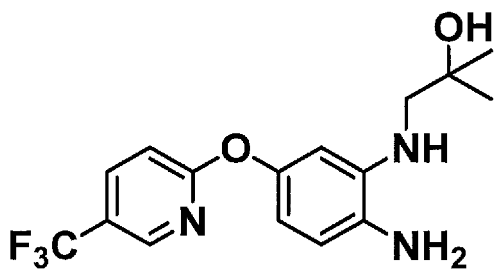

To a solution of the compound of Reference example 1 (500 mg) in methanol (6.7 mL) was added formic acid (0.52 mL). And zinc (440 mg) was gradually added thereto at 0°C. The reaction mixture was stirred at room temperature for 1 hour. The reaction solution was filtrated with Celite and concentrated *in vacuo.* To the obtained residue were added saturated aqueous Rochelle salt and ethyl acetate, and the desired product was extracted in the organic layer. The organic layer was washed with brine, dried over anhydrous sodium sulfate, and concentrated *in vacuo.* The obtained residue was purified by silica gel column chromatography (chloroform/methanol) to give the title compound (146 mg).
LC-MS, m/z; 342 $[M+H]^+$

Reference examples 3, 4, and 5:

The compounds of Reference examples 3 - 5 shown below were prepared with each corresponding starting material in the same manner as Reference example 1.

| Reference example | Structure | LC-MS,m/z |
|---|---|---|
| 3 | 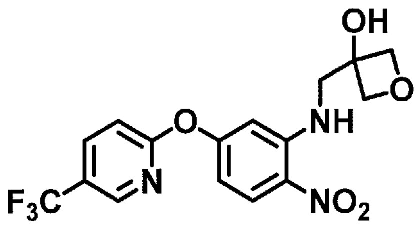 | 386 $[M+H]^+$ |
| 4 | 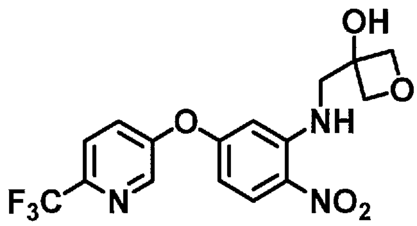 | 386 $[M+H]^+$ |
| 5 | 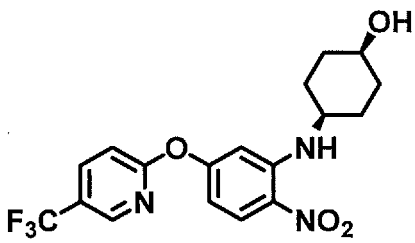 | 398 $[M+H]^+$ |

Reference example 6: 3-{[(5-fluoro-2-nitrophenyl)amino]methyl}oxetan-3-ol

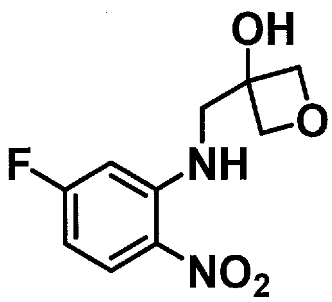

To a solution of 3-(aminomethyl)oxetan-3-ol (960 mg) in NMP (20 mL) were added 2,4-difluoronitrobenzene (1.48 g) and diisopropylethylamine (1.95 mL), and the mixture was stirred at room temperature for 9 hours. To the reaction solution were added water and ethyl acetate, and the desired product was extracted in the organic layer. The

organic layer was washed with brine, dried over anhydrous sodium sulfate, and concentrated *in vacuo.* The obtained residue was purified by silica gel column chromatography (hexane/ethyl acetate) to give the title compound (1.44 g).
LC-MS, m/z; 243 [M+H]$^+$

Reference example 7: 3-[({2-nitro-5-[4-(trifluoromethoxy)phenoxy]phenyl}amino)methyl]oxetan-3-ol

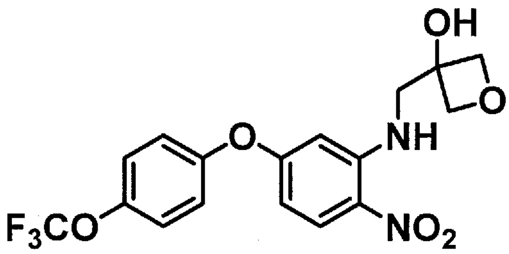

To a solution of the compound of Reference example 6 (529 mg) in NMP (10 mL) were added 4- (trifluoromethoxy) phenol (324 mg) and cesium carbonate (771 mg), and the mixture was stirred at 100°C for 4 hours. To the reaction solution were water and ethyl acetate, and the desired product was extracted in the organic layer. The organic layer was washed with brine, dried over anhydrous sodium sulfate, and concentrated *in vacuo.* The obtained residue was purified by silica gel column chromatography (hexane/ethyl acetate) to give the title compound (1.44 g).
LC-MS, m/z; 401 [M+H]$^+$

Reference example 8: 1-[(5-fluoro-2-nitrophenyl)amino]-2-methylpropan-2-ol

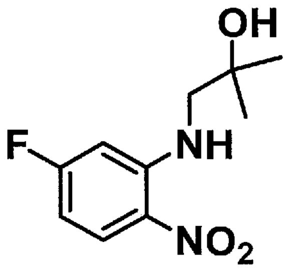

The title compound (1.1 g) was prepared with 1-amino-2-methylpropan-2-ol (500 mg) in the same manner as Reference example 6.
LC-MS, m/z; 229 [M+H]$^+$

Reference example 9: 1-{[5-(4-fluorophenoxy)-2-nitrophenyl]amino}-2-methylpropan-2-ol

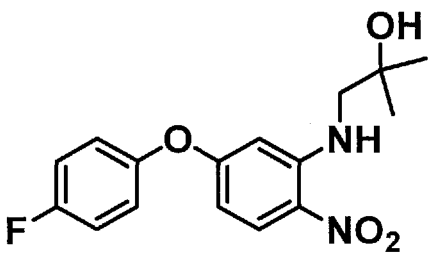

The title compound (1.33 g) was prepared with the compound of Reference example 8 (1.1 g) and 4-fluorophenol

(450 mg) in the same manner as Reference example 7.
LC-MS, m/z; 321 [M+H]$^+$

Reference example 10: 3-({[5-(4-fluorophenoxy)-2-nitrophenyl]amino}methyl)oxetan-3-ol

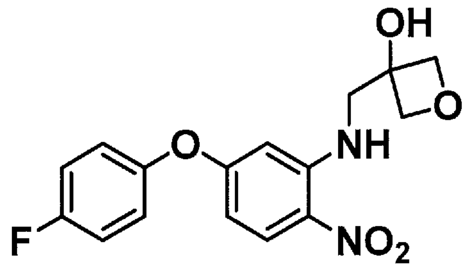

The title compound (354 mg) was prepared with 4-fluorophenol (116 mg) in the same manner as Reference example 7.
LC-MS, m/z; 335 [M+H]$^+$

Reference example 11: 3-({[5-(4-chlorophenoxy)-2-nitrophenyl]amino}methyl)oxetan-3-ol

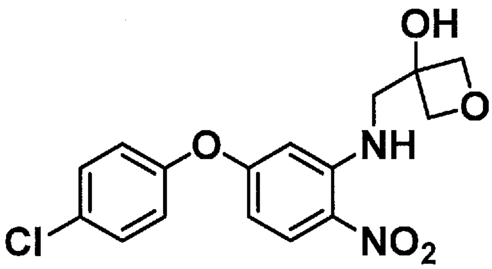

The title compound (416 mg) was prepared with 4-chlorophenol (150 mg) in the same manner as Reference example 7.
LC-MS, m/z; 351 [M+H]$^+$

Reference examples 12, 13, 14, 15, 16, 17, and 18:

The compounds of Reference examples 12, 13, 14, 15, 16, 17, and 18 shown below were prepared with each corresponding starting material in the same manner as Reference example 2.

| Reference example | Structure | Starting material | LC-MS,m/z |
|---|---|---|---|
| 12 | 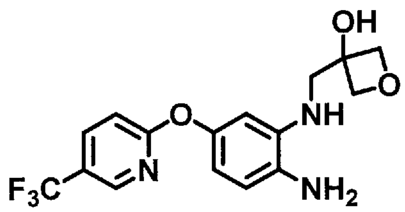 | Reference example 3 | 356 [M+H]$^+$ |

(continued)

| Reference example | Structure | Starting material | LC-MS,m/z |
|---|---|---|---|
| 13 | 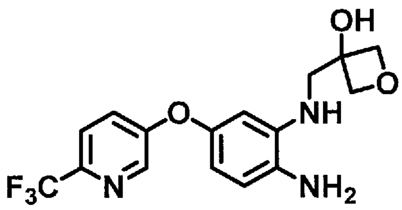 | Reference example 4 | 356 $[M+H]^+$ |
| 14 | 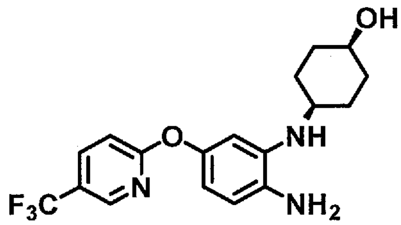 | Reference example 5 | 368 $[M+H]^+$ |
| 15 | 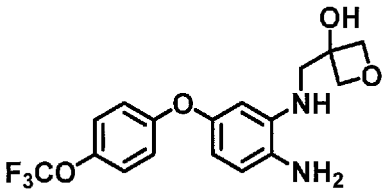 | Reference example 7 | 371 $[M+H]^+$ |
| 16 | 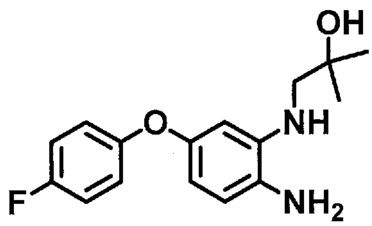 | Reference example 9 | 291 $[M+H]^+$ |
| 17 | 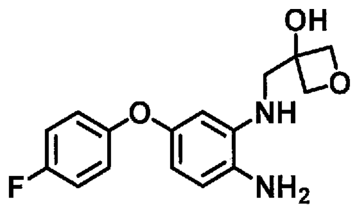 | Reference example 10 | 305 $[M+H]^+$ |
| 18 | 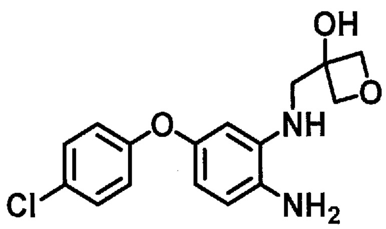 | Reference example 11 | 321 $[M+H]^+$ |

Reference example 19: 1-[(5-bromo-2-nitrophenyl)amino]-2-methylpropan-2-ol

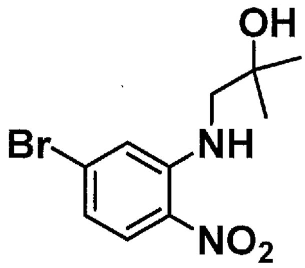

To a solution of 4-bromo-2-fluoronitrobenzene (5.0 g) in NMP (40 mL) were added 1-amino-2-methylpropan-2-ol (2.43 g) and diisopropylethylamine (5.95 mL), and the mixture was stirred at 100°C for 3 hours. To the reaction solution were water and ethyl acetate, and the desired product was extracted in the organic layer. The organic layer was washed with brine, dried over anhydrous sodium sulfate, and concentrated in *vacuo.* The obtained residue was purified by trituration with hexane and ethyl acetate to give the title compound (5.18 g).
LC-MS, m/z; 289 $[M]^+$, 291 $[M+2]^+$

Reference example 20: 1-[(2-amino-5-bromophenyl)amino]-2-methylpropan-2-ol

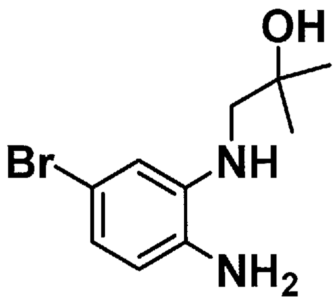

To a solution of the compound of Reference example 19 (2.65 g) in THF (30 mL) was added 3 % Pt sulfide on carbon (1.3 g), and the mixture was stirred under hydrogen atmosphere at room temperature for 10 hours. The reaction solution was filtrated with Celite and concentrated *in vacuo.* The obtained residue was purified by trituration with hexane and ethyl acetate to give the title compound (2.09 g).
LC-MS, m/z; 259 $[M]^+$, 261 $[M+2]^+$

Reference example 21: 6-bromo-1-(2-hydroxy-2-methylpropyl)-1,3-dihydro-2H-benzimidazol-2-one

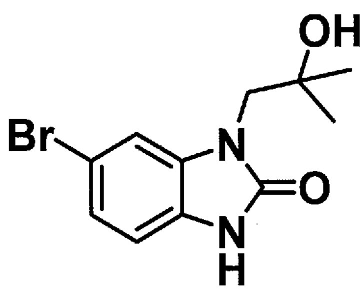

To a solution of the compound of Reference example 20 (1.51 g) in THF (60 mL) was added N,N'-carbonyldiimidazole (1.13 g) in ice bath, and the mixture was stirred for 8 hours warming gradually to room temperature. The reaction solution was concentrated *in vacuo,* and the obtained residue was purified by silica gel column chromatography (chloroform/methanol) to give the title compound (1.16 g).
LC-MS, m/z; 285 $[M]^+$, 287 $[M+2]^+$

Reference example 22: 1-[(4-bromo-2-nitrophenyl)amino]-2-methylpropan-2-ol

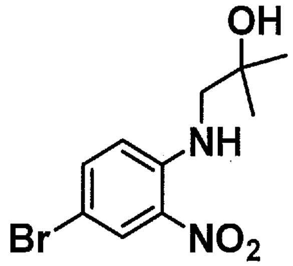

The title compound (1.8 g) was prepared with 5-bromo-2-fluoronitrobenzene (2.06 g) in the same manner as Reference example 19.
LC-MS, m/z; 289 $[M]^+$, 291 $[M+2]^+$

Reference example 23: 1-[(2-amino-4-bromophenyl)amino]-2-methylpropan-2-ol

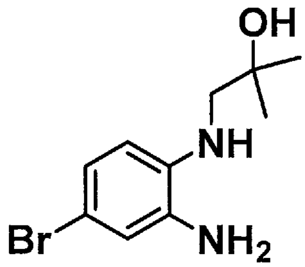

The title compound (930 mg) was prepared with the compound of Reference example 22 (416 mg) in the same manner as Reference example 20.
LC-MS, m/z; 259 $[M]^+$, 261 $[M+2]^+$

Reference example 24: 1-{[4-(benzyloxy)-2-nitrophenyl]amino}-2-methylpropan-2-ol

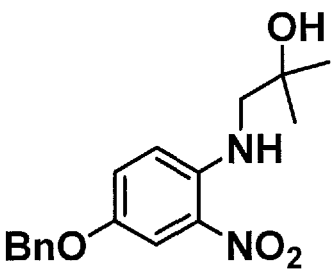

To a solution of 4-(benzyloxy)-1-fluoro-2-nitrobenzene (1.1 g) in NMP (9 mL) were added 1-amino-2-methylpropan-2-ol (476 mg) and diisopropylethylamine (2.72 mL), and the mixture was stirred at 110°C for 2 hours. To the reaction solution were water and ethyl acetate, and the desired product was extracted in the organic layer. The organic layer was washed with brine, dried over anhydrous sodium sulfate, and concentrated *in vacuo.* The obtained residue was purified by silica gel column chromatography (hexane/ethyl acetate) to give the title compound (1.4 g).
LC-MS, m/z; 317 $[M+H]^+$

Reference example 25: 1-{[2-amino-4-(benzyloxy)phenyl]amino}-2-methylpropan-2-ol

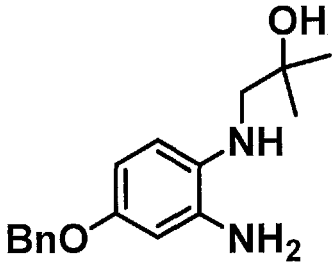

The title compound (0.92 g) was prepared with the compound of Reference example 24 (1.4 g) in the same manner as Reference example 2.
LC-MS, m/z; 287 $[M+H]^+$

Reference example 26: 3-({[4-(benzyloxy)-2-nitrophenyl]amino}methyl)oxetan-3-ol

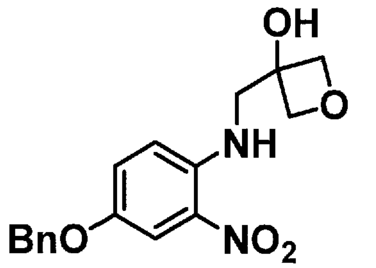

The title compound (2.95 g) was prepared with 3-(aminomethyl)oxetan-3-ol (1.25 g) in the same manner as Reference example 24.
LC-MS, m/z; 331 $[M+H]^+$

Reference example 27: 3-({[2-amino-4-(benzyloxy)phenyl]amino}methyl)oxetan-3-ol

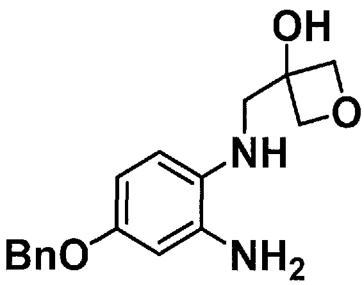

The title compound (1.2 g) was prepared with the compound of Reference example 26 (2.92 g) in the same manner as Reference example 2.
LC-MS, m/z; 301 $[M+H]^+$

Reference examples 28, 29, and 30:

The compounds of Reference examples 28, 29, and 30 shown below were prepared with each corresponding starting material in the same manner as Reference example 21.

| Reference example | Structure | Starting material | LC-MS, m/z |
|---|---|---|---|
| 28 | 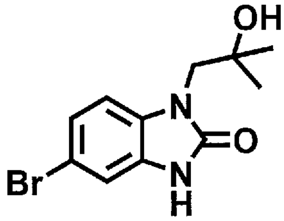 | Reference example 23 | 285 [M]$^+$, 287 [M+2]$^+$ |
| 29 | 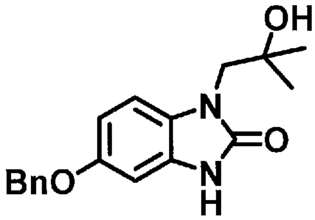 | Reference example 25 | 313 [M+H]$^+$ |
| 30 | 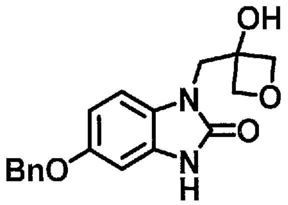 | Reference example 27 | 327 [M+H]$^+$ |

Reference example 31: 5-hydroxy-1-(2-hydroxy-2-methylpropyl)-1,3-dihydro-2H-benzimidazol-2-one

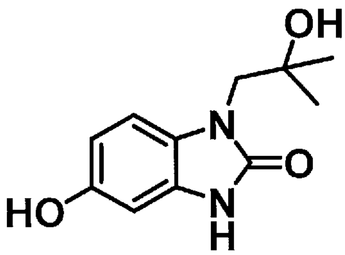

To a solution of the compound of Reference example 29 (750 mg) in methanol (6 mL) was added 10 % palladium-carbon (375 mg), and the mixture was stirred under hydrogen atmosphere for 6 hours. The reaction solution was filtrated with Celite and concentrated *in vacuo* to give the title compound (521 mg) .
LC-MS, m/z; 223 [M+H]$^+$

Reference example 32: 5-hydroxy-1-[(3-hydroxyoxetan-3-yl)methyl]-1,3-dihydro-2H-benzimidazol-2-one

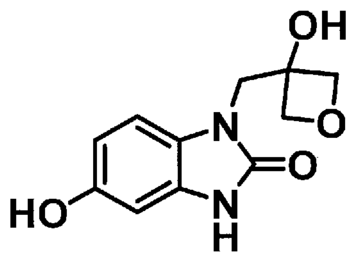

The title compound (0.43 g) was prepared with the compound of Reference example 30 (1.1 g) in the same manner as Reference example 31.
LC-MS, m/z; 237 $[M+H]^+$

Reference example 33: 1-(2-hydroxy-2-methylpropyl)-6-(4,4,5,5-tetramethyl-1,3,2-dioxaborolan-2-yl)-1,3-dihydro-2H-benzimidazol-2-one

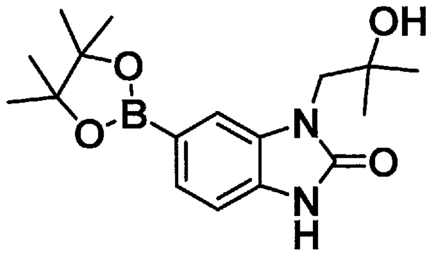

To a solution of the compound of Reference example 21 (124 mg) in dioxane (2 mL) were added bis(pinacolate)diboron (166 mg), potassium carbonate (85 mg), and [1,1'-bis(diphenylphosphino)ferrocene]palladium(II) chloride (35.5 mg), and the mixture was stirred at 110°C for 3 hours. To the reaction solution were water and ethyl acetate, and the desired product was extracted in the organic layer. The organic layer was washed with brine, dried over anhydrous sodium sulfate, and concentrated *in vacuo*. The obtained residue was purified by silica gel column chromatography (chloroform/methanol) to give the title compound (123 mg).
LC-MS, m/z; 333 $[M+H]^+$

Reference example 34: (3S)-3-amino-2-methylbutan-2-ol hydrochloride

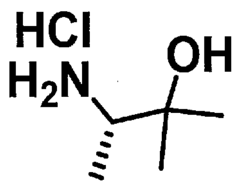

To a solution of N-α-t-butoxycarbonyl-L-alanine methyl ester (5.0 g) in diethyl ether (80 mL) was added methylmagnesium bromide (29.5 mL, in diethyl ether (3 mol/L)) in ice bath, and the mixture was stirred for 30 minutes. And then, the reaction mixture was warmed to room temperature, and stirred for 3 hours. To the reaction solution were added ethyl acetate and aqueous ammonium chloride, and the desired product was extracted in the organic layer. The organic layer was washed with brine, dried over anhydrous sodium sulfate, and concentrated *in vacuo*.
The obtained compound (5.4 g) was dissolved in ethyl acetate (40 mL), and 4 mol/L hydrochloric acid/1,4-dioxane solution (7.5 mL) was added thereto. The mixture was stirred at room temperature for 2 hours. The reaction solution was concentrated *in vacuo,* and toluene was added to the residue. The toluene solution was concentrated *in vacuo.* The procedure was repeated 3 times. The obtained residue was purified by trituration with hexane and ethyl acetate to give the title compound (3.08 g).

$^1$H-NMR (400 MHz, DMSO-D6) δ: 1.07 (3H, s), 1.11 (3H, d, J = 6.8 Hz), 1.15 (3H, s), 2.98 (1H, q, J = 6.8, 14 Hz), 5.11 (1H, s), 7.76 (3H, br).

Reference example 35: (3S)-2-methyl-3-[(2-nitro-5-{[5-(trifluoromethyl)pyridin-2-yl]oxy}phenyl)amino]butan-2-ol

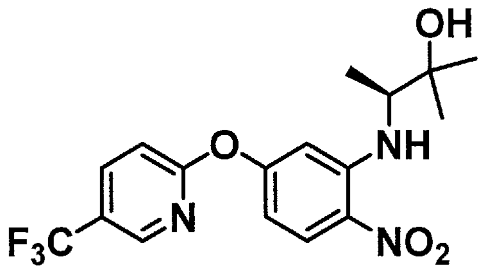

The title compound (500 mg) was prepared with the compound of Reference example 34 (300 mg) and 2-fluoro-5-(trifluoromethyl)pyridine (0.337 mL) in the same manner as Reference example 1.
LC-MS, m/z; 386 $[M+H]^+$

Reference example 36: (3S)-3-[(2-amino-5-{[5-(trifluoromethyl)pyridin-2-yl]oxy}phenyl)amino]-2-methylbutan-2-ol

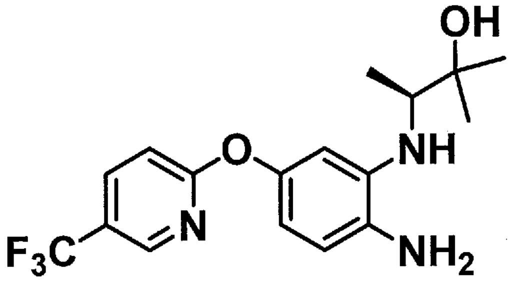

To a solution of the compound of Reference example 35 (26.1 g) in methanol (250 mL) was added hydrochloric acid (67.7 mL, 5 M). And zinc (440 mg) was gradually added thereto at 0°C. The reaction mixture was stirred at room temperature for 2 hours. The reaction solution was filtrated with Celite and concentrated *in vacuo.* To the obtained residue were added saturated aqueous Rochelle salt and ethyl acetate, and the desired product was extracted in the organic layer. The organic layer was washed with brine, dried over anhydrous sodium sulfate, and concentrated *in vacuo.* The obtained residue was purified by amino silica gel column chromatography (chloroform/methanol) to give the title compound (22.4 g).
LC-MS, m/z; 356 $[M+H]^+$

Reference example 37: (3S)-3-{[5-(4-fluorophenoxy)-2-nitrophenyl]amino}-2-methylbutan-2-ol

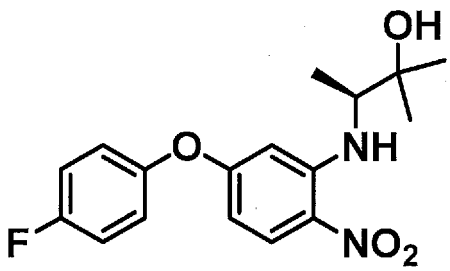

To a solution of 2,4-difluoronitrobenzene (316 mg) in NMP (4 mL) were added the compound of Reference example 34 (305 mg) and diisopropylethylamine(1.04 mL), and the mixture was stirred at room temperature for 3 hours. 4-Fluorophenol (334 mg) and cesium carbonate (971 mg) were added to the reaction solution, and the mixture was stirred at 100°C for 8 hours. The reaction solution was cooled to room temperature, and water and ethyl acetate were added to the reaction solution. The desired product was extracted in the organic layer. The organic layer was washed with brine, dried over anhydrous sodium sulfate, and concentrated *in vacuo.* The obtained residue was purified by silica gel column chromatography (hexane/ethyl acetate) to give the title compound (545 mg).
LC-MS, m/z; 335[M+H]$^+$

Reference example 38: (3S)-3-{ [2-amino-5-(4-fluorophenoxy)phenyl] amino}-2-methylbutan-2-ol

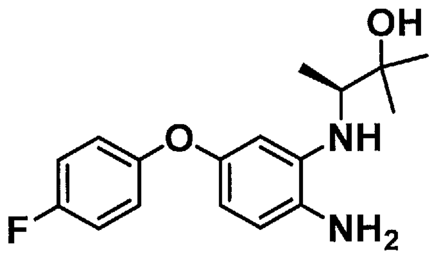

To a solution of the compound of Reference example 37 (545 mg) in methanol (8 mL) was added 10 % palladium-carbon (100 mg), and the mixture was stirred under hydrogen atmosphere for 6 hours. The reaction solution was filtrated with Celite and concentrated *in vacuo* to give the title compound (485 mg) .
LC-MS, m/z; 305 [M+H]$^+$

Reference example 39: cis-4-{[5-(4-fluorophenoxy)-2-nitrophenyl]amino}cyclohexanol

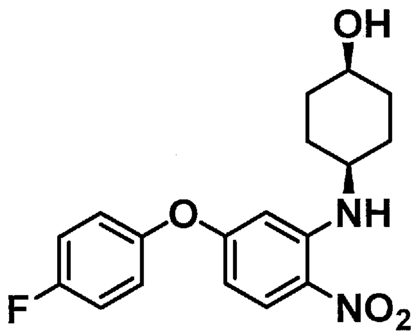

The title compound (755 mg) was prepared with cis-4-aminocyclohexanol hydrochloride (415 mg) in the same manner as Reference example 37.
LC-MS, m/z; 347 [M+H]$^+$

Reference example 40: cis-4-{[2-amino-5-(4-fluorophenoxy)phenyl]amino}cyclohexanol

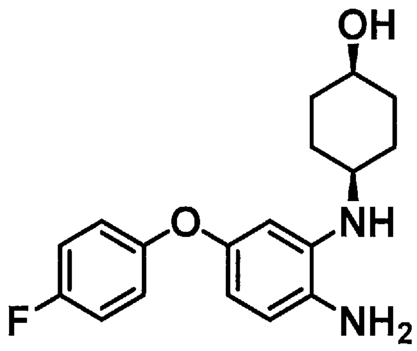

The title compound (625 mg) was prepared with the compound of Reference example 39 (755 mg) in the same manner as Reference example 38.
LC-MS, m/z; 317 [M+H]+

Examples 1 - 11:

The compounds of Examples 1 - 11 shown below were prepared with each corresponding starting material in the same manner as Reference example 21.

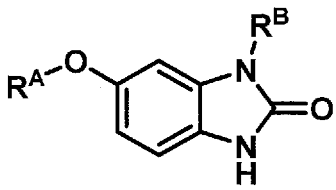

| Example | $R^A$- | $R^B$- | Starting material | Spectrum data |
|---|---|---|---|---|
| 1 | 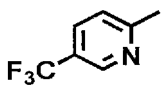 | 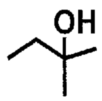 | Reference example 2 | $^1$H-NMR (400 MHz, DMSO-D6) δ: 1.11 (6H, s), 3.64 (2H, s), 4.55 (1H, s), 6.77 (1H, dd, J = 8.4, 2.4 Hz), 6.97 (1H, d, J = 8.4 Hz), 7.13-7.15 (2H, m), 8.18 (1H, dd, J = 9.2, 2.4 Hz), 8.54-8.55 (1H, m), 10.89 (1H, s). |
| 2 | 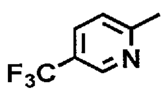 | 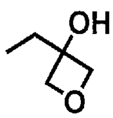 | Reference example 12 | $^1$H-NMR (400 MHz, DMSO-D6) δ: 4.11 (2H, s), 4.45 (2H, d, J = 6.8 Hz), 4.62 (2H, d, J = 6.8 Hz), 6.05 (1H, s), 6.85-6.88 (1H, m), 7.06 (1H, d, J = 8.4 Hz), 7.16 (1H, d, J=2.4 Hz), 7.22 (1H, d, J = 8.4 Hz), 8.26 (1H, dd, J = 9.2, 2.4 Hz), 8.61-8.62 (1H, m), 11.06 (1H, s). |
| 3 | 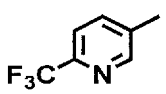 | 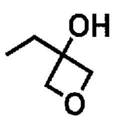 | Reference example 13 | $^1$H-NMR (400 MHz, DMSO-D6) δ: 4.06 (2H, s), 4.38 (2H, d, J = 6.8 Hz), 4.54 (2H, d, J = 6.8 Hz), 5.99 (1H, s), 6.83 (1H, dd, J = 8.0, 2.4 Hz), 7.02 (1H, d, J = 8.4 Hz), 7.13 (1H, d, J = 2.4 Hz), 7.42-7.45 (1H, m), 7.84 (1H, d, J = 8.4 Hz), 8.49 (1H, d, J = 2.4 Hz), 11.03 (1H, s). |

(continued)

| Example | $R^A$- | $R^B$- | Starting material | Spectrum data |
|---|---|---|---|---|
| 4 | 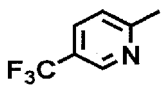 | 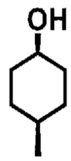 | Reference example 14 | $^1$H-NMR (400 MHz, DMSO-D6) δ: 1.39-1.42 (2H, m), 1.53-1.57 (2H, m), 1.74-1.77 (2H, m), 2.38-2.42 (2H, m), 3.83-3.86 (1H, m),4.13-4.20 (1H, m), 4.46 (1H, d, J = 3.2 Hz), 6.78 (1H, dd, J = 8.8, 2.4 Hz), 6.99 (1H, d, J = 8.0 Hz), 7.08 (1H, d, J = 2.4 Hz), 7.17 (1H, d, J = 8.0 Hz), 8.19 (1H, dd, J = 12.0, 2.8 Hz), 8.55-8.56 (1H, m), 10.89 (1H, s). |
| 5 | 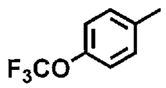 | 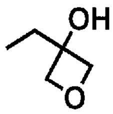 | Reference example 15 | $^1$H-NMR (400 MHz, DMSO-D6) δ: 4.04 (2H, s), 4.38 (2H, d, J = 6.8 Hz), 4.54 (2H, d, J = 6.8 Hz), 5.97 (1H, s), 6.69-6.72 (1H, m), 6.96-7.01 (3H, m), 7.04 (1H, d, J = 2.4 Hz), 7.30-7.33 (2H, m), 10.95 (1H, s). |
| 6 | 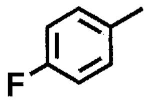 | 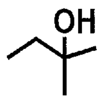 | Reference example 16 | $^1$H-NMR (400 MHz, DMSO-D6) δ: 1.10 (6H, s), 3.63 (2H, s), 4.59 (1H, s),6.62 (1H, dd, J = 8.4, 2.4 Hz), 6.92-6.96 (3H, m), 7.04 (1H, d, J = 2.4 Hz), 7.12-7.19 (2H, m), 10.84 (1H, s). |
| 7 | 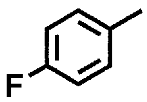 | 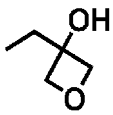 | Reference example 17 | $^1$H-NMR (400 MHz, DMSO-D6) δ: 4.02 (2H, s), 4.37 (2H, d, J = 6.8 Hz), 4.54 (2H, d, J = 6.8 Hz), 5.98 (1H, s), 6.64 (1H, d, J = 8.4, 2.4 Hz), 6.93-6.98 (4H, m), 7.14-7.18 (2H, m), 10.92 (1H, s). |
| 8 | 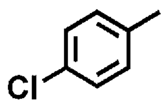 | 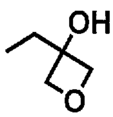 | Reference example18 | $^1$H-NMR (400 MHz, DMSO-D6) δ: 4.03 (2H, s), 4.37 (2H, d, J = 6.8 Hz), 4.54 (2H, d, J = 6.8 Hz), 5.98 (1H, s), 6.67-6.70 (1H, m), 6.91-6.95 (2H, m), 6.96 (1H, d, J = 8.4 Hz), 7.00 (1H, d, J = 2.4 Hz), 7.34-7.38 (2H, m), 10.94 (1H, s). |
| 9 | 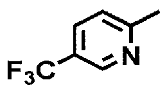 | 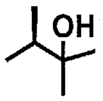 | Reference example 36 | $^1$H-NMR (400 MHz, DMSO-D6) δ: 1.21 (3H, s), 1.33 (3H, s), 1.55 (3H, d, J = 7.3 Hz), 4.05 (1H, br s), 6.87 (1H, dd, J = 8.5, 2.4 Hz), 6.96 (1H, s), 7.01 (1H, d, J = 9.1 Hz), 7.09 (1H, d, J = 8.5 Hz), 7.89 (1H, dd, J = 8.5, 2.4 Hz), 8.41 (1H, d, J = 2.4 Hz), 9.03 (1H, s) . |
| 10 | 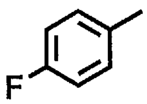 | 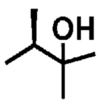 | Reference example 38 | $^1$H-NMR (400 MHz, DMSO-D6) δ: 1.19 (3H, s), 1.33 (3H, s), 1.54 (3H, d, J = 7.3 Hz), 4.00 (1H, br s), 6.71 (1H, dd, J = 8.5, 2.4 Hz), 6.81 (1H, br s), 6.89-6.94 (2H, m), 6.97-7.03 (3H, m), 9.52 (1H, s). |
| 11 | 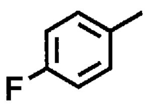 | 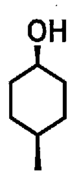 | Reference example 40 | $^1$H-NMR (400 MHz, DMSO-D6) δ: 1.42 (1H, d, J = 3.0 Hz), 1.62-1.73 (4H, m), 1.94 (2H, d, J = 13.4 Hz), 2.46-2.57 (2H, m), 4.12 (1H, d, J = 2.4 Hz), 4.28-4.38 (1H, m), 6.64 (1H, dd, J = 8.5, 2.4 Hz), 6.89-7.01 (5H, m), 7.02 (1H, d, J = 2.4 Hz), 9.15 (1H, s). |

Example 12: 1-ethyl-3-(2-hydroxy-2-methylpropyl)-5-{[S-(trifluoromethyl)pyridin-2-yl]oxy}-1,3-dihydro-2H-benzimidazol-2-one

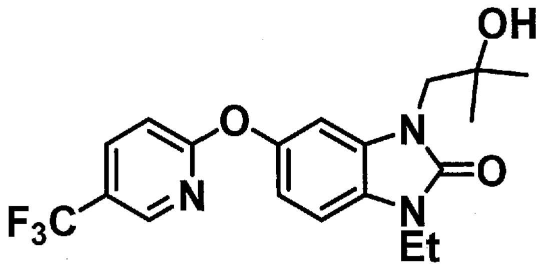

To a solution of the compound of Example 1 (100 mg) in DMSO (1 mL) was added sodium hydride (13.1 mg), and the mixture was stirred for 1 hour. Further, ethyl iodide (37 μL) was added to the reaction mixture, and the mixture was stirred at 60°C for 4 hours. To the reaction solution were water and ethyl acetate, and the desired product was extracted in the organic layer. The organic layer was washed with brine, dried over anhydrous sodium sulfate, and concentrated in *vacuo.* The obtained residue was purified by silica gel column chromatography (hexane/ethyl acetate) to give the title compound (93 mg).
LC-MS, m/z; 396 $[M+H]^+$ (RT 0.934 min)
$^1$H-NMR (400 MHz, DMSO-D6) δ: 1.10 (6H, s), 1.22 (3H, t, J = 7.0 Hz), 3.68 (2H, s), 3.89 (2H, q, J = 14.0, 6.8 Hz), 4.57 (1H, s), 6.86 (1H, dd, J = 8.4, 2.4 Hz), 7.15-7.22 (3H, m), 8.19 (1H, dd, J = 8.4, 2.4 Hz), 8.53-8.55 (1H, m).

Example 13: 3-(2-hydroxy-2-methylpropyl)-1-methyl-5-{[5-(trifluoromethyl)pyridin-2-yl]oxy}-1,3-dihydro-2H-benzimidazol-2-one

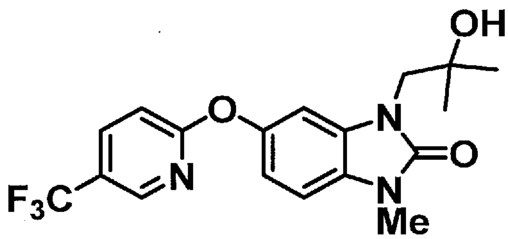

The title compound (133 mg) was prepared with the compound of Example 1 (180 mg) and methyl iodide (61 pL) in the same manner as Example 12.
LC-MS, m/z; 382 $[M+H]^+$ (RT 0.803 min)
$^1$H-NMR (400 MHz, DMSO-D6) δ: 1.23 (6H, s), 3.44 (3H, s), 3.84 (2H, s), 6.88-6.91 (1H, m), 6.95 (1H, d, J = 2.4 Hz), 6.99 (1H, d, J = 2.8 Hz), 7.01 (1H, d, J = 2.8 Hz), 7.88 (1H, dd, J = 8.8, 2.8 Hz), 8.38-8.39 (1H, m).

Examples 14 - 18:

The compounds of Examples 14 - 18 shown below were prepared with each corresponding starting material in the same manner as Example 13.

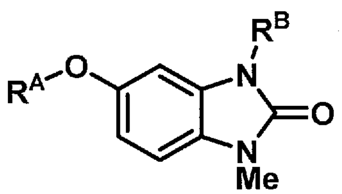

| Example | $R^A$- | $R^B$- | Starting material | Spectrum data |
|---|---|---|---|---|
| 14 | 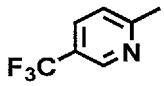 | 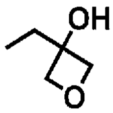 | Example 2 | $^1$H-NMR (400 MHz, DMSO-D6) δ: 3.36 (3H, s), 4.09 (2H, s), 4.38 (2H, d, J = 6.8 Hz), 4.56 (2H, d, J = 6.8 Hz), 5.97 (1H, s), 6.89 (1H, dd, J = 8.8, 2.4 Hz), 7.15 (1H, d, J = 2.4 Hz), 7.17 (2H, d, J = 8.8 Hz), 8,18-8.21 (1H, m), 8.53-8.54 (1H, m). |
| 15 | 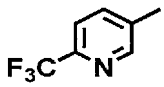 | 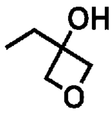 | Example 3 | $^1$H-NMR (400 MHz, DMSO-D6) δ: 3.36 (3H, s), 4.11 (2H, s), 4.38 (2H, d, J = 6.8 Hz), 4.55 (2H, d, J = 6.8 Hz), 5.99 (1H, s), 6.92-6.95 (1H, m), 7.19 (1H, d, J = 2.4 Hz), 7.21 (1H, d, J = 8.4 Hz), 7.44 (1H, dd, J = 8.8, 2.4 Hz), 7.85 (1H, d, J = 8.4 Hz), 8.49 (1H, d, J = 2.4 Hz). |
| 16 | 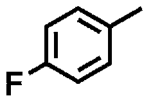 | 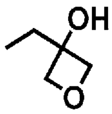 | Example 7 | $^1$H-NMR (400 MHz, DMSO-D6) δ: 3.30 (3H, s), 4.08 (2H, s), 4.37 (2H, d, J = 6.4 Hz), 4.54 (2H, d, J = 6.4 Hz), 5.97 (1H, s), 6.74 (1H, dd, J = 8.4, 2.4 Hz), 6.93-6.97 (2H, m), 7.03-7.04 (1H, m), 7.12 (1H, d, J = 8.4 Hz), 7.14-7.18 (2H, m). |
| 17 | 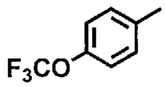 | 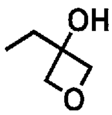 | Example 5 | $^1$H-NMR (400 MHz, DMSO-D6) δ: 3.35 (3H, s), 4.09 (2H, s), 4.38 (2H, d, J = 6.8 Hz), 4.55 (2H, d, J = 6.8 Hz), 5.97 (1H, s), 6.81 (1H, dd, J = 8.0, 2.4 Hz), 6.98-7.02 (2H, m), 7.10 (1H, d, J = 2.4 Hz), 7.16 (1H, d, J = 8.4 Hz), 7.31-7.33 (2H, m). |
| 18 | 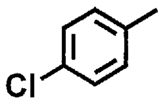 | 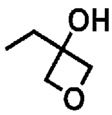 | Example 8 | $^1$H-NMR (400 MHz, DMSO-D6) δ: 3.34 (3H, s), 4.09 (2H, s), 4.37 (2H, d, J = 6.4 Hz), 4.54 (2H, d, J = 6.4 Hz), 5.97 (1H, s), 6.78 (1H, dd, J = 8.8, 2.4 Hz), 6.92-6.94 (2H, m), 7.06 (1H, d, J = 2.8 Hz), 7.14 (1H, d, J = 8.8Hz), 7.35-7.38 (2H, m). |

Example 19: 1-(2-hydroxy-2-methylpropyl)-6-[6-(trifluoromethyl)pyridin-3-yl]-1,3-dihydro-2H-benzimidazol-2-one

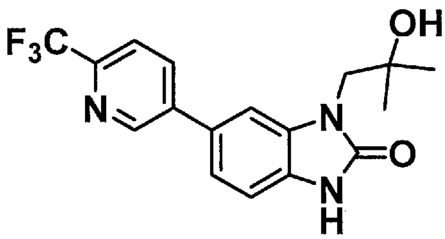

The compound of Reference example 21 (100 mg) was dissolved in a mixture solvent of distilled water (0.5 mL) and 1,4-dioxane (1.5 mL), and tetrakis(triphenylphosphine)palladium(0) (40.5 mg), 2-(trifluoromethyl)pyridin-5-ylboronic acid (100 mg), and potassium carbonate (145 mg) were added thereto. The mixture was stirred at 100°C for 6 hours. To the reaction solution were water and ethyl acetate, and the desired product was extracted in the organic layer. The organic layer was washed with brine, dried over anhydrous sodium sulfate, and concentrated *in vacuo.* The obtained residue was purified by amino silica gel column chromatography (chloroform/methanol) and by trituration with hexane and ethyl acetate to give the title compound (48 mg).
LC-MS, m/z; 352 $[M+H]^+$ (RT 0.802 min)
$^1$H-NMR (400 MHz, DMSO-D6) δ: 1.15 (6H, s), 3.77 (2H, s), 4.67 (1H, s), 7.10 (1H, d, J = 7.9 Hz), 7.42 (1H, dd, J = 7.9, 1.8 Hz), 7.71 (1H, d, J = 1.8 Hz), 7.95 (1H, d, J = 8.5 Hz), 8.29 (1H, dd, J = 8.5, 2.4 Hz), 9.05 (1H, d, J = 2.4 Hz), 11.05 (1H, s).

Example 20: 1-(2-hydroxy-2-methylpropyl)-6-[5-(trifluoromethyl)pyridin-2-yl]-1,3-dihydro-2H-benzimidazol-2-one

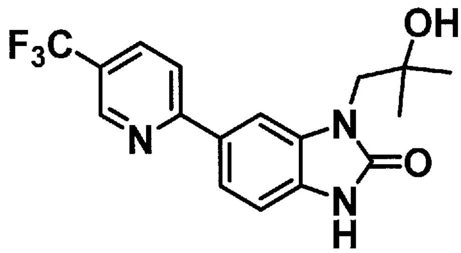

The compound of Reference example 33 (123 mg) was dissolved in a mixture solvent of distilled water (1 mL) and 1,4-dioxane (3 mL), and tetrakis(triphenylphosphine)palladium(0) (42.8 mg), 2-bromo-5-(trifluoromethyl)pyridine (126 mg), and potassium carbonate (154 mg) were added thereto. The mixture was stirred at 100°C for 6 hours. To the reaction solution were water and ethyl acetate, and the desired product was extracted in the organic layer. The organic layer was washed with brine, dried over anhydrous sodium sulfate, and concentrated *in vacuo*. The obtained residue was purified by amino silica gel column chromatography (chloroform/methanol) and by trituration with hexane and ethyl acetate to give the title compound (64 mg).
LC-MS, m/z; 352 $[M+H]^+$ (RT 0.848 min)
$^1$H-NMR (400 MHz, DMSO-D6) δ: 1.16 (6H, s), 3.76 (2H, s), 4.70 (1H, s), 7.08 (1H, d, J = 8.2 Hz), 7.84 (1H, dd, J = 8.2, 1.8 Hz), 8.05 (1H, d, J = 1.8 Hz), 8.12 (1H, d, J = 8.5 Hz), 8.22 (1H, dd, J = 8.5, 1.8 Hz), 8.97 (1H, s), 11.09 (1H, s).

Examples 21 - 24:

The compounds of Examples 21 - 24 shown below were prepared with each corresponding starting material in the same manner as Example 19.

| Example | Structure | Starting material | Spectrum data |
|---|---|---|---|
| 21 | 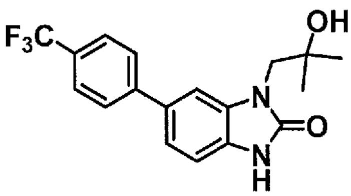 | Reference example 21 | $^1$H-NMR (400 MHz, DMSO-D6) δ: 1.15 (6H, s), 3.76 (2H, s), 4.68 (1H, s), 7.06 (1H, d, J = 7.9 Hz), 7.34 (1H, dd, J = 8.2, 1.5 Hz), 7.63 (1H, d, J = 1.8 Hz), 7.77-7.85 (4H, m), 10.98 (1H, s). |
| 22 | 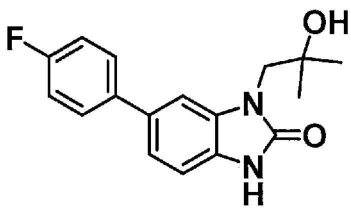 | Reference example 21 | $^1$H-NMR (400 MHz, DMSO-D6) δ: 1.15 (6H, s), 3.74 (2H, s), 4.67 (1H, s), 7.01 (1H, d, J = 7.9 Hz), 7.20-7.29 (3H, m), 7.52 (1H, d, J = 1.8 Hz), 7.61-7.65 (2H, m), 10.89 (1H, s). |
| 23 | 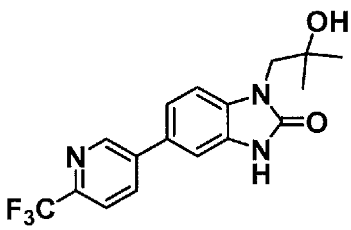 | Reference example 28 | $^1$H-NMR (400 MHz, DMSO-D6) δ: 1.14 (6H, s), 3.72 (2H, s), 4.67 (1H, s), 7.33-7.45 (3H, m), 7.92 (1H, d, J = 8.5 Hz), 8.30 (1H, dd, J = 8.5, 2.4 Hz), 9.04 (1H, d, J = 1.8 Hz), 11.08 (1H, s). |

(continued)

| Example | Structure | Starting material | Spectrum data |
|---|---|---|---|
| 24 | 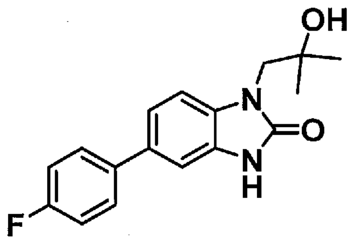 | Reference example 28 | $^1$H-NMR (400 MHz, DMSO-D6) δ: 1.14 (6H, s), 3.69 (2H, s), 4.65 (1H, s), 7.14 (1H, d, J = 1.8 Hz), 7.22-7.30 (4H, m), 7.60-7.64 (2H, m), 10.93 (1H, s). |

Example 25: 1-(2-hydroxy-2-methylpropyl)-5-[4-(trifluoromethoxy)phenoxy]-1,3-dihydro-2H-benzimidazol-2-one

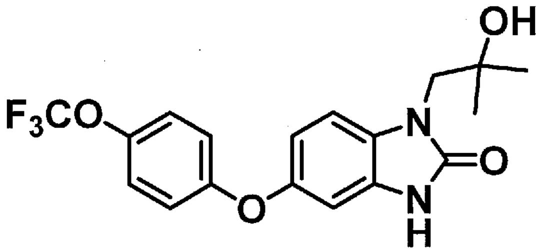

The compound of Reference example 31 (50 mg) was dissolved in DMF (1 mL), and (4-(trifluoromethoxy)phenyl)boronic acid (139 mg), triethylamine (94 μL), and copper acetate (12.3 mg) were added thereto. The mixture was stirred at 80°C for 9 hours in open air. To the reaction solution were water and ethyl acetate, and the desired product was extracted in the organic layer. The organic layer was washed with brine, dried over anhydrous sodium sulfate, and concentrated *in vacuo.* The obtained residue was purified by silica gel column chromatography (hexane/ethyl acetate) to give the title compound (8 mg).
LC-MS, m/z; 383 $[M+H]^+$ (RT 0.866 min)
$^1$H-NMR (400 MHz, DMSO-D6) δ: 1.16 (6H, s), 3.71 (2H, s), 4.65 (1H, s), 6.48 (1H, d, J = 2.4 Hz), 6.52 (1H, dd, J = 8.8, 2.4 Hz), 7.16 (1H, d, J = 8.4 Hz), 7.29-7.31 (1H, m), 7.55-7.58 (2H, m), 7.66-7.68 (1H, m), 9.10 (1H, s).

Example 26: 1-(2-hydroxy-2-methylpropyl)-5-{[5-(trifluoromethyl)pyridin-2-yl]oxy}-1,3-dihydro-2H-benzimidazol-2-one

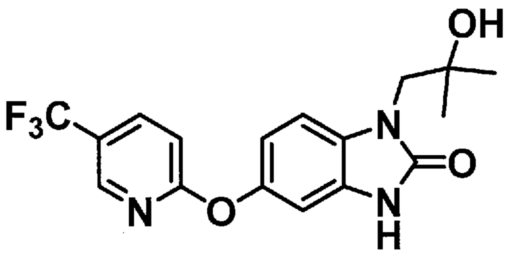

To a solution of the compound of Reference example 31 (50 mg) in NMP (1 mL) were added 2-fluoro-5-(trifluoromethyl)pyridine (45 mg) and cesium carbonate (110 mg), and the mixture was stirred at 110°C for 4 hours. To the reaction solution were water and ethyl acetate, and the desired product was extracted in the organic layer. The organic layer was washed with brine, dried over anhydrous sodium sulfate, and concentrated *in vacuo.* The obtained residue was purified by silica gel column chromatography (hexane/ethyl acetate) to give the title compound (9.0 mg). LC-MS, m/z; 368 $[M+H]^+$ (RT 0.734 min)

$^1$H-NMR (400 MHz, DMSO-D6) δ: 1.14 (6H, s), 3.68 (2H, s), 4.66 (1H, s), 6.79-6.80 (2H, m), 7.16 (1H, d, J = 8.4 Hz), 7.24-7.26 (1H, m), 8.18 (1H, dd, J = 8.8, 2.4 Hz), 8.54-8.55 (1H, m), 10.93 (1H, s).

Example 27: 1-[(3-hydroxyoxetan-3-yl)methyl]-5-[4-(trifluoromethoxy)phenoxy]-1,3-dihydro-2H-benzimidazol-2-one

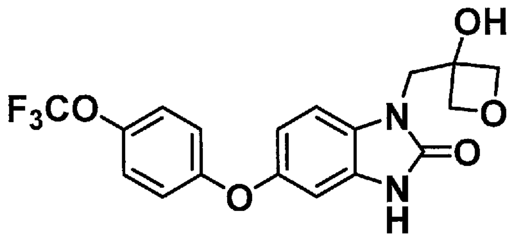

The title compound (31 mg) was prepared with the compound of Reference example 32 (150 mg) in the same manner as Example 25.
LC-MS, m/z; 397 $[M+H]^+$ (RT 0.893 min)
$^1$H-NMR (400 MHz, DMSO-D6) δ: 4.11 (2H, s), 4.43 (2H, d, J = 6.8 Hz), 4.60 (2H, d, J = 6.8 Hz), 6.07 (1H, s), 6.48 (1H, d, J = 2.4 Hz), 6.52-6.55 (1H, m), 7.13 (1H, d, J = 8.4 Hz), 7.56-7.59 (2H, m), 7.67-7.69 (2H, m), 9.13 (1H, s).

Example 28: 1-[(3-hydroxyoxetan-3-yl)methyl]-3-methyl-5-[4-(trifluoromethoxy)phenoxy]-1,3-dihydro-2H-benzimidazol-2-one

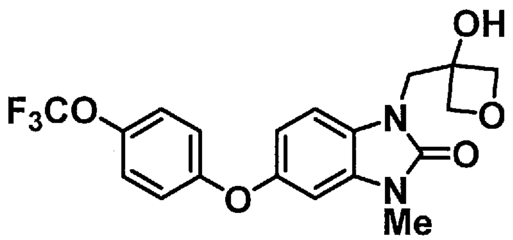

The title compound (12 mg) was prepared with the compound of Example 27 (50 mg) in the same manner as Example 13.
LC-MS, m/z; 411 $[M+H]^+$ (RT 1.068 min)
$^1$H-NMR (400 MHz, DMSO-D6) δ: 3.70 (3H, s), 4.15 (2H, s), 4.44 (2H, d, J = 6.8 Hz), 4.601 (2H, d, J = 6.8 Hz), 6.09 (1H, s), 6.63 (1H, d, J = 2.4 Hz), 6.73 (1H, dd, J = 8.8, 2.4 Hz), 7.26 (1H, d, J = 8.8 Hz), 7.56 (2H, d, J = 8.4 Hz), 7.70-7.72 (2H, m).

Pharmacological test

Measurement of Na ion current in voltage-dependent Na channel gene expressed cell
Nav 1.7 current was measured by automated patch clamp assay using cells stably-expressing human SCN9A.

Cells stably-expressing human SCN9A

Tetracycline-induced cells stably-expressing SCN9A were obtained from ChanTest Corporation. The cells were passaged in Ham's F-12 medium containing 10 % fetal bovine serum, 100 units/mL Penicillin-Streptomycin, 0.01 mg/mL Blasticidin, and 400 μg/ mL Zeocin. The cell seeded on a glass slide was cultured in Ham's F-12 medium containing 1 pg/mL tetracycline, 1 μg/mL sodium butyrate, 10 % fetal bovine serum, and 100 units/mL Penicillin-Streptomycin. Next day, the Na ion current was measured by automated patch clamp assay.

Electrophysiologic measurement of Na ion current The Na ion current was measured by automated patch clamp assay using the following extracellular solution and intracellular solution.

Extracellular solution (mmol/L): NaCl 130, $MgCl_2$ 2, $CaCl_2$ 2, $CdCl_2$ 0.1, $NiCl_2$ 0.1, Tetraethylammonium-Cl 18,

4-aminopyridine 1, HEPES (4-(2-hydroxyethyl)-1-piperazineethanesulfonic acid) 10, (adjusting pH 7.4 with NaOH)

Intracellular solution (mmol/L): CsF 120, EGTA (ethylene glycol tetraacetic acid) 10, NaCl 15, HEPES 10, (adjusting pH 7.2 with CsOH)

The control of the stimulating pulse and the data acquisition were carried out using EPC10 amplifier and Patch Master Software (HEKA). Data were sampled at 20 kHz, and low-pass filtered at 2.9 kHz. All the measurements were carried out at room temperature. The holding potential was set at a potential inactivating 50 % Nav 1.7 channel (around -60 mV), and depolarizing pulse of 20 milliseconds (around 0 mV) was given at a frequency of 10 Hz 30 times. The inhibitory rate of the test compounds was calculated based on the results of cells whose peak current was 500 pA or more when the first stimulating pulse was given and whose whole-cell parameter did not greatly vary until the end of the data acquisition. The inhibitory rate of the Na ion current by the test compounds was calculated according to the following calculating formula with the peak current value generated by the 30th depolarizing pulse.

Inhibitory rate of Na ion current (%) = 100 x [(Peak current value in the absence of Test Compound)-(Peak current value in the presence of Test Compound)]/(Peak current value in the absence of Test Compound)

Result:

The inhibitory rate of Na ion current by each Example compound was evaluated. The results showed that the compounds of the present invention exhibit the inhibitory effect for Nav 1.7. The inhibitory rate (%) wherein the concentration of each compound is 10 μmol/L is shown in the following table.

| Example | Inhibitory rate (%) | Example | Inhibitory rate (%) | Example | Inhibitory rate (%) |
|---|---|---|---|---|---|
| 1 | 33 | 2 | 26 | 3 | 22 |
| 4 | 39 | 5 | 86 | 6 | 40 |
| 7 | 40 | 8 | 80 | 9 | 27 |
| 10 | 50 | 11 | 65 | 12 | 14 |
| 13 | 38 | 14 | 39 | 15 | 46 |
| 16 | 61 | 17 | 87 | 18 | 86 |
| 19 | 8 | 20 | 29 | 21 | 96 |
| 22 | 43 | 23 | 36 | 24 | 45 |
| 25 | 42 | 26 | 32 | 27 | 38 |
| 28 | 39 | | | | |

Industrial Applicability

The compounds of the present invention can be used as a useful medicament for treating a disease involving Nav 1.7, for example, neuropathic pain, nociceptive pain, inflammatory pain, small-fiber neuropathy, erythromelalgia, paroxysmal extreme pain disorder, dysuria, and multiple sclerosis. Thus, the compounds of the present invention can be very useful pharmaceuticals.

## Claims

1. A compound of formula (I):

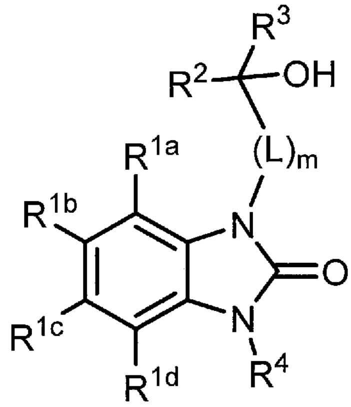

(I)

or a pharmaceutically acceptable salt thereof, wherein

$R^{1a}$, $R^{1b}$, $R^{1c}$, and $R^{1d}$ are independently hydrogen, halogen, $C_{1-4}$ alkyl, $C_{1-4}$ alkoxy (wherein the alkyl and the alkyl moiety in the alkoxy may be independently substituted with 1 to 5 substituents selected independently from the group consisting of halogen, hydroxy group, $C_{1-4}$ alkoxy optionally-substituted with 1 to 3 substituents selected independently from Substituent-group A, $C_{3-7}$ cycloalkyl optionally-substituted with 1 to 3 substituents selected independently from Substituent-group B, $C_{3-7}$ cycloalkoxy optionally-substituted with 1 to 3 substituents selected independently from Substituent-group B, and 3- to 7-membered non-aromatic heterocyclyl optionally-substituted with 1 to 3 substituents selected independently from Substituent-group B), $C_{3-7}$ cycloalkyl, $C_{3-7}$ cycloalkoxy (wherein the cycloalkyl and the cycloalkyl moiety in the cycloalkoxy may be independently substituted with 1 to 5 substituents selected independently from the group consisting of halogen, hydroxy group, $C_{1-4}$ alkyl optionally-substituted with 1 to 3 substituents selected independently from Substituent-group A, $C_{1-4}$ alkoxy optionally-substituted with 1 to 3 substituents selected independently from Substituent-group A, $C_{3-7}$ cycloalkyl optionally-substituted with 1 to 3 substituents selected independently from Substituent-group B, and $C_{3-7}$ cycloalkoxy optionally-substituted with 1 to 3 substituents selected independently from Substituent-group B), $C_{6-10}$ aryl, $C_{6-10}$ aryloxy, 5- to 12-membered heteroaryl, or 5- to 12-membered heteroaryloxy (wherein the aryl and the aryl moiety in the aryloxy, and the heteroaryl and the heteroaryl moiety in the heteroaryloxy may be independently substituted with 1 to 5 substituents selected independently from the group consisting of halogen, cyano, $C_{1-4}$ alkyl optionally-substituted with 1 to 3 substituents selected independently from Substituent-group A, $C_{1-4}$ alkoxy optionally-substituted with 1 to 3 substituents selected independently from Substituent-group A, $C_{3-7}$ cycloalkyl optionally-substituted with 1 to 3 substituents selected independently from Substituent-group B, $C_{3-7}$ cycloalkoxy optionally-substituted with 1 to 3 substituents selected independently from Substituent-group B, and 3- to 7-membered non-aromatic heterocyclyl optionally-substituted with 1 to 3 substituents selected independently from Substituent-group B), provided that at least one of $R^{1a}$, $R^{1b}$, $R^{1c}$ and $R^{1d}$ is the above $C_{6-10}$ aryl, $C_{6-10}$ aryloxy, 5- to 12-membered heteroaryl or 5- to 12-membered heteroaryloxy,
$R^2$ and $R^3$ are independently hydrogen, $C_{1-6}$ alkyl (which may be independently substituted with 1 to 5 substituents selected independently from the group consisting of cyano, halogen, hydroxy group, $C_{1-4}$ alkoxy optionally-substituted with 1 to 3 substituents selected independently from Substituent-group A, $C_{3-7}$ cycloalkyl optionally-substituted with 1 to 3 substituents selected independently from Substituent-group B, and $C_{3-7}$ cycloalkoxy optionally-substituted with 1 to 3 substituents selected independently from Substituent-group B), or $C_{3-10}$ cycloalkyl,
$R^4$ is hydrogen, $C_{1-4}$ alkyl, or $C_{3-4}$ cycloalkyl wherein the $C_{1-4}$ alkyl and the $C_{3-4}$ cycloalkyl may be substituted with 1 - 5 the same or different halogen atoms,
m is 1, 2, or 3,
L is $CR^7R^8$ provided that when m is 2 or 3, each $CR^7R^8$ is independently the same or different,
$R^7$ and $R^8$ are independently hydrogen, hydroxy group, $C_{1-4}$ alkyl, $C_{1-4}$ alkoxy (wherein the alkyl and the alkyl moiety in the alkoxy may be independently substituted with 1 to 3 substituents selected independently from the group consisting of halogen, hydroxy group, $C_{1-4}$ alkoxy optionally-substituted with 1 to 3 substituents selected independently from Substituent-group A, $C_{3-7}$ cycloalkyl optionally-substituted with 1 to 3 substituents selected independently from Substituent-group B, $C_{3-7}$ cycloalkoxy optionally-substituted with 1 to 3 substituents selected independently from Substituent-group B, and 3- to 7-membered non-aromatic heterocyclyl optionally-substituted with 1 to 3 substituents selected independently from Substituent-group B), $C_{3-7}$ cycloalkyl, or $C_{3-7}$ cycloalkoxy (wherein the cycloalkyl and the cycloalkyl moiety in the cycloalkoxy may be independently substituted with 1 to 3 substituents selected independently from the group consisting of halogen, hydroxy group, $C_{1-4}$ alkyl optionally-

substituted with 1 to 3 substituents selected independently from Substituent-group A, $C_{1-4}$ alkoxy optionally-substituted with 1 to 3 substituents selected independently from Substituent-group A, $C_{3-7}$ cycloalkyl optionally-substituted with 1 to 3 substituents selected independently from Substituent-group B, and $C_{3-7}$ cycloalkoxy optionally-substituted with 1 to 3 substituents selected independently from Substituent-group B), or
in $R^2$, $R^3$, and the hydroxy group bound to the carbon atom which is connected to $R^2$ and $R^3$,
$R^2$ and $R^3$ may be combined together with the carbon atom to which they are attached to form the following group of formula (II) with the hydroxy group

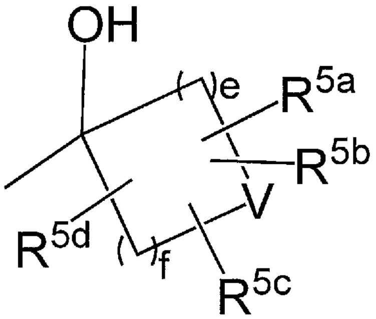
(II)

in formula (II),

e and f are independently 1, 2 or 3,
V is single bond or oxygen atom,
$R^{5a}$, $R^{5b}$, $R^{5c}$, and $R^{5d}$ are independently hydrogen, halogen, hydroxy group, $C_{1-4}$ alkyl, or $C_{1-4}$ alkoxy, wherein the alkyl and the alkyl moiety in the alkoxy may be independently substituted with 1 to 3 substituents selected independently from the group consisting of halogen, hydroxy group, $C_{1-4}$ alkoxy optionally-substituted with 1 to 3 substituents selected independently from Substituent-group A, $C_{3-7}$ cycloalkyl optionally-substituted with 1 to 3 substituents selected independently from Substituent-group B, $C_{3-7}$ cycloalkoxy optionally-substituted with 1 to 3 substituents selected independently from Substituent-group B, and 3- to 7-membered non-aromatic heterocyclyl optionally-substituted with 1 to 3 substituents selected independently from Substituent-group B, or
in $R^2$, $R^3$, the hydroxy group bound to the carbon atom which is connected to $R^2$ and $R^3$, and $CR^7R^8$ in L,
$R^2$ and $R^7$ may be combined together with the carbon atom to which they are attached to form the following group of formula (III) with $R^3$, the hydroxy group and $R^8$

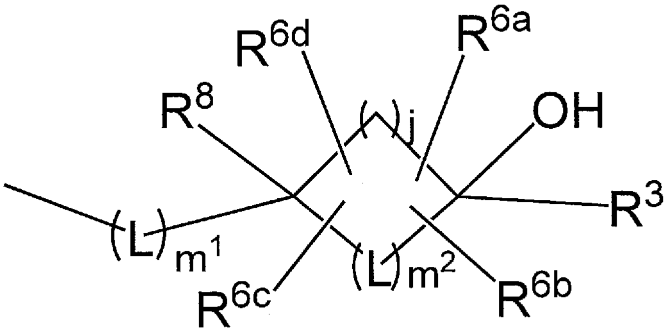
(III)

in formula (III),

$m^1$ is 0 or 1,
$m^2$ is 0 or 1 and j is 1, 2, 3 or 4 when $m^1$ is 1, or
$m^2$ is 0, 1 or 2 and j is 1, 2, 3 or 4 when $m^1$ is 0,
$R^8$ and L are as defined above,
$R^{6a}$, $R^{6b}$, $R^{6c}$, and $R^{6d}$ are independently hydrogen, halogen, hydroxy group, $C_{1-4}$ alkyl, or $C_{1-4}$ alkoxy, wherein the alkyl and the alkyl moiety in the alkoxy may be independently substituted with 1 to 3 substituents selected independently from the group consisting of halogen, hydroxy group, $C_{1-4}$ alkoxy optionally-substituted with 1 to 3 substituents selected independently from Substituent-group A, $C_{3-7}$ cycloalkyl optionally-substituted with 1 to 3 substituents selected independently from Substituent-group B, $C_{3-7}$ cycloalkoxy optionally-substituted with 1

to 3 substituents selected independently from Substituent-group B, and 3- to 7-membered non-aromatic heterocyclyl optionally-substituted with 1 to 3 substituents selected independently from Substituent-group B,
Substituent-group A is independently halogen, hydroxy group, $C_{1-4}$ alkoxy, $C_{3-7}$ cycloalkyl, or $C_{3-7}$ cycloalkoxy,
Substituent-group B is independently halogen, hydroxy group, $C_{1-4}$ alkyl, $C_{1-4}$ alkoxy, $C_{3-7}$ cycloalkyl, or $C_{3-7}$ cycloalkoxy,
provided that the following compounds are excluded:

5-(2-butyl-1-oxo-1,2,3,4-tetrahydropyrrolo[1,2-a]pyrazin-6-yl)-1-(2-hydroxyethyl)-1,3-dihydro-2H-benzimidazol-2-one,
3-[3-methyl-2-oxo-1-(3,3,3-trifluoro-2-hydroxypropyl)-2,3-dihydro-1H-benzimidazol-5-yl]pyridine-4-carbonitrile, and
3-[3-methyl-2-oxo-1-(3,3,3-trifluoro-2-hydroxy-2-methylpropyl)-2,3-dihydro-1H-benzimidazol-5-yl]pyridine-4-carbonitrile.

**2.** The compound of claim 1 or a pharmaceutically acceptable salt thereof, wherein
$R^{1a}$, $R^{1b}$ , $R^{1c}$, and $R^{1d}$ are independently, hydrogen, halogen, $C_{1-4}$ alkyl, $C_{1-4}$ alkoxy (wherein the alkyl and the alkyl moiety in the alkoxy may be independently substituted with 1 to 3, the same or different halogen atoms), $C_{6-10}$ aryl, $C_{6-10}$ aryloxy, 5- to 12-membered heteroaryl, or 5- to 12-membered heteroaryloxy (wherein the aryl and the aryl moiety in the aryloxy, and the heteroaryl and the heteroaryl moiety in the heteroaryloxy may be independently substituted with 1 to 3 substituents selected independently from the group consisting of halogen, cyano, $C_{1-4}$ alkyl optionally-substituted with 1 to 3 substituents selected independently from Substituent-group A, and $C_{1-4}$ alkoxy optionally-substituted with 1 to 3 substituents selected independently from Substituent-group A).

**3.** The compound of claim 1 or 2 or a pharmaceutically acceptable salt thereof, wherein
$R^{1a}$, $R^{1b}$, $R^{1c}$, and $R^{1d}$ are independently, hydrogen, $C_{6-10}$ aryl, $C_{6-10}$ aryloxy, 5- to 12-membered heteroaryl, or 5- to 12-membered heteroaryloxy, wherein the aryl and the aryl moiety in the aryloxy, and the heteroaryl and the heteroaryl moiety in the heteroaryloxy may be independently substituted with 1 to 3 substituents selected independently from the group consisting of halogen, cyano, $C_{1-4}$ alkyl optionally-substituted with 1 to 3 substituents selected independently from Substituent-group A, and $C_{1-4}$ alkoxy optionally-substituted with 1 to 3 substituents selected independently from Substituent-group A.

**4.** The compound of any one of claims 1 to 3 or a pharmaceutically acceptable salt thereof, wherein $R^{1a}$ and $R^{1d}$ are hydrogen.

**5.** The compound of any one of claims 1 to 4 or a pharmaceutically acceptable salt thereof, wherein
$R^{1b}$ or $R^{1c}$ is $C_{6-10}$ aryl, $C_{6-10}$ aryloxy, 5- to 12-membered heteroaryl, or 5- to 12-membered heteroaryloxy, wherein the aryl and the aryl moiety in the aryloxy, and the heteroaryl and the heteroaryl moiety in the heteroaryloxy may be independently substituted with 1 to 3 substituents selected independently from the group consisting of halogen, $C_{1-4}$ alkyl optionally-substituted with 1 to 3 substituents selected independently from Substituent-group A, and $C_{1-4}$ alkoxy optionally-substituted with 1 to 3 substituents selected independently from Substituent-group A.

**6.** The compound of any one of claims 1 to 5 or a pharmaceutically acceptable salt thereof, wherein
$R^2$ and $R^3$ are independently hydrogen or $C_{1-6}$ alkyl which may be independently substituted with 1 to 5 substituents selected independently from the group consisting of halogen, hydroxy group, and $C_{1-4}$ alkoxy optionally-substituted with 1 to 3 substituents selected independently from Substituent-group A, or
in $R^2$, $R^3$, and the hydroxy group bound to the carbon atom which is connected to $R^2$ and $R^3$,
$R^2$ and $R^3$ may be combined together with the carbon atom to which they are attached to form the following group of formula (IIa) with the hydroxy group

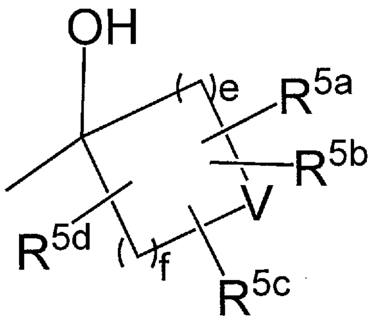

(IIa)

in formula (IIa),

e and f are independently 1 or 2,
V is as defined in claim 1, and
$R^{5a}$, $R^{5b}$, $R^{5c}$, and $R^{5d}$ are independently hydrogen or halogen, or
in $R^2$, $R^3$, the hydroxy group bound to the carbon atom which is connected to $R^2$ and $R^3$, and $CR^7R^8$ in L, $R^2$ and $R^7$ may be combined together with the carbon atom to which they are attached to form the following group of formula (IIIa) with $R^3$, the hydroxy group and $R^8$

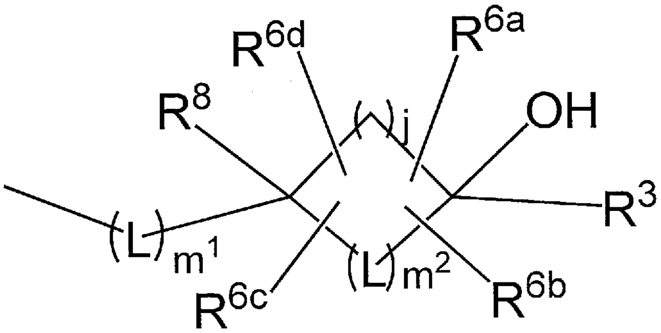

(IIIa)

in formula (IIIa),

$m^1$ is 0,
$m^2$ is 1 or 2, j is 1 or 2,
$R^8$ is hydrogen,
L is as defined in claim 1,
$R^{6a}$, $R^{6b}$, $R^{6c}$, and $R^{6d}$ are independently hydrogen or halogen.

**7.** The compound of any one of claims 1 to 6 or a pharmaceutically acceptable salt thereof, wherein
$R^7$ and $R^8$ are independently hydrogen or $C_{1-4}$ alkyl which may be substituted with 1 to 3 substituents selected independently from the group consisting of halogen, hydroxy group, $C_{1-4}$ alkoxy optionally-substituted with 1 to 3 substituents selected independently from Substituent-group A, $C_{3-7}$ cycloalkyl optionally-substituted with 1 to 3 substituents selected independently from Substituent-group B, $C_{3-7}$ cycloalkoxy optionally-substituted with 1 to 3 substituents selected independently from Substituent-group B, and 3- to 7-membered non-aromatic heterocyclyl optionally-substituted with 1 to 3 substituents selected independently from Substituent-group B, and
m is 1 or 2.

**8.** The compound of any one of claims 1 to 7 or a pharmaceutically acceptable salt thereof, wherein $R^7$ and $R^8$ are hydrogen, and m is 1.

**9.** The compound of any one of claims 1 to 8 or a pharmaceutically acceptable salt thereof, wherein $R^4$ is hydrogen or $C_{1-4}$ alkyl optionally-substituted with 1 to 5 the same or different halogen atoms.

**10.** The compound of any one of claims 1 to 9 or a pharmaceutically acceptable salt thereof, wherein
$R^{1b}$ is $C_{6-10}$ aryl, $C_{6-10}$ aryloxy, 5- to 12-membered heteroaryl, or 5- to 12-membered heteroaryloxy, wherein the

aryl and the aryl moiety in the aryloxy, and the heteroaryl and the heteroaryl moiety in the heteroaryloxy may be independently substituted with 1 to 3 substituents selected independently from the group consisting of halogen, cyano, $C_{1-4}$ alkyl optionally-substituted with 1 to 3 substituents selected independently from Substituent-group A, and $C_{1-4}$ alkoxy optionally-substituted with 1 to 3 substituents selected independently from Substituent-group A.

**11.** The compound of any one of claims 1 to 9 or a pharmaceutically acceptable salt thereof, wherein $R^{1c}$ is $C_{6-10}$ aryl, $C_{6-10}$ aryloxy, 5- to 12-membered heteroaryl, or 5- to 12-membered heteroaryloxy, wherein the aryl and the aryl moiety in the aryloxy, and the heteroaryl and the heteroaryl moiety in the heteroaryloxy may be independently substituted with 1 to 3 substituents selected independently from the group consisting of halogen, cyano, $C_{1-4}$ alkyl optionally-substituted with 1 to 3 substituents selected independently from Substituent-group A, and $C_{1-4}$ alkoxy optionally-substituted with 1 to 3 substituents selected independently from Substituent-group A.

**12.** The compound of claim 1 or a pharmaceutically acceptable salt thereof, which is selected from the following compounds:

1-(2-hydroxy-2-methylpropyl)-6-{[5-(trifluoromethyl)pyridin-2-yl]oxy}-1,3-dihydro-2H-benzimidazol-2-one,
1-[(3-hydroxyoxetan-3-yl)methyl]-6-{[5-(trifluoromethyl)pyridin-2-yl]oxy}-1,3-dihydro-2H-benzimidazol-2-one,
1-[(3-hydroxyoxetan-3-yl)methyl]-6-{[6-(trifluoromethyl)pyridin-3-yl]oxy}-1,3-dihydro-2H-benzimidazol-2-one,
1-(cis-4-hydroxycyclohexyl)-6-{[5-(trifluoromethyl)pyridin-2-yl]oxy}-1,3-dihydro-2H-benzimidazol-2-one,
1-[(3-hydroxyoxetan-3-yl)methyl]-6-[4-(trifluoromethoxy)phenoxy]-1,3-dihydro-2H-benzimidazol-2-one,
6-(4-fluorophenoxy)-1-(2-hydroxy-2-methylpropyl)-1,3-dihydro-2H-benzimidazol-2-one,
6-(4-fluorophenoxy)-1-[(3-hydroxyoxetan-3-yl)methyl]-1,3-dihydro-2H-benzimidazol-2-one,
6-(4-chlorophenoxy)-1-[(3-hydroxyoxetan-3-yl)methyl]-1,3-dihydro-2H-benzimidazol-2-one,
1-[(2S)-3-hydroxy-3-methylbutan-2-yl]-6-{ [5-(trifluoromethyl)pyridin-2-yl]oxy}-1,3-dihydro-2H-benzimidazol-2-one,
6-(4-fluorophenoxy)-1-[(2S)-3-hydroxy-3-methylbutan-2-yl]-1,3-dihydro-2H-benzimidazol-2-one,
6-(4-fluorophenoxy)-1-(cis-4-hydroxycyclohexyl)-1,3-dihydro-2H-benzimidazol-2-one,
1-ethyl-3-(2-hydroxy-2-methylpropyl)-5-{[5-(trifluoromethyl)pyridin-2-yl]oxy}-1,3-dihydro-2H-benzimidazol-2-one,
3-(2-hydroxy-2-methylpropyl)-1-methyl-5-{[5-(trifluoromethyl)pyridin-2-yl]oxy}-1,3-dihydro-2H-benzimidazol-2-one,
3-[(3-hydroxyoxetan-3-yl)methyl]-1-methyl-5-{[5-(trifluoromethyl)pyridin-2-yl]oxy}-1,3-dihydro-2H-benzimidazol-2-one,
3-[(3-hydroxyoxetan-3-yl)methyl]-1-methyl-5-{[6-(trifluoromethyl)pyridin-3-yl]oxy}-1,3-dihydro-2H-benzimidazol-2-one,
5-(4-fluorophenoxy)-3-[(3-hydroxyoxetan-3-yl)methyl]-1-methyl-1,3-dihydro-2H-benzimidazol-2-one,
3-[(3-hydroxyoxetan-3-yl)methyl]-1-methyl-5-[4-(trifluoromethoxy)phenoxy]-1,3-dihydro-2H-benzimidazol-2-one,
5-(4-chlorophenoxy)-3-[(3-hydroxyoxetan-3-yl)methyl]-1-methyl-1,3-dihydro-2H-benzimidazol-2-one,
1-(2-hydroxy-2-methylpropyl)-6-[6-(trifluoromethyl)pyridin-3-yl]-1,3-dihydro-2H-benzimidazol-2-one,
1-(2-hydroxy-2-methylpropyl)-6-[5-(trifluoromethyl)pyridin-2-yl]-1,3-dihydro-2H-benzimidazol-2-one,
1-(2-hydroxy-2-methylpropyl)-6-[4-(trifluoromethyl)phenyl]-1,3-dihydro-2H-benzimidazol-2-one,
6-(4-fluorophenyl)-1-(2-hydroxy-2-methylpropyl)-1,3-dihydro-2H-benzimidazol-2-one,
1-(2-hydroxy-2-methylpropyl)-5-[6-(trifluoromethyl)pyridin-3-yl]-1,3-dihydro-2H-benzimidazol-2-one,
5-(4-fluorophenyl)-1-(2-hydroxy-2-methylpropyl)-1,3-dihydro-2H-benzimidazol-2-one,
1-(2-hydroxy-2-methylpropyl)-5-[4-(trifluoromethoxy)phenoxy]-1,3-dihydro-2H-benzimidazol-2-one,
1-(2-hydroxy-2-methylpropyl)-5-{[5-(trifluoromethyl)pyridin-2-yl]oxy}-1,3-dihydro-2H-benzimidazol-2-one,
1-[(3-hydroxyoxetan-3-yl)methyl]-5-[4-(trifluoromethoxy)phenoxy]-1,3-dihydro-2H-benzimidazol-2-one, and
1-[(3-hydroxyoxetan-3-yl)methyl]-3-methyl-5-[4-(trifluoromethoxy)phenoxy]-1,3-dihydro-2H-benzimidazol-2-one.

**13.** The compound of claim 1 or a pharmaceutically acceptable salt thereof, which is selected from the following compounds:

1-(2-hydroxy-2-methylpropyl)-6-{[5-(trifluoromethyl)pyridin-2-yl]oxy}-1,3-dihydro-2H-benzimidazol-2-one,
1-[(3-hydroxyoxetan-3-yl)methyl]-6-[4-(trifluoromethoxy)phenoxy]-1,3-dihydro-2H-benzimidazol-2-one,
6-(4-chlorophenoxy)-1-[(3-hydroxyoxetan-3-yl)methyl]-1,3-dihydro-2H-benzimidazol-2-one,
6-(4-fluorophenoxy)-1-[(2S)-3-hydroxy-3-methylbutan-2-yl]-1,3-dihydro-2H-benzimidazol-2-one,

6-(4-fluorophenoxy)-1-(cis-4-hydroxycyclohexyl)-1,3-dihydro-2H-benzimidazol-2-one,
3-[(3-hydroxyoxetan-3-yl)methyl]-1-methyl-5-[4-(trifluoromethoxy)phenoxy]-1,3-dihydro-2H-benzimidazol-2-one,
1-(2-hydroxy-2-methylpropyl)-6-[5-(trifluoromethyl)pyridin-2-yl]-1,3-dihydro-2H-benzimidazol-2-one,
1-(2-hydroxy-2-methylpropyl)-6-[4-(trifluoromethyl)phenyl]-1,3-dihydro-2H-benzimidazol-2-one,
1-(2-hydroxy-2-methylpropyl)-5-[6-(trifluoromethyl)pyridin-3-yl]-1,3-dihydro-2H-benzimidazol-2-one,
5-(4-fluorophenyl)-1-(2-hydroxy-2-methylpropyl)-1,3-dihydro-2H-benzimidazol-2-one,
1-(2-hydroxy-2-methylpropyl)-5-{[5-(trifluoromethyl)pyridin-2-yl]oxy}-1,3-dihydro-2H-benzimidazol-2-one, and
1-[(3-hydroxyoxetan-3-yl)methyl]-3-methyl-5-[4-(trifluoromethoxy)phenoxy]-1,3-dihydro-2H-benzimidazol-2-one.

**14.** A pharmaceutical combination comprising the compound of any one of claims 1 to 13 or a pharmaceutically acceptable salt thereof.

**15.** A medicament for treating a disease involving Nav 1.7 (SCN9A), comprising the compound of any one of claims 1 to 13 or a pharmaceutically acceptable salt thereof as an active ingredient.

**16.** A medicament for treating neuropathic pain, nociceptive pain, inflammatory pain, small-fiber neuropathy, erythromelalgia, paroxysmal extreme pain disorder, dysuria, or multiple sclerosis, which comprises the compound of any one of claims 1 to 13 or a pharmaceutically acceptable salt thereof as an active ingredient.

**17.** A pharmaceutical combination comprising the compound of any one of claims 1 to 13 or a pharmaceutically acceptable salt thereof, and at least one drug selected from the group consisting of an antiepileptic agent, an antidepressive agent, a narcotic analgesic, an anti-inflammatory agent, a reductase inhibitor, and a prostaglandin derivative drug.

**18.** Use of the compound of any one of claims 1 to 13 or a pharmaceutically acceptable salt thereof in the manufacture of a medicament for treating neuropathic pain, nociceptive pain, inflammatory pain, small-fiber neuropathy, erythromelalgia, paroxysmal extreme pain disorder, dysuria, or multiple sclerosis.

**19.** A method for treating neuropathic pain, nociceptive pain, inflammatory pain, small-fiber neuropathy, erythromelalgia, paroxysmal extreme pain disorder, dysuria, or multiple sclerosis, which comprises administering a therapeutically effective amount of the compound of any one of claims 1 to 13 or a pharmaceutically acceptable salt thereof to a mammal in need thereof.

**INTERNATIONAL SEARCH REPORT**

International application No. PCT/JP2018/009881

A. CLASSIFICATION OF SUBJECT MATTER
Int.Cl. C07D235/26(2006.01)i, A61K31/4439(2006.01)i, A61K45/00(2006.01)i, A61P13/00(2006.01)i, A61P25/00(2006.01)i, A61P25/04(2006.01)i, A61P29/00(2006.01)i, A61P43/00(2006.01)i, C07D401/12(2006.01)i, C07D405/12(2006.01)i, C07D405/14(2006.01)i

According to International Patent Classification (IPC) or to both national classification and IPC

B. FIELDS SEARCHED

Minimum documentation searched (classification system followed by classification symbols)
Int.Cl. C07D235/26, A61K31/4439, A61K45/00, A61P13/00, A61P25/00, A61P25/04, A61P29/00, A61P43/00, C07D401/12, C07D405/12, C07D405/14

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2012/021382 A1 (MERCK SHARP & DOHME CORP.) 16 February 2012, claims 1, 15-17, page 9, lines 18-30, page 14, line 10 to page 15, line 13, table 1, compounds 1-150, 1-164 & US 2013/0143880 A1 & EP 2603079 A1 | 1-16, 18-19<br>17 |
| Y | JP 2016-132649 A (SUMITOMO DAINIPPON PHARMA CO., LTD.) 25 July 2016, claim 17, paragraphs [0033], [0209] (Family: none) | 17 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 June 2018 (07.06.2018) | 19 June 2018 (19.06.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2018/009881

C (Continuation). DOCUMENTS CONSIDERED TO BE RELEVANT

| Category* | Citation of document, with indication, where appropriate, of the relevant passages | Relevant to claim No. |
|---|---|---|
| A | JP 2010-527974 A (ABBOTT GMBH & CO. KG) 19 August 2010, claims 55, 57, 60 & US 2008/0300260 A1, claims 19-20 & JP 5615700 B2 & WO 2008/145616 A1 & EP 2167464 A1 & CA 2686651 A & CN 101687790 A & KR 10-2010-0017372 A & IL 201995 D & MX 2009012749 A & AU 2008257559 A & RU 2009148336 A & TW 200902501 A & NZ 581127 A | 1-19 |
| A | JP 2012-521428 A (MERCK SHARP & DOHME CORP.) 13 September 2012, claim 13 & US 2012/0064181 A1, claim 13 & WO 2010/111058 A1 & EP 2410857 A1 & CA 2755680 A & AU 2010229142 A | 1-19 |
| A | WO 01/057019 A1 (COR THERAPEUTICS, INC.) 09 August 2001, claim 1 & US 2002/0019395 A1 & EP 1263754 A1 & AU 3470501 A | 1-19 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2001057019 A


### Non-patent literature cited in the description


- *Nat Rev Neurosci.,* 2013, vol. 14, 49
- *Nat Commun.,* 2012, vol. 3, 791
- **R. C. LAROCK.** *Comprehensive Organic Transformations,* 1989